# EUROPEAN PATENT SPECIFICATION

(11) **EP 3 266 455 B1**
(45) Date of publication and mention of the grant of the patent: **30.12.2020**
(21) Application number: 16761953.5
(22) Date of filing: 07.03.2016
(51) Int. Cl.: A61K 31/436, A61K 31/4375, A61K 31/519, A61K 31/497, A61K 31/5377, C12Q 1/68, G01N 33/68, C12N 15/85, A01K 67/027

(54) **COMPOSITION FOR PREVENTION OR TREATMENT OF INTRACTABLE EPILEPSY COMPRISING MTOR INHIBITOR**
ZUSAMMENSETZUNG ZUR PRÄVENTION ODER BEHANDLUNG VON SCHWER BEHANDELBARER EPILEPSIE MIT MTOR-INHIBITOR
COMPOSITION POUR LA PRÉVENTION OU LE TRAITEMENT DE L'ÉPILEPSIE RÉFRACTAIRE COMPRENANT UN INHIBITEUR MTOR

(30) Priority: 06.03.2015 KR 20150031606; 20.03.2015 KR 20150038668; 29.01.2016 KR 20160011747; 04.03.2016 KR 20160026643
(43) Date of publication of application: 10.01.2018
(73) Proprietor: Korea Advanced Institute of Science and Technology, Daejeon 34141 (KR); Yonsei University, University-Industry Foundation(UIF)., Seoul 03722 (KR)
(72) Inventor: LEE, Jeong Ho, Daejeon 34141 (KR); LIM, Jae Seok, Daejeon 34141 (KR); KIM, Woo-Il, Daejeon 34141 (KR); KIM, Dong Seok, Seoul 03631 (KR); KANG, Hoon Chul, Seoul 03722 (KR); KIM, Se Hoon, Seoul 03789 (KR)
(74) Representative: Grünecker Patent- und Rechtsanwälte PartG mbB
(86) International application number: PCT/KR2016/002248
(87) International publication number: WO 2016/144066

(56) References cited:
- KR-A- 20070 116 555
- US-A1- 2015 065 380
- US-A1- 2015 065 380
- M. C. LJUNGBERG ET AL: "Rapamycin suppresses seizures and neuronal hypertrophy in a mouse model of cortical dysplasia", DISEASE MODELS & MECHANISMS, vol. 2, no. 7-8, 26 May 2009 (2009-05-26), pages 389-398, XP055323889, GB ISSN: 1754-8403, DOI: 10.1242/dmm.002386
- MICHAEL CARDAMONE ET AL: "Mammalian Target of Rapamycin Inhibitors for Intractable Epilepsy and Subependymal Giant Cell Astrocytomas in Tuberous Sclerosis Complex", JOURNAL OF PEDIATRICS., vol. 164, no. 5, 1 May 2014 (2014-05-01), pages 1195-1200, XP055489978, US ISSN: 0022-3476, DOI: 10.1016/j.jpeds.2013.12.053
- PAOLO CURATOLO ET AL: "mTOR inhibitors as a new therapeutic option for epilepsy", EXPERT REVIEW OF NEUROTHERAPEUTICS, vol. 13, no. 6, 1 June 2013 (2013-06-01), pages 627-638, XP055489958, GB ISSN: 1473-7175, DOI: 10.1586/ern.13.49
- LING-HUI ZENG ET AL: "Rapamycin prevents epilepsy in a mouse model of tuberous sclerosis complex", ANNALS OF NEUROLOGY., vol. 63, no. 4, 1 April 2008 (2008-04-01), pages 444-453, XP055490101, BOSTON, US ISSN: 0364-5134, DOI: 10.1002/ana.21331
- JAE SEOK LIM ET AL: "Brain somatic mutations in MTOR cause focal cortical dysplasia type II leading to intractable epilepsy", NATURE MEDICINE, vol. 21, no. 4, 23 March 2015 (2015-03-23) , pages 395-400, XP055490174, New York ISSN: 1078-8956, DOI: 10.1038/nm.3824
- LJUNGBERG, M. C. ET AL.: 'Rapamycin suppresses Seizures and Neuronal Hypertrophy in a Mouse Model of Cortical Dysplasia' DISEASE MODELS AND MECHANISMS vol. 2, no. 7-8, 2009, pages 389 - 398, XP055323889
- D'GAMA, A. M. ET AL.: 'Mammalian Target of Rapamycin Pathway Mutations cause Hemimegalencephaly and Focal Cortical Dysplasia' ANNALS OF NEUROLOGY vol. 77, 26 February 2015, pages 720 - 725, XP055323904
- SANCAK, O. ET AL.: 'Mutational Analysis of the TSC1 and TSC2 Genes in a Diagnostic Setting: Genotype-phenotype Correlations and Comparison of Diagnostic DNA Techniques in Tuberous Sclerosis Complex' EUROPEAN JOURNAL OF HUMAN GENETICS vol. 13, 2005, pages 731 - 741, XP055323910

<110> KOREA ADVANCED INSTITUTE OF SCIENCE AND TECHNOLOGY Yonsei University, University - Industry Foundation(UIF)
<120> Composition for diagnosis or treatment of intractable epilepsy
<130> OPP20154369KR
<160> 30
<170> Kopatentln 1.71
<210> 1
   <211> 7650
   <212> DNA
   <213> Homo sapiens
<220>
   <221> gene
   <222> (1)..(7650)
   <223> wild type mTOR
<400> 1 
atgcttggaa ccggacctgc cgccgccacc accgctgcca ccacatctag caatgtgagc 60

gtcctgcagc agtttgccag tggcctaaag agccggaatg aggaaaccag ggccaaagcc 120

gccaaggagc tccagcacta tgtcaccatg gaactccgag agatgagtca agaggagtct 180

actcgcttct atgaccaact gaaccatcac atttttgaat tggtttccag ctcagatgcc 240

aatgagagga aaggtggcat cttggccata gctagcctca taggagtgga aggtgggaat 300

gccacccgaa ttggcagatt tgccaactat cttcggaacc tcctcccctc caatgaccca 360

gttgtcatgg aaatggcatc caaggccatt ggccgtcttg ccatggcagg ggacactttt 420

accgctgagt acgtggaatt tgaggtgaag cgagccctgg aatggctggg tgctgaccgc 480

aatgagggcc ggagacatgc agctgtcctg gttctccgtg agctggccat cagcgtccct 540

accttcttct tccagcaagt gcaacccttc tttgacaaca tttttgtggc cgtgtgggac 600

cccaaacagg ccatccgtga gggagctgta gccgcccttc gtgcctgtct gattctcaca 660

acccagcgtg agccgaagga gatgcagaag cctcagtggt acaggcacac atttgaagaa 720

gcagagaagg gatttgatga gaccttggcc aaagagaagg gcatgaatcg ggatgatcgg 780

atccatggag ccttgttgat ccttaacgag ctggtccgaa tcagcagcat ggagggagag 840

cgtctgagag aagaaatgga agaaatcaca cagcagcagc tggtacacga caagtactgc 900

aaagatctca tgggcttcgg aacaaaacct cgtcacatta cccccttcac cagtttccag 960

gctgtacagc cccagcagtc aaatgccttg gtggggctgc tggggtacag ctctcaccaa 1020

ggcctcatgg gatttgggac ctcccccagt ccagctaagt ccaccctggt ggagagccgg 1080

tgttgcagag acttgatgga ggagaaattt gatcaggtgt gccagtgggt gctgaaatgc 1140

aggaatagca agaactcgct gatccaaatg acaatcctta atttgttgcc ccgcttggct 1200

gcattccgac cttctgcctt cacagatacc cagtatctcc aagataccat gaaccatgtc 1260

ctaagctgtg tcaagaagga gaaggaacgt acagcggcct tccaagccct ggggctactt 1320

tctgtggctg tgaggtctga gtttaaggtc tatttgcctc gcgtgctgga catcatccga 1380
 
gcggccctgc ccccaaagga cttcgcccat aagaggcaga aggcaatgca ggtggatgcc 1440
acagtcttca cttgcatcag catgctggct cgagcaatgg ggccaggcat ccagcaggat 1500
atcaaggagc tgctggagcc catgctggca gtgggactaa gccctgccct cactgcagtg 1560
ctctacgacc tgagccgtca gattccacag ctaaagaagg acattcaaga tgggctactg 1620
aaaatgctgt ccctggtcct tatgcacaaa ccccttcgcc acccaggcat gcccaagggc 1680
ctggcccatc agctggcctc tcctggcctc acgaccctcc ctgaggccag cgatgtgggc 1740
agcatcactc ttgccctccg aacgcttggc agctttgaat ttgaaggcca ctctctgacc 1800
caatttgttc gccactgtgc ggatcatttc ctgaacagtg agcacaagga gatccgcatg 1860
gaggctgccc gcacctgctc ccgcctgctc acaccctcca tccacctcat cagtggccat 1920
gctcatgtgg ttagccagac cgcagtgcaa gtggtggcag atgtgcttag caaactgctc 1980
gtagttggga taacagatcc tgaccctgac attcgctact gtgtcttggc gtccctggac 2040
gagcgctttg atgcacacct ggcccaggcg gagaacttgc aggccttgtt tgtggctctg 2100
aatgaccagg tgtttgagat ccgggagctg gccatctgca ctgtgggccg actcagtagc 2160
atgaaccctg cctttgtcat gcctttcctg cgcaagatgc tcatccagat tttgacagag 2220
ttggagcaca gtgggattgg aagaatcaaa gagcagagtg cccgcatgct ggggcacctg 2280
gtctccaatg ccccccgact catccgcccc tacatggagc ctattctgaa ggcattaatt 2340
ttgaaactga aagatccaga ccctgatcca aacccaggtg tgatcaataa tgtcctggca 2400
acaataggag aattggcaca ggttagtggc ctggaaatga ggaaatgggt tgatgaactt 2460
tttattatca tcatggacat gctccaggat tcctctttgt tggccaaaag gcaggtggct 2520
ctgtggaccc tgggacagtt ggtggccagc actggctatg tagtagagcc ctacaggaag 2580
taccctactt tgcttgaggt gctactgaat tttctgaaga ctgagcagaa ccagggtaca 2640
cgcagagagg ccatccgtgt gttagggctt ttaggggctt tggatcctta caagcacaaa 2700
gtgaacattg gcatgataga ccagtcccgg gatgcctctg ctgtcagcct gtcagaatcc 2760
aagtcaagtc aggattcctc tgactatagc actagtgaaa tgctggtcaa catgggaaac 2820
ttgcctctgg atgagttcta cccagctgtg tccatggtgg ccctgatgcg gatcttccga 2880
gaccagtcac tctctcatca tcacaccatg gttgtccagg ccatcacctt catcttcaag 2940
tccctgggac tcaaatgtgt gcagttcctg ccccaggtca tgcccacgtt ccttaacgtc 3000
attcgagtct gtgatggggc catccgggaa tttttgttcc agcagctggg aatgttggtg 3060
tcctttgtga agagccacat cagaccttat atggatgaaa tagtcaccct catgagagaa 3120
ttctgggtca tgaacacctc aattcagagc acgatcattc ttctcattga gcaaattgtg 3180
gtagctcttg ggggtgaatt taagctctac ctgccccagc tgatcccaca catgctgcgt 3240
gtcttcatgc atgacaacag cccaggccgc attgtctcta tcaagttact ggctgcaatc 3300
cagctgtttg gcgccaacct ggatgactac ctgcatttac tgctgcctcc tattgttaag 3360
ttgtttgatg cccctgaagc tccactgcca tctcgaaagg cagcgctaga gactgtggac 3420
 
cgcctgacgg agtccctgga tttcactgac tatgcctccc ggatcattca ccctattgtt 3480
cgaacactgg accagagccc agaactgcgc tccacagcca tggacacgct gtcttcactt 3540
gtttttcagc tggggaagaa gtaccaaatt ttcattccaa tggtgaataa agttctggtg 3600
cgacaccgaa tcaatcatca gcgctatgat gtgctcatct gcagaattgt caagggatac 3660
acacttgctg atgaagagga ggatcctttg atttaccagc atcggatgct taggagtggc 3720
caaggggatg cattggctag tggaccagtg gaaacaggac ccatgaagaa actgcacgtc 3780
agcaccatca acctccaaaa ggcctggggc gctgccagga gggtctccaa agatgactgg 3840
ctggaatggc tgagacggct gagcctggag ctgctgaagg actcatcatc gccctccctg 3900
cgctcctgct gggccctggc acaggcctac aacccgatgg ccagggatct cttcaatgct 3960
gcatttgtgt cctgctggtc tgaactgaat gaagatcaac aggatgagct catcagaagc 4020
atcgagttgg ccctcacctc acaagacatc gctgaagtca cacagaccct cttaaacttg 4080
gctgaattca tggaacacag tgacaagggc cccctgccac tgagagatga caatggcatt 4140
gttctgctgg gtgagagagc tgccaagtgc cgagcatatg ccaaagcact acactacaaa 4200
gaactggagt tccagaaagg ccccacccct gccattctag aatctctcat cagcattaat 4260
aataagctac agcagccgga ggcagcggcc ggagtgttag aatatgccat gaaacacttt 4320
ggagagctgg agatccaggc tacctggtat gagaaactgc acgagtggga ggatgccctt 4380
gtggcctatg acaagaaaat ggacaccaac aaggacgacc cagagctgat gctgggccgc 4440
atgcgctgcc tcgaggcctt gggggaatgg ggtcaactcc accagcagtg ctgtgaaaag 4500
tggaccctgg ttaatgatga gacccaagcc aagatggccc ggatggctgc tgcagctgca 4560
tggggtttag gtcagtggga cagcatggaa gaatacacct gtatgatccc tcgggacacc 4620
catgatgggg cattttatag agctgtgctg gcactgcatc aggacctctt ctccttggca 4680
caacagtgca ttgacaaggc cagggacctg ctggatgctg aattaactgc gatggcagga 4740
gagagttaca gtcgggcata tggggccatg gtttcttgcc acatgctgtc cgagctggag 4800
gaggttatcc agtacaaact tgtccccgag cgacgagaga tcatccgcca gatctggtgg 4860
gagagactgc agggctgcca gcgtatcgta gaggactggc agaaaatcct tatggtgcgg 4920
tcccttgtgg tcagccctca tgaagacatg agaacctggc tcaagtatgc aagcctgtgc 4980
ggcaagagtg gcaggctggc tcttgctcat aaaactttag tgttgctcct gggagttgat 5040
ccgtctcggc aacttgacca tcctctgcca acagttcacc ctcaggtgac ctatgcctac 5100
atgaaaaaca tgtggaagag tgcccgcaag atcgatgcct tccagcacat gcagcatttt 5160
gtccagacca tgcagcaaca ggcccagcat gccatcgcta ctgaggacca gcagcataag 5220
caggaactgc acaagctcat ggcccgatgc ttcctgaaac ttggagagtg gcagctgaat 5280
ctacagggca tcaatgagag cacaatcccc aaagtgctgc agtactacag cgccgccaca 5340
gagcacgacc gcagctggta caaggcctgg catgcgtggg cagtgatgaa cttcgaagct 5400
gtgctacact acaaacatca gaaccaagcc cgcgatgaga agaagaaact gcgtcatgcc 5460
 
agcggggcca acatcaccaa cgccaccact gccgccacca cggccgccac tgccaccacc 5520
actgccagca ccgagggcag caacagtgag agcgaggccg agagcaccga gaacagcccc 5580
accccatcgc cgctgcagaa gaaggtcact gaggatctgt ccaaaaccct cctgatgtac 5640
acggtgcctg ccgtccaggg cttcttccgt tccatctcct tgtcacgagg caacaacctc 5700
caggatacac tcagagttct caccttatgg tttgattatg gtcactggcc agatgtcaat 5760
gaggccttag tggagggggt gaaagccatc cagattgata cctggctaca ggttatacct 5820
cagctcattg caagaattga tacgcccaga cccttggtgg gacgtctcat tcaccagctt 5880
ctcacagaca ttggtcggta ccacccccag gccctcatct acccactgac agtggcttct 5940
aagtctacca cgacagcccg gcacaatgca gccaacaaga ttctgaagaa catgtgtgag 6000
cacagcaaca ccctggtcca gcaggccatg atggtgagcg aggagctgat ccgagtggcc 6060
atcctctggc atgagatgtg gcatgaaggc ctggaagagg catctcgttt gtactttggg 6120
gaaaggaacg tgaaaggcat gtttgaggtg ctggagccct tgcatgctat gatggaacgg 6180
ggcccccaga ctctgaagga aacatccttt aatcaggcct atggtcgaga tttaatggag 6240
gcccaagagt ggtgcaggaa gtacatgaaa tcagggaatg tcaaggacct cacccaagcc 6300
tgggacctct attatcatgt gttccgacga atctcaaagc agctgcctca gctcacatcc 6360
ttagagctgc aatatgtttc cccaaaactt ctgatgtgcc gggaccttga attggctgtg 6420
ccaggaacat atgaccccaa ccagccaatc attcgcattc agtccatagc accgtctttg 6480
caagtcatca catccaagca gaggccccgg aaattgacac ttatgggcag caacggacat 6540
gagtttgttt tccttctaaa aggccatgaa gatctgcgcc aggatgagcg tgtgatgcag 6600
ctcttcggcc tggttaacac ccttctggcc aatgacccaa catctcttcg gaaaaacctc 6660
agcatccaga gatacgctgt catcccttta tcgaccaact cgggcctcat tggctgggtt 6720
ccccactgtg acacactgca cgccctcatc cgggactaca gggagaagaa gaagatcctt 6780
ctcaacatcg agcatcgcat catgttgcgg atggctccgg actatgacca cttgactctg 6840
atgcagaagg tggaggtgtt tgagcatgcc gtcaataata cagctgggga cgacctggcc 6900
aagctgctgt ggctgaaaag ccccagctcc gaggtgtggt ttgaccgaag aaccaattat 6960
acccgttctt tagcggtcat gtcaatggtt gggtatattt taggcctggg agatagacac 7020
ccatccaacc tgatgctgga ccgtctgagt gggaagatcc tgcacattga ctttggggac 7080
tgctttgagg ttgctatgac ccgagagaag tttccagaga agattccatt tagactaaca 7140
agaatgttga ccaatgctat ggaggttaca ggcctggatg gcaactacag aatcacatgc 7200
cacacagtga tggaggtgct gcgagagcac aaggacagtg tcatggccgt gctggaagcc 7260
tttgtctatg accccttgct gaactggagg ctgatggaca caaataccaa aggcaacaag 7320
cgatcccgaa cgaggacgga ttcctactct gctggccagt cagtcgaaat tttggacggt 7380
gtggaacttg gagagccagc ccataagaaa acggggacca cagtgccaga atctattcat 7440
tctttcattg gagacggttt ggtgaaacca gaggccctaa ataagaaagc tatccagatt 7500
 
attaacaggg ttcgagataa gctcactggt cgggacttct ctcatgatga cactttggat 7560
gttccaacgc aagttgagct gctcatcaaa caagcgacat cccatgaaaa cctctgccag 7620
tgctatattg gctggtgccc tttctggtaa 7650
<210> 2
   <211> 2549
   <212> PRT
   <213> Homo sapiens
<220>
   <221> PEPTIDE
   <222> (1)..(2549)
   <223> wild type mTOR
<400> 2 
   Met Leu Gly Thr Gly Pro Ala Ala Ala Thr Thr Ala Ala Thr Thr Ser
   1               5                   10                  15
   Ser Asn Val Ser Val Leu Gln Gln Phe Ala Ser Gly Leu Lys Ser Arg
               20                  25                  30
   Asn Glu Glu Thr Arg Ala Lys Ala Ala Lys Glu Leu Gln His Tyr Val
           35                  40                  45
   Thr Met Glu Leu Arg Glu Met Ser Gln Glu Glu Ser Thr Arg Phe Tyr
       50                  55                  60
   Asp Gln Leu Asn His His Ile Phe Glu Leu Val Ser Ser Ser Asp Ala
   65                  70                  75                  80
   Asn Glu Arg Lys Gly Gly Ile Leu Ala Ile Ala Ser Leu Ile Gly Val
                   85                  90                  95
   Glu Gly Gly Asn Ala Thr Arg Ile Gly Arg Phe Ala Asn Tyr Leu Arg
               100                 105                 110
   Asn Leu Leu Pro Ser Asn Asp Pro Val Val Met Glu Met Ala Ser Lys
           115                 120                 125
   Ala Ile Gly Arg Leu Ala Met Ala Gly Asp Thr Phe Thr Ala Glu Tyr
       130                 135                 140
   Val Glu Phe Glu Val Lys Arg Ala Leu Glu Trp Leu Gly Ala Asp Arg
   145                 150                 155                 160
   Asn Glu Gly Arg Arg His Ala Ala Val Leu Val Leu Arg Glu Leu Ala
                   165                 170                 175
   Ile Ser Val Pro Thr Phe Phe Phe Gln Gln Val Gln Pro Phe Phe Asp
               180                 185                 190
   Asn Ile Phe Val Ala Val Trp Asp Pro Lys Gln Ala Ile Arg Glu Gly
           195                 200                 205
   Ala Val Ala Ala Leu Arg Ala Cys Leu Ile Leu Thr Thr Gln Arg Glu
       210                 215                 220
   Pro Lys Glu Met Gln Lys Pro Gln Trp Tyr Arg His Thr Phe Glu Glu
   225                 230                 235                 240
   Ala Glu Lys Gly Phe Asp Glu Thr Leu Ala Lys Glu Lys Gly Met Asn
                   245                 250                 255
   Arg Asp Asp Arg Ile His Gly Ala Leu Leu Ile Leu Asn Glu Leu Val
   260 265 270
   Arg Ile Ser Ser Met Glu Gly Glu Arg Leu Arg Glu Glu Met Glu Glu
   275 280 285
   Ile Thr Gln Gln Gln Leu Val His Asp Lys Tyr Cys Lys Asp Leu Met
   290 295 300
   Gly Phe Gly Thr Lys Pro Arg His Ile Thr Pro Phe Thr Ser Phe Gln
   305 310 315 320
   Ala Val Gln Pro Gln Gln Ser Asn Ala Leu Val Gly Leu Leu Gly Tyr
   325 330 335
   Ser Ser His Gln Gly Leu Met Gly Phe Gly Thr Ser Pro Ser Pro Ala
   340 345 350
   Lys Ser Thr Leu Val Glu Ser Arg Cys Cys Arg Asp Leu Met Glu Glu
   355 360 365
   Lys Phe Asp Gln Val Cys Gln Trp Val Leu Lys Cys Arg Asn Ser Lys
   370 375 380
   Asn Ser Leu Ile Gln Met Thr Ile Leu Asn Leu Leu Pro Arg Leu Ala
   385 390 395 400
   Ala Phe Arg Pro Ser Ala Phe Thr Asp Thr Gln Tyr Leu Gln Asp Thr
   405 410 415
   Met Asn His Val Leu Ser Cys Val Lys Lys Glu Lys Glu Arg Thr Ala
   420 425 430
   Ala Phe Gln Ala Leu Gly Leu Leu Ser Val Ala Val Arg Ser Glu Phe
   435 440 445
   Lys Val Tyr Leu Pro Arg Val Leu Asp Ile Ile Arg Ala Ala Leu Pro
   450 455 460
   Pro Lys Asp Phe Ala His Lys Arg Gln Lys Ala Met Gln Val Asp Ala
   465 470 475 480
   Thr Val Phe Thr Cys Ile Ser Met Leu Ala Arg Ala Met Gly Pro Gly
   485 490 495
   Ile Gln Gln Asp Ile Lys Glu Leu Leu Glu Pro Met Leu Ala Val Gly
   500 505 510
   Leu Ser Pro Ala Leu Thr Ala Val Leu Tyr Asp Leu Ser Arg Gln Ile
   515 520 525
   Pro Gln Leu Lys Lys Asp Ile Gln Asp Gly Leu Leu Lys Met Leu Ser
   530 535 540
   Leu Val Leu Met His Lys Pro Leu Arg His Pro Gly Met Pro Lys Gly
   545 550 555 560
   Leu Ala His Gln Leu Ala Ser Pro Gly Leu Thr Thr Leu Pro Glu Ala
   565 570 575
   Ser Asp Val Gly Ser Ile Thr Leu Ala Leu Arg Thr Leu Gly Ser Phe
   580 585 590
   Glu Phe Glu Gly His Ser Leu Thr Gln Phe Val Arg His Cys Ala Asp
   595 600 605
   His Phe Leu Asn Ser Glu His Lys Glu Ile Arg Met Glu Ala Ala Arg
   610 615 620
   Thr Cys Ser Arg Leu Leu Thr Pro Ser Ile His Leu Ile Ser Gly His
   625                 630                 635                 640
   Ala His Val Val Ser Gln Thr Ala Val Gln Val Val Ala Asp Val Leu
                   645                 650                 655
   Ser Lys Leu Leu Val Val Gly Ile Thr Asp Pro Asp Pro Asp Ile Arg
               660                 665                 670
   Tyr Cys Val Leu Ala Ser Leu Asp Glu Arg Phe Asp Ala His Leu Ala
           675                 680                 685
   Gln Ala Glu Asn Leu Gln Ala Leu Phe Val Ala Leu Asn Asp Gln Val
       690                 695                 700
   Phe Glu Ile Arg Glu Leu Ala Ile Cys Thr Val Gly Arg Leu Ser Ser
   705                 710                 715                 720
   Met Asn Pro Ala Phe Val Met Pro Phe Leu Arg Lys Met Leu Ile Gln
                   725                 730                 735
   Ile Leu Thr Glu Leu Glu His Ser Gly Ile Gly Arg Ile Lys Glu Gln
               740                 745                 750
   Ser Ala Arg Met Leu Gly His Leu Val Ser Asn Ala Pro Arg Leu Ile
           755                 760                 765
   Arg Pro Tyr Met Glu Pro Ile Leu Lys Ala Leu Ile Leu Lys Leu Lys
       770                 775                 780
   Asp Pro Asp Pro Asp Pro Asn Pro Gly Val Ile Asn Asn Val Leu Ala
   785                 790                 795                 800
   Thr Ile Gly Glu Leu Ala Gln Val Ser Gly Leu Glu Met Arg Lys Trp
                   805                 810                 815
   Val Asp Glu Leu Phe Ile Ile Ile Met Asp Met Leu Gln Asp Ser Ser
               820                 825                 830
   Leu Leu Ala Lys Arg Gln Val Ala Leu Trp Thr Leu Gly Gln Leu Val
           835                 840                 845
   Ala Ser Thr Gly Tyr Val Val Glu Pro Tyr Arg Lys Tyr Pro Thr Leu
       850                 855                 860
   Leu Glu Val Leu Leu Asn Phe Leu Lys Thr Glu Gln Asn Gln Gly Thr
   865                 870                 875                 880
   Arg Arg Glu Ala Ile Arg Val Leu Gly Leu Leu Gly Ala Leu Asp Pro
                   885                 890                 895
   Tyr Lys His Lys Val Asn Ile Gly Met Ile Asp Gln Ser Arg Asp Ala
               900                 905                 910
   Ser Ala Val Ser Leu Ser Glu Ser Lys Ser Ser Gln Asp Ser Ser Asp
           915                 920                 925
   Tyr Ser Thr Ser Glu Met Leu Val Asn Met Gly Asn Leu Pro Leu Asp
       930                 935                 940
   Glu Phe Tyr Pro Ala Val Ser Met Val Ala Leu Met Arg Ile Phe Arg
   945                 950                 955                 960
   Asp Gln Ser Leu Ser His His His Thr Met Val Val Gln Ala Ile Thr
                   965                 970                 975
   Phe Ile Phe Lys Ser Leu Gly Leu Lys Cys Val Gln Phe Leu Pro Gln
   980 985 990
   Val Met Pro Thr Phe Leu Asn Val Ile Arg Val Cys Asp Gly Ala Ile
   995 1000 1005
   Arg Glu Phe Leu Phe Gln Gln Leu Gly Met Leu Val Ser Phe Val Lys
   1010 1015 1020
   Ser His Ile Arg Pro Tyr Met Asp Glu Ile Val Thr Leu Met Arg Glu
   1025 1030 1035 1040
   Phe Trp Val Met Asn Thr Ser Ile Gln Ser Thr Ile Ile Leu Leu Ile
   1045 1050 1055
   Glu Gln Ile Val Val Ala Leu Gly Gly Glu Phe Lys Leu Tyr Leu Pro
   1060 1065 1070
   Gln Leu Ile Pro His Met Leu Arg Val Phe Met His Asp Asn Ser Pro
   1075 1080 1085
   Gly Arg Ile Val Ser Ile Lys Leu Leu Ala Ala Ile Gln Leu Phe Gly
   1090 1095 1100
   Ala Asn Leu Asp Asp Tyr Leu His Leu Leu Leu Pro Pro Ile Val Lys
   1105 1110 1115 1120
   Leu Phe Asp Ala Pro Glu Ala Pro Leu Pro Ser Arg Lys Ala Ala Leu
   1125 1130 1135
   Glu Thr Val Asp Arg Leu Thr Glu Ser Leu Asp Phe Thr Asp Tyr Ala
   1140 1145 1150
   Ser Arg Ile Ile His Pro Ile Val Arg Thr Leu Asp Gln Ser Pro Glu
   1155 1160 1165
   Leu Arg Ser Thr Ala Met Asp Thr Leu Ser Ser Leu Val Phe Gln Leu
   1170 1175 1180
   Gly Lys Lys Tyr Gln Ile Phe Ile Pro Met Val Asn Lys Val Leu Val
   1185 1190 1195 1200
   Arg His Arg Ile Asn His Gln Arg Tyr Asp Val Leu Ile Cys Arg Ile
   1205 1210 1215
   Val Lys Gly Tyr Thr Leu Ala Asp Glu Glu Glu Asp Pro Leu Ile Tyr
   1220 1225 1230
   Gln His Arg Met Leu Arg Ser Gly Gln Gly Asp Ala Leu Ala Ser Gly
   1235 1240 1245
   Pro Val Glu Thr Gly Pro Met Lys Lys Leu His Val Ser Thr Ile Asn
   1250 1255 1260
   Leu Gln Lys Ala Trp Gly Ala Ala Arg Arg Val Ser Lys Asp Asp Trp
   1265 1270 1275 1280
   Leu Glu Trp Leu Arg Arg Leu Ser Leu Glu Leu Leu Lys Asp Ser Ser
   1285 1290 1295
   Ser Pro Ser Leu Arg Ser Cys Trp Ala Leu Ala Gln Ala Tyr Asn Pro
   1300 1305 1310
   Met Ala Arg Asp Leu Phe Asn Ala Ala Phe Val Ser Cys Trp Ser Glu
   1315 1320 1325
   Leu Asn Glu Asp Gln Gln Asp Glu Leu Ile Arg Ser Ile Glu Leu Ala
   1330 1335 1340
   Leu Thr Ser Gln Asp Ile Ala Glu Val Thr Gln Thr Leu Leu Asn Leu
   1345                1350                1355                1360
   Ala Glu Phe Met Glu His Ser Asp Lys Gly Pro Leu Pro Leu Arg Asp
                   1365                1370                1375
   Asp Asn Gly Ile Val Leu Leu Gly Glu Arg Ala Ala Lys Cys Arg Ala
               1380                1385                1390
   Tyr Ala Lys Ala Leu His Tyr Lys Glu Leu Glu Phe Gln Lys Gly Pro
           1395                1400                1405
   Thr Pro Ala Ile Leu Glu Ser Leu Ile Ser Ile Asn Asn Lys Leu Gln
       1410                1415                1420
   Gln Pro Glu Ala Ala Ala Gly Val Leu Glu Tyr Ala Met Lys His Phe
   1425                1430                1435                1440
   Gly Glu Leu Glu Ile Gln Ala Thr Trp Tyr Glu Lys Leu His Glu Trp
                   1445                1450                1455
   Glu Asp Ala Leu Val Ala Tyr Asp Lys Lys Met Asp Thr Asn Lys Asp
               1460                1465                1470
   Asp Pro Glu Leu Met Leu Gly Arg Met Arg Cys Leu Glu Ala Leu Gly
           1475                1480                1485
   Glu Trp Gly Gln Leu His Gln Gln Cys Cys Glu Lys Trp Thr Leu Val
       1490                1495                1500
   Asn Asp Glu Thr Gln Ala Lys Met Ala Arg Met Ala Ala Ala Ala Ala
   1505                1510                1515                1520
   Trp Gly Leu Gly Gln Trp Asp Ser Met Glu Glu Tyr Thr Cys Met Ile
                   1525                1530                1535
   Pro Arg Asp Thr His Asp Gly Ala Phe Tyr Arg Ala Val Leu Ala Leu
               1540                1545                1550
   His Gln Asp Leu Phe Ser Leu Ala Gln Gln Cys Ile Asp Lys Ala Arg
           1555                1560                1565
   Asp Leu Leu Asp Ala Glu Leu Thr Ala Met Ala Gly Glu Ser Tyr Ser
       1570                1575                1580
   Arg Ala Tyr Gly Ala Met Val Ser Cys His Met Leu Ser Glu Leu Glu
   1585                1590                1595                1600
   Glu Val Ile Gln Tyr Lys Leu Val Pro Glu Arg Arg Glu Ile Ile Arg
                   1605                1610                1615
   Gln Ile Trp Trp Glu Arg Leu Gln Gly Cys Gln Arg Ile Val Glu Asp
               1620                1625                1630
   Trp Gln Lys Ile Leu Met Val Arg Ser Leu Val Val Ser Pro His Glu
           1635                1640                1645
   Asp Met Arg Thr Trp Leu Lys Tyr Ala Ser Leu Cys Gly Lys Ser Gly
       1650                1655                1660
   Arg Leu Ala Leu Ala His Lys Thr Leu Val Leu Leu Leu Gly Val Asp
   1665                1670                1675                1680
   Pro Ser Arg Gln Leu Asp His Pro Leu Pro Thr Val His Pro Gln Val
                   1685                1690                1695
   Thr Tyr Ala Tyr Met Lys Asn Met Trp Lys Ser Ala Arg Lys Ile Asp
               1700                1705                1710
   Ala Phe Gln His Met Gln His Phe Val Gln Thr Met Gln Gln Gln Ala
   1715 1720 1725
   Gln His Ala Ile Ala Thr Glu Asp Gln Gln His Lys Gln Glu Leu His
   1730 1735 1740
   Lys Leu Met Ala Arg Cys Phe Leu Lys Leu Gly Glu Trp Gln Leu Asn
   1745 1750 1755 1760
   Leu Gln Gly Ile Asn Glu Ser Thr Ile Pro Lys Val Leu Gln Tyr Tyr
   1765 1770 1775
   Ser Ala Ala Thr Glu His Asp Arg Ser Trp Tyr Lys Ala Trp His Ala
   1780 1785 1790
   Trp Ala Val Met Asn Phe Glu Ala Val Leu His Tyr Lys His Gln Asn
   1795 1800 1805
   Gln Ala Arg Asp Glu Lys Lys Lys Leu Arg His Ala Ser Gly Ala Asn
   1810 1815 1820
   Ile Thr Asn Ala Thr Thr Ala Ala Thr Thr Ala Ala Thr Ala Thr Thr
   1825 1830 1835 1840
   Thr Ala Ser Thr Glu Gly Ser Asn Ser Glu Ser Glu Ala Glu Ser Thr
   1845 1850 1855
   Glu Asn Ser Pro Thr Pro Ser Pro Leu Gln Lys Lys Val Thr Glu Asp
   1860 1865 1870
   Leu Ser Lys Thr Leu Leu Met Tyr Thr Val Pro Ala Val Gln Gly Phe
   1875 1880 1885
   Phe Arg Ser Ile Ser Leu Ser Arg Gly Asn Asn Leu Gln Asp Thr Leu
   1890 1895 1900
   Arg Val Leu Thr Leu Trp Phe Asp Tyr Gly His Trp Pro Asp Val Asn
   1905 1910 1915 1920
   Glu Ala Leu Val Glu Gly Val Lys Ala Ile Gln Ile Asp Thr Trp Leu
   1925 1930 1935
   Gln Val Ile Pro Gln Leu Ile Ala Arg Ile Asp Thr Pro Arg Pro Leu
   1940 1945 1950
   Val Gly Arg Leu Ile His Gln Leu Leu Thr Asp Ile Gly Arg Tyr His
   1955 1960 1965
   Pro Gln Ala Leu Ile Tyr Pro Leu Thr Val Ala Ser Lys Ser Thr Thr
   1970 1975 1980
   Thr Ala Arg His Asn Ala Ala Asn Lys Ile Leu Lys Asn Met Cys Glu
   1985 1990 1995 2000
   His Ser Asn Thr Leu Val Gln Gln Ala Met Met Val Ser Glu Glu Leu
   2005 2010 2015
   Ile Arg Val Ala Ile Leu Trp His Glu Met Trp His Glu Gly Leu Glu
   2020 2025 2030
   Glu Ala Ser Arg Leu Tyr Phe Gly Glu Arg Asn Val Lys Gly Met Phe
   2035 2040 2045
   Glu Val Leu Glu Pro Leu His Ala Met Met Glu Arg Gly Pro Gln Thr
   2050 2055 2060
   Leu Lys Glu Thr Ser Phe Asn Gln Ala Tyr Gly Arg Asp Leu Met Glu
   2065                    2070                    2075                    2080
   Ala Gln Glu Trp Cys Arg Lys Tyr Met Lys Ser Gly Asn Val Lys Asp
                   2085                    2090                    2095
   Leu Thr Gln Ala Trp Asp Leu Tyr Tyr His Val Phe Arg Arg Ile Ser
               2100                    2105                    2110
   Lys Gln Leu Pro Gln Leu Thr Ser Leu Glu Leu Gln Tyr Val Ser Pro
           2115                    2120                    2125
   Lys Leu Leu Met Cys Arg Asp Leu Glu Leu Ala Val Pro Gly Thr Tyr
       2130                    2135                    2140
   Asp Pro Asn Gln Pro Ile Ile Arg Ile Gln Ser Ile Ala Pro Ser Leu
   2145                    2150                    2155                    2160
   Gln Val Ile Thr Ser Lys Gln Arg Pro Arg Lys Leu Thr Leu Met Gly
                   2165                    2170                    2175
   Ser Asn Gly His Glu Phe Val Phe Leu Leu Lys Gly His Glu Asp Leu
               2180                    2185                    2190
   Arg Gln Asp Glu Arg Val Met Gln Leu Phe Gly Leu Val Asn Thr Leu
           2195                    2200                    2205
   Leu Ala Asn Asp Pro Thr Ser Leu Arg Lys Asn Leu Ser Ile Gln Arg
       2210                    2215                    2220
   Tyr Ala Val Ile Pro Leu Ser Thr Asn Ser Gly Leu Ile Gly Trp Val
   2225                    2230                    2235                    2240
   Pro His Cys Asp Thr Leu His Ala Leu Ile Arg Asp Tyr Arg Glu Lys
                   2245                    2250                    2255
   Lys Lys Ile Leu Leu Asn Ile Glu His Arg Ile Met Leu Arg Met Ala
               2260                    2265                    2270
   Pro Asp Tyr Asp His Leu Thr Leu Met Gln Lys Val Glu Val Phe Glu
           2275                    2280                    2285
   His Ala Val Asn Asn Thr Ala Gly Asp Asp Leu Ala Lys Leu Leu Trp
       2290                    2295                    2300
   Leu Lys Ser Pro Ser Ser Glu Val Trp Phe Asp Arg Arg Thr Asn Tyr
   2305                    2310                    2315                    2320
   Thr Arg Ser Leu Ala Val Met Ser Met Val Gly Tyr Ile Leu Gly Leu
                   2325                    2330                    2335
   Gly Asp Arg His Pro Ser Asn Leu Met Leu Asp Arg Leu Ser Gly Lys
               2340                    2345                    2350
   Ile Leu His Ile Asp Phe Gly Asp Cys Phe Glu Val Ala Met Thr Arg
           2355                    2360                    2365
   Glu Lys Phe Pro Glu Lys Ile Pro Phe Arg Leu Thr Arg Met Leu Thr
       2370                    2375                    2380
   Asn Ala Met Glu Val Thr Gly Leu Asp Gly Asn Tyr Arg Ile Thr Cys
   2385                    2390                    2395                    2400
   His Thr Val Met Glu Val Leu Arg Glu His Lys Asp Ser Val Met Ala
                   2405                    2410                    2415
   Val Leu Glu Ala Phe Val Tyr Asp Pro Leu Leu Asn Trp Arg Leu Met
               2420                    2425                    2430
   Asp Thr Asn Thr Lys Gly Asn Lys Arg Ser Arg Thr Arg Thr Asp Ser
           2435                2440                2445
   Tyr Ser Ala Gly Gln Ser Val Glu Ile Leu Asp Gly Val Glu Leu Gly
       2450                2455                2460
   Glu Pro Ala His Lys Lys Thr Gly Thr Thr Val Pro Glu Ser Ile His
   2465                2470                2475                2480
   Ser Phe Ile Gly Asp Gly Leu Val Lys Pro Glu Ala Leu Asn Lys Lys
                   2485                2490                2495
   Ala Ile Gln Ile Ile Asn Arg Val Arg Asp Lys Leu Thr Gly Arg Asp
               2500                2505                2510
   Phe Ser His Asp Asp Thr Leu Asp Val Pro Thr Gln Val Glu Leu Leu
           2515                2520                2525
   Ile Lys Gln Ala Thr Ser His Glu Asn Leu Cys Gln Cys Tyr Ile Gly
       2530                2535                2540
   Trp Cys Pro Phe Trp
   2545
<210> 3
   <211> 3495
   <212> DNA
   <213> Homo sapiens
<220>
   <221> gene
   <222> (1)..(3495)
   <223> wild type TSC1
<400> 3 
atggcccaac aagcaaatgt cggggagctt cttgccatgc tggactcccc catgctgggt 60
gtgcgggacg acgtgacagc tgtctttaaa gagaacctca attctgaccg tggccctatg 120
cttgtaaaca ccttggtgga ttattacctg gaaaccagct ctcagccggc attgcacatc 180
ctgaccacct tgcaagagcc acatgacaag cacctcttgg acaggattaa cgaatatgtg 240
ggcaaagccg ccactcgttt atccatcctc tcgttactgg gtcatgtcat aagactgcag 300
ccatcttgga agcataagct ctctcaagca cctcttttgc cttctttact aaaatgtctc 360
aagatggaca ctgacgtcgt tgtcctcaca acaggcgtct tggtgttgat aaccatgcta 420
ccaatgattc cacagtctgg gaaacagcat cttcttgatt tctttgacat ttttggccgt 480
ctgtcatcat ggtgcctgaa gaaaccaggc cacgtggcgg aagtctatct cgtccatctc 540
catgccagtg tgtacgcact ctttcatcgc ctttatggaa tgtacccttg caacttcgtc 600
tcctttttgc gttctcatta cagtatgaaa gaaaacctgg agacttttga agaagtggtc 660
aagccaatga tggagcatgt gcgaattcat ccggaattag tgactggatc caaggaccat 720
gaactggacc ctcgaaggtg gaagagatta gaaactcatg atgttgtgat cgagtgtgcc 780
aaaatctctc tggatcccac agaagcctca tatgaagatg gctattctgt gtctcaccaa 840
atctcagccc gctttcctca tcgttcagcc gatgtcacca ccagccctta tgctgacaca 900
cagaatagct atgggtgtgc tacttctacc ccttactcca cgtctcggct gatgttgtta 960
 
aatatgccag ggcagctacc tcagactctg agttccccat cgacacggct gataactgaa 1020
ccaccacaag ctactctttg gagcccatct atggtttgtg gtatgaccac tcctccaact 1080
tctcctggaa atgtcccacc tgatctgtca cacccttaca gtaaagtctt tggtacaact 1140
gcaggtggaa aaggaactcc tctgggaacc ccagcaacct ctcctcctcc agccccactc 1200
tgtcattcgg atgactacgt gcacatttca ctcccccagg ccacagtcac accccccagg 1260
aaggaagaga gaatggattc tgcaagacca tgtctacaca gacaacacca tcttctgaat 1320
gacagaggat cagaagagcc acctggcagc aaaggttctg tcactctaag tgatcttcca 1380
gggtttttag gtgatctggc ctctgaagaa gatagtattg aaaaagataa agaagaagct 1440
gcaatatcta gagaactttc tgagatcacc acagcagagg cagagcctgt ggttcctcga 1500
ggaggctttg actctccctt ttaccgagac agtctcccag gttctcagcg gaagacccac 1560
tcggcagcct ccagttctca gggcgccagc gtgaaccctg agcctttaca ctcctccctg 1620
gacaagcttg ggcctgacac accaaagcaa gcctttactc ccatagacct gccctgcggc 1680
agtgctgatg aaagccctgc gggagacagg gaatgccaga cttctttgga gaccagtatc 1740
ttcactccca gtccttgtaa aattccacct ccgacgagag tgggctttgg aagcgggcag 1800
cctcccccgt atgatcatct ttttgaggtg gcattgccaa agacagccca tcattttgtc 1860
atcaggaaga ctgaggagct gttaaagaaa gcaaaaggaa acacagagga agatggtgtg 1920
ccctctacct ccccaatgga agtgctggac agactgatac agcagggagc agacgcgcac 1980
agcaaggagc tgaacaagtt gcctttaccc agcaagtctg tcgactggac ccactttgga 2040
ggctctcctc cttcagatga gatccgcacc ctccgagacc agttgctttt actgcacaac 2100
cagttactct atgagcgttt taagaggcag cagcatgccc tccggaacag gcggctcctc 2160
cgcaaggtga tcaaagcagc agctctggag gaacataatg ctgccatgaa agatcagttg 2220
aagttacaag agaaggacat ccagatgtgg aaggttagtc tgcagaaaga acaagctaga 2280
tacaatcagc tccaggagca gcgtgacact atggtaacca agctccacag ccagatcaga 2340
cagctgcagc atgaccgaga ggaattctac aaccagagcc aggaattaca gacgaagctg 2400
gaggactgca ggaacatgat tgcggagctg cggatagaac tgaagaaggc caacaacaag 2460
gtgtgtcaca ctgagctgct gctcagtcag gtttcccaaa agctctcaaa cagtgagtcg 2520
gtccagcagc agatggagtt cttgaacagg cagctgttgg ttcttgggga ggtcaacgag 2580
ctctatttgg aacaactgca gaacaagcac tcagatacca caaaggaagt agaaatgatg 2640
aaagccgcct atcggaaaga gctagaaaaa aacagaagcc atgttctcca gcagactcag 2700
aggcttgata cctcccaaaa acggattttg gaactggaat ctcacctggc caagaaagac 2760
caccttcttt tggaacagaa gaaatatcta gaggatgtca aactccaggc aagaggacag 2820
ctgcaggccg cagagagcag gtatgaggct cagaaaagga taacccaggt gtttgaattg 2880
gagatcttag atttatatgg caggttggag aaagatggcc tcctgaaaaa acttgaagaa 2940
gaaaaagcag aagcagctga agcagcagaa gaaaggcttg actgttgtaa tgacgggtgc 3000
 
tcagattcca tggtagggca caatgaagag gcatctggcc acaacggtga gaccaagacc 3060

cccaggccca gcagcgcccg gggcagtagt ggaagcagag gtggtggagg cagcagcagc 3120

agcagcagcg agctttctac cccagagaaa cccccacacc agagggcagg cccattcagc 3180

agtcggtggg agacgactat gggagaagcg tctgccagca tccccaccac tgtgggctca 3240

cttcccagtt caaaaagctt cctgggtatg aaggctcgag agttatttcg taataagagc 3300

gagagccagt gtgatgagga cggcatgacc agtagccttt ctgagagcct aaagacagaa 3360

ctgggcaaag acttgggtgt ggaagccaag attcccctga acctagatgg ccctcacccg 3420

tctcccccga ccccggacag tgttggacag ctacatatca tggactacaa tgagactcat 3480

catgaacaca gctaa 3495
<210> 4
   <211> 1164
   <212> PRT
   <213> Homo sapiens
<220>
   <221> PEPTIDE
   <222> (1)..(1164)
   <223> wild type TSC1
<400> 4
<210> 5
   <211> 5424
   <212> DNA
   <213> Homo sapiens
<220>
   <221> gene
   <222> (1)..(5424)
   <223> wild type TSC2
<400> 5 
atggccaaac caacaagcaa agattcaggc ttgaaggaga agtttaagat tctgttggga 60

ctgggaacac cgaggccaaa tcccaggtct gcagagggta aacagacgga gtttatcatc 120

accgcggaaa tactgagaga actgagcatg gaatgtggcc tcaacaatcg catccggatg 180

atagggcaga tttgtgaagt cgcaaaaacc aagaaatttg aagagcacgc agtggaagca 240
 
ctctggaagg cggtcgcgga tctgttgcag ccggagcggc cgctggaggc ccggcacgcg 300
gtgctggctc tgctgaaggc catcgtgcag gggcagggcg agcgtttggg ggtcctcaga 360
gccctcttct ttaaggtcat caaggattac ccttccaacg aagaccttca cgaaaggctg 420
gaggttttca aggccctcac agacaatggg agacacatca cctacttgga ggaagagctg 480
gctgactttg tcctgcagtg gatggatgtt ggcttgtcct cggaattcct tctggtgctg 540
gtgaacttgg tcaaattcaa tagctgttac ctcgacgagt acatcgcaag gatggttcag 600
atgatctgtc tgctgtgcgt ccggaccgcg tcctctgtgg acatagaggt ctccctgcag 660
gtgctggacg ccgtggtctg ctacaactgc ctgccggctg agagcctccc gctgttcatc 720
gttaccctct gtcgcaccat caacgtcaag gagctctgcg agccttgctg gaagctgatg 780
cggaacctcc ttggcaccca cctgggccac agcgccatct acaacatgtg ccacctcatg 840
gaggacagag cctacatgga ggacgcgccc ctgctgagag gagccgtgtt ttttgtgggc 900
atggctctct ggggagccca ccggctctat tctctcagga actcgccgac atctgtgttg 960
ccatcatttt accaggccat ggcatgtccg aacgaggtgg tgtcctatga gatcgtcctg 1020
tccatcacca ggctcatcaa gaagtatagg aaggagctcc aggtggtggc gtgggacatt 1080
ctgctgaaca tcatcgaacg gctccttcag cagctccaga ccttggacag cccggagctc 1140
aggaccatcg tccatgacct gttgaccacg gtggaggagc tgtgtgacca gaacgagttc 1200
cacgggtctc aggagagata ctttgaactg gtggagagat gtgcggacca gaggcctgag 1260
tcctccctcc tgaacctgat ctcctataga gcgcagtcca tccacccggc caaggacggc 1320
tggattcaga acctgcaggc gctgatggag agattcttca ggagcgagtc ccgaggcgcc 1380
gtgcgcatca aggtgctgga cgtgctgtcc tttgtgctgc tcatcaacag gcagttctat 1440
gaggaggagc tgattaactc agtggtcatc tcgcagctct cccacatccc cgaggataaa 1500
gaccaccagg tccgaaagct ggccacccag ttgctggtgg acctggcaga gggctgccac 1560
acacaccact tcaacagcct gctggacatc atcgagaagg tgatggcccg ctccctctcc 1620
ccacccccgg agctggaaga aagggatgtg gccgcatact cggcctcctt ggaggatgtg 1680
aagacagccg tcctggggct tctggtcatc cttcagacca agctgtacac cctgcctgca 1740
agccacgcca cgcgtgtgta tgagatgctg gtcagccaca ttcagctcca ctacaagcac 1800
agctacaccc tgccaatcgc gagcagcatc cggctgcagg cctttgactt cctgttgctg 1860
ctgcgggccg actcactgca ccgcctgggc ctgcccaaca aggatggagt cgtgcggttc 1920
agcccctact gcgtctgcga ctacatggag ccagagagag gctctgagaa gaagaccagc 1980
ggcccccttt ctcctcccac agggcctcct ggcccggcgc ctgcaggccc cgccgtgcgg 2040
ctggggtccg tgccctactc cctgctcttc cgcgtcctgc tgcagtgctt gaagcaggag 2100
tctgactgga aggtgctgaa gctggttctg ggcaggctgc ctgagtccct gcgctataaa 2160
gtgctcatct ttacttcccc ttgcagtgtg gaccagctgt gctctgctct ctgctccatg 2220
ctttcaggcc caaagacact ggagcggctc cgaggcgccc cagaaggctt ctccagaact 2280
 
gacttgcacc tggccgtggt tccagtgctg acagcattaa tctcttacca taactacctg 2340
gacaaaacca aacagcgcga gatggtctac tgcctggagc agggcctcat ccaccgctgt 2400
gccagccagt gcgtcgtggc cttgtccatc tgcagcgtgg agatgcctga catcatcatc 2460
aaggcgctgc ctgttctggt ggtgaagctc acgcacatct cagccacagc cagcatggcc 2520
gtcccactgc tggagttcct gtccactctg gccaggctgc cgcacctcta caggaacttt 2580
gccgcggagc agtatgccag tgtgttcgcc atctccctgc cgtacaccaa cccctccaag 2640
tttaatcagt acatcgtgtg tctggcccat cacgtcatag ccatgtggtt catcaggtgc 2700
cgcctgccct tccggaagga ttttgtccct ttcatcacta agggcctgcg gtccaatgtc 2760
ctcttgtctt ttgatgacac ccccgagaag gacagcttca gggcccggag tactagtctc 2820
aacgagagac ccaagagtct gaggatagcc agacccccca aacaaggctt gaataactct 2880
ccacccgtga aagaattcaa ggagagctct gcagccgagg ccttccggtg ccgcagcatc 2940
agtgtgtctg aacatgtggt ccgcagcagg atacagacgt ccctcaccag tgccagcttg 3000
gggtctgcag atgagaactc cgtggcccag gctgacgata gcctgaaaaa cctccacctg 3060
gagctcacgg aaacctgtct ggacatgatg gctcgatacg tcttctccaa cttcacggct 3120
gtcccgaaga ggtctcctgt gggcgagttc ctcctagcgg gtggcaggac caaaacctgg 3180
ctggttggga acaagcttgt cactgtgacg acaagcgtgg gaaccgggac ccggtcgtta 3240
ctaggcctgg actcggggga gctgcagtcc ggcccggagt cgagctccag ccccggggtg 3300
catgtgagac agaccaagga ggcgccggcc aagctggagt cccaggctgg gcagcaggtg 3360
tcccgtgggg cccgggatcg ggtccgttcc atgtcggggg gccatggtct tcgagttggc 3420
gccctggacg tgccggcctc ccagttcctg ggcagtgcca cttctccagg accacggact 3480
gcaccagccg cgaaacctga gaaggcctca gctggcaccc gggttcctgt gcaggagaag 3540
acgaacctgg cggcctatgt gcccctgctg acccagggct gggcggagat cctggtccgg 3600
aggcccacag ggaacaccag ctggctgatg agcctggaga acccgctcag ccctttctcc 3660
tcggacatca acaacatgcc cctgcaggag ctgtctaacg ccctcatggc ggctgagcgc 3720
ttcaaggagc accgggacac agccctgtac aagtcactgt cggtgccggc agccagcacg 3780
gccaaacccc ctcctctgcc tcgctccaac acagtggcct ctttctcctc cctgtaccag 3840
tccagctgcc aaggacagct gcacaggagc gtttcctggg cagactccgc cgtggtcatg 3900
gaggagggaa gtccgggcga ggttcctgtg ctggtggagc ccccagggtt ggaggacgtt 3960
gaggcagcgc taggcatgga caggcgcacg gatgcctaca gcaggtcgtc ctcagtctcc 4020
agccaggagg agaagtcgct ccacgcggag gagctggttg gcaggggcat ccccatcgag 4080
cgagtcgtct cctcggaggg tggccggccc tctgtggacc tctccttcca gccctcgcag 4140
cccctgagca agtccagctc ctctcccgag ctgcagactc tgcaggacat cctcggggac 4200
cctggggaca aggccgacgt gggccggctg agccctgagg ttaaggcccg gtcacagtca 4260
gggaccctgg acggggaaag tgctgcctgg tcggcctcgg gcgaagacag tcggggccag 4320
 
cccgagggtc ccttgccttc cagctccccc cgctcgccca gtggcctccg gccccgaggt 4380
tacaccatct ccgactcggc cccatcacgc aggggcaaga gagtagagag ggacgcctta 4440
aagagcagag ccacagcctc caatgcagag aaagtgccag gcatcaaccc cagtttcgtg 4500
ttcctgcagc tctaccattc ccccttcttt ggcgacgagt caaacaagcc aatcctgctg 4560
cccaatgagt cacagtcctt tgagcggtcg gtgcagctcc tcgaccagat cccatcatac 4620
gacacccaca agatcgccgt cctgtatgtt ggagaaggcc agagcaacag cgagctcgcc 4680
atcctgtcca atgagcatgg ctcctacagg tacacggagt tcctgacggg cctgggccgg 4740
ctcatcgagc tgaaggactg ccagccggac aaggtgtacc tgggaggcct ggacgtgtgt 4800
ggtgaggacg gccagttcac ctactgctgg cacgatgaca tcatgcaagc cgtcttccac 4860
atcgccaccc tgatgcccac caaggacgtg gacaagcacc gctgcgacaa gaagcgccac 4920
ctgggcaacg actttgtgtc cattgtctac aatgactccg gtgaggactt caagcttggc 4980
accatcaagg gccagttcaa ctttgtccac gtgatcgtca ccccgctgga ctacgagtgc 5040
aacctggtgt ccctgcagtg caggaaagac atggagggcc ttgtggacac cagcgtggcc 5100
aagatcgtgt ctgaccgcaa cctgcccttc gtggcccgcc agatggccct gcacgcaaat 5160
atggcctcac aggtgcatca tagccgctcc aaccccaccg atatctaccc ctccaagtgg 5220
attgcccggc tccgccacat caagcggctc cgccagcgga tctgcgagga agccgcctac 5280
tccaacccca gcctacctct ggtgcaccct ccgtcccata gcaaagcccc tgcacagact 5340
ccagccgagc ccacacctgg ctatgaggtg ggccagcgga agcgcctcat ctcctcggtg 5400
gaggacttca ccgagtttgt gtga 5424
<210> 6
   <211> 1807
   <212> PRT
   <213> Homo sapiens
<220>
   <221> PEPTIDE
   <222> (1)..(1807)
   <223> wild type TSC2
<400> 6
<210> 7
   <211> 1398
   <212> DNA
   <213> Homo sapiens
<220>
   <221> gene
   <222> (1)..(1398)
   <223> wild type AKT3
<400> 7 
atgagcgatg ttaccattgt gaaagaaggt tgggttcaga agaggggaga atatataaaa 60

aactggaggc caagatactt ccttttgaag acagatggct cattcatagg atataaagag 120
 
aaacctcaag atgtggattt accttatccc ctcaacaact tttcagtggc aaaatgccag 180
ttaatgaaaa cagaacgacc aaagccaaac acatttataa tcagatgtct ccagtggact 240
actgttatag agagaacatt tcatgtagat actccagagg aaagggaaga atggacagaa 300
gctatccagg ctgtagcaga cagactgcag aggcaagaag aggagagaat gaattgtagt 360
ccaacttcac aaattgataa tataggagag gaagagatgg atgcctctac aacccatcat 420
aaaagaaaga caatgaatga ttttgactat ttgaaactac taggtaaagg cacttttggg 480
aaagttattt tggttcgaga gaaggcaagt ggaaaatact atgctatgaa gattctgaag 540
aaagaagtca ttattgcaaa ggatgaagtg gcacacactc taactgaaag cagagtatta 600
aagaacacta gacatccctt tttaacatcc ttgaaatatt ccttccagac aaaagaccgt 660
ttgtgttttg tgatggaata tgttaatggg ggcgagctgt ttttccattt gtcgagagag 720
cgggtgttct ctgaggaccg cacacgtttc tatggtgcag aaattgtctc tgccttggac 780
tatctacatt ccggaaagat tgtgtaccgt gatctcaagt tggagaatct aatgctggac 840
aaagatggcc acataaaaat tacagatttt ggactttgca aagaagggat cacagatgca 900
gccaccatga agacattctg tggcactcca gaatatctgg caccagaggt gttagaagat 960
aatgactatg gccgagcagt agactggtgg ggcctagggg ttgtcatgta tgaaatgatg 1020
tgtgggaggt tacctttcta caaccaggac catgagaaac tttttgaatt aatattaatg 1080
gaagacatta aatttcctcg aacactctct tcagatgcaa aatcattgct ttcagggctc 1140
ttgataaagg atccaaataa acgccttggt ggaggaccag atgatgcaaa agaaattatg 1200
agacacagtt tcttctctgg agtaaactgg caagatgtat atgataaaaa gcttgtacct 1260
ccttttaaac ctcaagtaac atctgagaca gatactagat attttgatga agaatttaca 1320
gctcagacta ttacaataac accacctgaa aaatgtcagc aatcagattg tggcatgctg 1380
ggtaactgga aaaaataa 1398
<210> 8
   <211> 465
   <212> PRT
   <213> Homo sapiens
<220>
   <221> PEPTIDE
   <222> (1)..(465)
   <223> wild type AKT3
<400> 8 
Met Ser Asp Val Thr Ile Val Lys Glu Gly Trp Val Gln Lys Arg Gly
1               5                   10                  15

Glu Tyr Ile Lys Asn Trp Arg Pro Arg Tyr Phe Leu Leu Lys Thr Asp
            20                  25                  30

Gly Ser Phe Ile Gly Tyr Lys Glu Lys Pro Gln Asp Val Asp Leu Pro
        35                  40                  45

Tyr Pro Leu Asn Asn Phe Ser Val Ala Lys Cys Gln Leu Met Lys Thr
 
50                  55                  60

Glu Arg Pro Lys Pro Asn Thr Phe Ile Ile Arg Cys Leu Gln Trp Thr
65                  70                  75                  80

Thr Val Ile Glu Arg Thr Phe His Val Asp Thr Pro Glu Glu Arg Glu
                85                  90                  95

Glu Trp Thr Glu Ala Ile Gln Ala Val Ala Asp Arg Leu Gln Arg Gln
            100                 105                 110

Glu Glu Glu Arg Met Asn Cys Ser Pro Thr Ser Gln Ile Asp Asn Ile
        115                 120                 125

Gly Glu Glu Glu Met Asp Ala Ser Thr Thr His His Lys Arg Lys Thr
    130                 135                 140

Met Asn Asp Phe Asp Tyr Leu Lys Leu Leu Gly Lys Gly Thr Phe Gly
145                 150                 155                 160

Lys Val Ile Leu Val Arg Glu Lys Ala Ser Gly Lys Tyr Tyr Ala Met
                165                 170                 175

Lys Ile Leu Lys Lys Glu Val Ile Ile Ala Lys Asp Glu Val Ala His
            180                 185                 190

Thr Leu Thr Glu Ser Arg Val Leu Lys Asn Thr Arg His Pro Phe Leu
        195                 200                 205

Thr Ser Leu Lys Tyr Ser Phe Gln Thr Lys Asp Arg Leu Cys Phe Val
    210                 215                 220

Met Glu Tyr Val Asn Gly Gly Glu Leu Phe Phe His Leu Ser Arg Glu
225                 230                 235                 240

Arg Val Phe Ser Glu Asp Arg Thr Arg Phe Tyr Gly Ala Glu Ile Val
                245                 250                 255

Ser Ala Leu Asp Tyr Leu His Ser Gly Lys Ile Val Tyr Arg Asp Leu
            260                 265                 270

Lys Leu Glu Asn Leu Met Leu Asp Lys Asp Gly His Ile Lys Ile Thr
        275                 280                 285

Asp Phe Gly Leu Cys Lys Glu Gly Ile Thr Asp Ala Ala Thr Met Lys
    290                 295                 300

Thr Phe Cys Gly Thr Pro Glu Tyr Leu Ala Pro Glu Val Leu Glu Asp
305                 310                 315                 320

Asn Asp Tyr Gly Arg Ala Val Asp Trp Trp Gly Leu Gly Val Val Met
                325                 330                 335

Tyr Glu Met Met Cys Gly Arg Leu Pro Phe Tyr Asn Gln Asp His Glu
            340                 345                 350

Lys Leu Phe Glu Leu Ile Leu Met Glu Asp Ile Lys Phe Pro Arg Thr
        355                 360                 365

Leu Ser Ser Asp Ala Lys Ser Leu Leu Ser Gly Leu Leu Ile Lys Asp
    370                 375                 380

Pro Asn Lys Arg Leu Gly Gly Gly Pro Asp Asp Ala Lys Glu Ile Met
385                 390                 395                 400

Arg His Ser Phe Phe Ser Gly Val Asn Trp Gln Asp Val Tyr Asp Lys
                405                 410                 415
 
Lys Leu Val Pro Pro Phe Lys Pro Gln Val Thr Ser Glu Thr Asp Thr
            420                 425                 430

Arg Tyr Phe Asp Glu Glu Phe Thr Ala Gln Thr Ile Thr Ile Thr Pro
        435                 440                 445

Pro Glu Lys Cys Gln Gln Ser Asp Cys Gly Met Leu Gly Asn Trp Lys
    450                 455                 460

Lys
465
<210> 9
   <211> 3207
   <212> DNA
   <213> Homo sapiens
<220>
   <221> gene
   <222> (1)..(3207)
   <223> wild type PIK3CA
<400> 9 
atgcctccac gaccatcatc aggtgaactg tggggcatcc acttgatgcc cccaagaatc 60
ctagtagaat gtttactacc aaatggaatg atagtgactt tagaatgcct ccgtgaggct 120
acattaataa ccataaagca tgaactattt aaagaagcaa gaaaataccc cctccatcaa 180
cttcttcaag atgaatcttc ttacattttc gtaagtgtta ctcaagaagc agaaagggaa 240
gaattttttg atgaaacaag acgactttgt gaccttcggc tttttcaacc ctttttaaaa 300
gtaattgaac cagtaggcaa ccgtgaagaa aagatcctca atcgagaaat tggttttgct 360
atcggcatgc cagtgtgtga atttgatatg gttaaagatc cagaagtaca ggacttccga 420
agaaatattc tgaacgtttg taaagaagct gtggatctta gggacctcaa ttcacctcat 480
agtagagcaa tgtatgtcta tcctccaaat gtagaatctt caccagaatt gccaaagcac 540
atatataata aattagataa agggcaaata atagtggtga tctgggtaat agtttctcca 600
aataatgaca agcagaagta tactctgaaa atcaaccatg actgtgtacc agaacaagta 660
attgctgaag caatcaggaa aaaaactcga agtatgttgc tatcctctga acaactaaaa 720
ctctgtgttt tagaatatca gggcaagtat attttaaaag tgtgtggatg tgatgaatac 780
ttcctagaaa aatatcctct gagtcagtat aagtatataa gaagctgtat aatgcttggg 840
aggatgccca atttgatgtt gatggctaaa gaaagccttt attctcaact gccaatggac 900
tgttttacaa tgccatctta ttccagacgc atttccacag ctacaccata tatgaatgga 960
gaaacatcta caaaatccct ttgggttata aatagtgcac tcagaataaa aattctttgt 1020
gcaacctacg tgaatgtaaa tattcgagac attgataaga tctatgttcg aacaggtatc 1080
taccatggag gagaaccctt atgtgacaat gtgaacactc aaagagtacc ttgttccaat 1140
cccaggtgga atgaatggct gaattatgat atatacattc ctgatcttcc tcgtgctgct 1200
cgactttgcc tttccatttg ctctgttaaa ggccgaaagg gtgctaaaga ggaacactgt 1260
ccattggcat ggggaaatat aaacttgttt gattacacag acactctagt atctggaaaa 1320
 
atggctttga atctttggcc agtacctcat ggattagaag atttgctgaa ccctattggt 1380
gttactggat caaatccaaa taaagaaact ccatgcttag agttggagtt tgactggttc 1440
agcagtgtgg taaagttccc agatatgtca gtgattgaag agcatgccaa ttggtctgta 1500
tcccgagaag caggatttag ctattcccac gcaggactga gtaacagact agctagagac 1560
aatgaattaa gggaaaatga caaagaacag ctcaaagcaa tttctacacg agatcctctc 1620
tctgaaatca ctgagcagga gaaagatttt ctatggagtc acagacacta ttgtgtaact 1680
atccccgaaa ttctacccaa attgcttctg tctgttaaat ggaattctag agatgaagta 1740
gcccagatgt attgcttggt aaaagattgg cctccaatca aacctgaaca ggctatggaa 1800
cttctggact gtaattaccc agatcctatg gttcgaggtt ttgctgttcg gtgcttggaa 1860
aaatatttaa cagatgacaa actttctcag tatttaattc agctagtaca ggtcctaaaa 1920
tatgaacaat atttggataa cttgcttgtg agatttttac tgaagaaagc attgactaat 1980
caaaggattg ggcacttttt cttttggcat ttaaaatctg agatgcacaa taaaacagtt 2040
agccagaggt ttggcctgct tttggagtcc tattgtcgtg catgtgggat gtatttgaag 2100
cacctgaata ggcaagtcga ggcaatggaa aagctcatta acttaactga cattctcaaa 2160
caggagaaga aggatgaaac acaaaaggta cagatgaagt ttttagttga gcaaatgagg 2220
cgaccagatt tcatggatgc tctacagggc tttctgtctc ctctaaaccc tgctcatcaa 2280
ctaggaaacc tcaggcttga agagtgtcga attatgtcct ctgcaaaaag gccactgtgg 2340
ttgaattggg agaacccaga catcatgtca gagttactgt ttcagaacaa tgagatcatc 2400
tttaaaaatg gggatgattt acggcaagat atgctaacac ttcaaattat tcgtattatg 2460
gaaaatatct ggcaaaatca aggtcttgat cttcgaatgt taccttatgg ttgtctgtca 2520
atcggtgact gtgtgggact tattgaggtg gtgcgaaatt ctcacactat tatgcaaatt 2580
cagtgcaaag gcggcttgaa aggtgcactg cagttcaaca gccacacact acatcagtgg 2640
ctcaaagaca agaacaaagg agaaatatat gatgcagcca ttgacctgtt tacacgttca 2700
tgtgctggat actgtgtagc taccttcatt ttgggaattg gagatcgtca caatagtaac 2760
atcatggtga aagacgatgg acaactgttt catatagatt ttggacactt tttggatcac 2820
aagaagaaaa aatttggtta taaacgagaa cgtgtgccat ttgttttgac acaggatttc 2880
ttaatagtga ttagtaaagg agcccaagaa tgcacaaaga caagagaatt tgagaggttt 2940
caggagatgt gttacaaggc ttatctagct attcgacagc atgccaatct cttcataaat 3000
cttttctcaa tgatgcttgg ctctggaatg ccagaactac aatcttttga tgacattgca 3060
tacattcgaa agaccctagc cttagataaa actgagcaag aggctttgga gtatttcatg 3120
aaacaaatga atgatgcaca tcatggtggc tggacaacaa aaatggattg gatcttccac 3180
acaattaaac agcatgcatt gaactga 3207
<210> 10
   <211> 1068
   <212> PRT
   <213> Homo sapiens
<220>
   <221> PEPTIDE
   <222> (1)..(1068)
   <223> wild type PIK3CA
<400> 10
<210> 11
   <211> 20
   <212> DNA
   <213> Artificial Sequence
<220>
   <223> C1483 sense primer
<400> 11
   taggttacag gcctggatgg 20
<210> 12
   <211> 20
   <212> DNA
   <213> Artificial Sequence
<220>
   <223> C1483 antisense primer
<400> 12
   cttggcctcc caaaatgtta 20
<210> 13
   <211> 21
   <212> DNA
   <213> Artificial Sequence
<220>
   <223> L2427 sense primer
<400> 13
   tccaggctac ctggtatgag a 21
<210> 14
   <211> 20
   <212> DNA
   <213> Artificial Sequence
<220>
   <223> L2427 antisense primer
<400> 14
   gccttccttt caaatccaaa 20
<210> 15
   <211> 36
   <212> DNA
   <213> Artificial Sequence
<220>
   <223> annealing forward primer for pCIG-mTOR mutant-IRES-EGFP
<400> 15
   aattccaatt gcccgggctt aagatcgata cgcgta 36
<210> 16
   <211> 36
   <212> DNA
   <213> Artificial Sequence
<220>
   <223> annealing reverse primer for pCIG-mTOR mutant-IRES-EGFP
<400> 16
   ccggtacgcg tatcgatctt aagcccgggc aattgg 36
<210> 17
   <211> 50
   <212> DNA
   <213> Artificial Sequence
<220>
   <223> forward primer for pCIG-mTOR mutant-IRES-EGFP
<400> 17
   gatcacaatt gtggccacca tggactacaa ggacgacgat gacaagatgc 50
<210> 18
   <211> 41
   <212> DNA
   <213> Artificial Sequence
<220>
   <223> reverse primer for pCIG-mTOR mutant-IRES-EGFP
<400> 18
   tgatcaacgc gtttaccaga aagggcacca gccaatatag c 41
<210> 19
   <211> 33
   <212> DNA
   <213> Artificial Sequence
<220>
   <223> Y1450D sense primer
<400> 19
   tcgtgcagtt tctcatccca ggtagcctgg atc 33
<210> 20
   <211> 33
   <212> DNA
   <213> Artificial Sequence
<220>
   <223> Y1450D antisense primer
<400> 20
   gatccaggct acctgggatg agaaactgca cga 33
<210> 21
   <211> 23
   <212> DNA
   <213> Artificial Sequence
<220>
   <223> C1483R sense primer
<400> 21
   ggcctcgagg cggcgcatgc ggc 23
<210> 22
   <211> 23
   <212> DNA
   <213> Artificial Sequence
<220>
   <223> C1483R antisense primer
<400> 22
   gccgcatgcg ccgcctcgag gcc 23
<210> 23
   <211> 33
   <212> DNA
   <213> Artificial Sequence
<220>
   <223> L2427Q sense primer
<400> 23
   gtctatgacc ccttgcagaa ctggaggctg atg 33
<210> 24
   <211> 33
   <212> DNA
   <213> Artificial Sequence
<220>
   <223> L2427Q antisense primer
<400> 24
   catcagcctc cagttctgca aggggtcata gac 33
<210> 25
   <211> 33
   <212> DNA
   <213> Artificial Sequence
<220>
   <223> L2427P sense primer
<400> 25
   gtctatgacc ccttgccgaa ctggaggctg atg 33
<210> 26
   <211> 33
   <212> DNA
   <213> Artificial Sequence
<220>
   <223> L2427P antisense primer
<400> 26
   catcagcctc cagttcggca aggggtcata gac 33
<210> 27
   <211> 21
   <212> DNA
   <213> Artificial Sequence
<220>
   <223> 7280T sense primer
<400> 27
   cccaggcact tgatgatact c 21
<210> 28
   <211> 20
   <212> DNA
   <213> Artificial Sequence
<220>
   <223> 7280T antisense primer
<400> 28
   cttgctttgg gtggagagtt 20
<210> 29
   <211> 27
   <212> DNA
   <213> Artificial Sequence
<220>
   <223> TSC-1 R22W-F primer
<400> 29
   gtcacgtcgt cccacacacc cagcatg 27
<210> 30
   <211> 27
   <212> DNA
   <213> Artificial Sequence
<220>
   <223> TSC-1 R22W-R primer
<400> 30
   catgctgggt gtgtgggacg acgtgac 27
<210> 31
   <211> 43
   <212> DNA
   <213> Artificial Sequence
<220>
   <223> TSC-1 R204C-F primer
<400> 31
   ctttcatact gtaatgagaa cacaaaaagg agacgaagtt gca 43
<210> 32
   <211> 43
   <212> DNA
   <213> Artificial Sequence
<220>
   <223> TSC-1 R204C-R primer
<400> 32
   tgcaacttcg tctccttttt gtgttctcat tacagtatga aag 43
<210> 33
   <211> 33
   <212> DNA
   <213> Artificial Sequence
<220>
   <223> TSC-2 V15471-F primer
<400> 33
   tctccaacat acaggatggc gatcttgtgg gtg 33
<210> 34
   <211> 33
   <212> DNA
   <213> Artificial Sequence
<220>
   <223> TSC-2 V15471-R primer
<400> 34
   cacccacaag atcgccatcc tgtatgttgg aga 33
<210> 35
   <211> 35
   <212> DNA
   <213> Artificial Sequence
<220>
   <223> AKT3 R247H-F primer
<400> 35
   caccatagaa acgtgtgtgg tcctcagaga acacc 35
<210> 36
   <211> 35
   <212> DNA
   <213> Artificial Sequence
<220>
   <223> AKT3 R247H-R primer
<400> 36
   ggtgttctct gaggaccaca cacgtttcta tggtg 35
<210> 37
   <211> 20
   <212> DNA
   <213> Artificial Sequence
<220>
   <223> DNA sequence corresponding to TSC1 targetting sgRNA
<400> 37
   tgctggactc ctccacactg 20
<210> 38
   <211> 20
   <212> DNA
   <213> Artificial Sequence
<220>
   <223> DNA sequence corresponding to TSC2 targetting sgRNA
<400> 38
   aatcccaggt gtgcagaagg 20

## Description

### Technical Field

The invention relates to an mTOR inhibitor compound for use in prevention or treatment of Focal Cortical Dysplasia (FCD) type II or an epilepsy associated with FCD type II, wherein the FCD type II is caused by brain somatic mutation of mTOR, TSC1, TSC2, AKT3, or PIK3CA.

### Background Art

Epilepsy is a group of chronic neurological diseases characterized by repetitive seizures induced by excessive electric activity in some neurons within a short time, with the consequent incurrence of neurobiological, mental, perceptual, and social changes.

Epilepsy that is insensitive to anti-epileptic drugs developed thus far is called intractable epilepsy, and accounts for about 20% of all epilepsy cases diagnosed each year. Malformations of cortical developments (MCD) such as Focal Cortical Dysplasia (FCD), Hemimegalencephaly (HME) and Tuberous Sclerosis Complex (TSC), Hippocampal Sclerosis (HS), and Sturge-Weber Syndrome (SWS) are known as common causes of intractable epilepsy.

Insensitive to available antiepileptic drugs, intractable epilepsy requires neurosurgical treatment to resect a brain lesion to control epilepsy. Hence, there is a need for a technique for molecular biological diagnosis specific for malformations of cortical development or Hippocampal Sclerosis, both causative of intractable epilepsy.

FCD is an important cause of epilepsy that is difficult to control with available antiepileptic drugs, and this condition accounts for as much as 50% of juvenile patients undergoing epilepsy surgery. FCD is a sporadic developmental malformation of the cerebral cortex and is characterized by the disorganized structure of the cerebral cortex or cytological abnormalities ofthe neurons in focally affected regions.

Although surgical resection of FCD renders approximately 60% of patients seizure free, a significant portion of Focal Cortical Dysplasia patients continues to have seizures. Due to the lack of understanding of the molecular genetic etiology, the development of novel and more effective FCD therapies remains elusive. A long-held but unproven hypothesis is that FCD is caused by somatic mutations occurring in affected brain regions during brain development. However, no such mutations have been identified.

FCD can be classified into several types based on histopathology. In particular, FCD type II (FCDII) shows a homogenous pathology, characterized by disrupted cortical lamination, and dysmorphic neurons or balloon cells (Epilepsia 52, 158-174 (2011)). FCDII is diagnosed in 29-39% of all FCD patients who undergo epilepsy surgery (Brain 129, 1907-1916 (2006)). Although an association between human papilloma virus and FCDII has been reported, the molecular genetic etiology of FCDII remains poorly understood. Interestingly, the brain MRIs of FCDII patients are occasionally reported to be normal; however, microscopic examinations of surgical specimens reveal scattered dysmorphic cells surrounded by an abundance of normally appearing cells. These radiological and histopathological findings suggest that only a small fraction of cells containing somatic mutations exist in surgically resected tissue, and that these mutations might not be efficiently detected through capillary Sanger sequencing or typical whole exome sequencing (WES) with an average read depth of 100-150x.

Ljungberg M. C. et al., "Rapamycin suppresses seizures and neuronal hypertrophy in a mouse model of cortical dysplasia", Disease Models & Mechanisms, vol. 2, no. 7-8, pages 389-398, disclose the treatment of a mouse model of focal cortical dysplasia with the mTOR inhibitor rapamycin.

Cardamone M. et al., "Mammalian Target of Rapamycin Inhibitors for Intractable Epilepsy and Subependymal Giant Cell Astrocytomas in Tuberous Sclerosis Complex", Journal of Pediatrics, vol. 164, no. 5, pages 1195-1200, disclose treatment of children and adolescents with tuberous sclerosis complex (TSC)-associated intractable epilepsy with the mTOR inhibitors sirolimus and everolimus.

Curatolo P. et al., "mTOR inhibitors as a new therapeutic option for epilepsy", Expert Review of Neurotherapeutics, vol. 13, no. 6, pages 627-638, disclose mTOR inhibition as a therapy for epilepsy associated with TSC and FCD.

US 2015/0065380 A1 discloses mTOR proteins with mutations associated with intractable epilepsy.

Zeng, L.-H. et al., "Rapamycin prevents epilepsy in a mouse model of tuberous sclerosis complex", Annals of Neurology, vol. 63, no. 4, pages 444-453, disclose the effects of Rapamycin in a mouse model of TSC.

Against this background, the present inventors have identified FCD-specific somatic mutations in the brain tissues of FCD patients undergoing epilepsy surgery, using deep sequencing strategies such as deep whole exome sequencing, hybrid capture sequencing, and amplicon sequencing, established a transgenic animal with FCD, using the somatic mutations, and found that the administration of an mTOR inhibitor to the transgenic animal restrains FCD symptoms. The invention is defined in the appended claims.

### Technical Problem

An object of the present disclosure is to provide a method for preventing, ameliorating or treating intractable epilepsy caused by the brain somatic mutations in components of the PI3K-AKT-mTOR pathway, or by Focal Cortical Dysplasia (FCD), Hemimegalencephaly (HME), Hippocampal Sclerosis (HS) or Sturge-Weber Syndrome (SWS), using an mTOR inhibitor as an active ingredient.

Another object of the present disclosure is to provide the use of an mTOR in preventing, ameliorating, or treating intractable epilepsy or a disease causing intractable epilepsy. Here, the intractable epilepsy may be caused by FCD. In particular, FCD may be associated with cerebral somatic mutation.

Further disclosed is a pharmaceutical or food composition for preventing, ameliorating or treating intractable epilepsy caused by brain somatic mutations in components of the PI3K-AKT-mTOR pathway or by Focal Cortical Dysplasia (FCD), Hemimegalencephaly (HME), Hippocampal Sclerosis (HS) or Sturge-Weber Syndrome (SWS), comprising an mTOR inhibitor as an active ingredient.

Further disclosed is a diagnostic kit for intractable epilepsy, comprising an agent capable of detecting a mutation present in a gene or protein involved in a PI3K-AKT-mTOR pathway.

Further disclosed is a method for diagnosing intractable epilepsy, comprising detecting a mutation present in a gene or protein involved in the PI3K-AKT-mTOR pathway in a sample from a subject, using the diagnostic kit.

Further disclosed is a mutant of a gene or protein involved in the PI3K-AKT-mTOR pathway.

Further disclosed is a biomarker panel for the diagnosis of intractable epilepsy, comprising a mutant of a gene or protein involved in the PI3K-AKT-mTOR pathway.

Further disclosed is a composition for inducing intractable epilepsy, comprising a mutant of a gene or protein involved in the PI3K-AKT-mTOR pathway.

Further disclosed is a transgenic animal with intractable epilepsy, into which a gene or protein involved in the PI3K-AKT-mTOR pathway is introduced.

Further disclosed is a method for inducing intractable epilepsy, comprising introducing a gene or protein involved in the PI3K-AKT-mTOR pathway into cells, *ex vivo.*

### Technical Solution

The invention provides an mTOR inhibitor compound for use in prevention or treatment of Focal Cortical Dysplasia (FCD) type II or an epilepsy associated with FCD type II, wherein the FCD type II is caused by brain somatic mutation of mTOR, TSC1, TSC2, AKT3, or PIK3CA.

Further disclosed is a biomarker panel for the diagnosis of intractable epilepsy, and a diagnostic method of intractable epilepsy using the same. Within the scope of diseases causative of intractable epilepsy malformations of cortical developments (MCD) such as Focal Cortical Dysplasia (particularly, FCD type II), Hemimegalencephaly (HME) and Tuberous Sclerosis Complex (TSC), Hippocampal Sclerosis (HS), and Sturge-Weber Syndrome (SWS) fall.

Hereinafter, a detailed description will be given of the present invention.

Intensive and thorough research and analysis of tissue samples resected from brains of patients with intractable epilepsy caused by Focal Cortical Dysplasia (FCD), Hemimegalencephaly (HME), Hippocampal Sclerosis (HS), or Sturge-Weber Syndrome (SWS) resulted in the finding that brain somatic mutations specific for components of the PI3K-AKT-mTOR pathway exist and that the mutations can be used as a biomarker panel for diagnosing intractable epilepsy. Also, the present inventors discovered the fact that cells into which the mutations are introduced overexpress mTOR, inducing intractable epilepsy, and succeeded in developing a method useful in preventing, ameliorating or treating Focal Cortical Dysplasia (FCD), Tuberous Sclerosis Complex (TSC), Hemimegalencephaly (HME), Hippocampal Sclerosis (HS) or Sturge-Weber Syndrome (SWS), and the intractable epilepsy caused by Focal Cortical Dysplasia (FCD), Tuberous Sclerosis Complex (TSC), Hemimegalencephaly (HME), Hippocampal Sclerosis (HS) or Sturge-Weber Syndrome (SWS).

As described in the following, brain tissue, saliva and blood samples were obtained from FCD-caused intractable epilepsy patients undergoing epilepsy surgery, and nucleotide sequencing revealed nine different mTOR gene mutations specifically present in patients with FCD-caused intractable epilepsy, nine mTOR protein mutations corresponding thereto, and six different genetic mutations in components of the PI3K-AKT-mTOR pathway and six corresponding protein mutations corresponding thereto (Table 1).

**TABLE 1**

| No. | Gene | Mutation on | | Remark |
|---|---|---|---|---|
| | | mTOR Gene | mTOR Protein | |
| 1 | mTOR | C616T | R206C | Cytosine (C) at position 616 → Thymine (T)) |
| | | | | Arginine (R) at position 206→ Cysteine (C |
| 2 | mTOR | G1871A | R624H | Guanine(G) at position 1871 → Adenine(A) |
| | | | | Arginine(R) at position 624 → Histidine (H) |
| 3 | mTOR | T4348G | Y1450D | Thymine (T) at position 4348 → Guanine(G) |
| | | | | Tyrosine (Y) at position 1450 → Aspartic acid (D) |
| 4 | mTOR | T4447C | C1483R | Thymine(T) at position 4447 → Cytosine(C) |
| | | | | Cysteine(C) at position 1483 → Arginine(R) |
| 5 | mTOR | G5126A | R1709H | Guanine(G) at position 5126 → Adenine(A) |
| | | | | Arginine(R) at position 1709→ Histidine(H) |
| 6 | mTOR | C5930A | T1977K | Cytosine(C) at position 5930→ Adenine(A) |
| | | | | Threonine (T) at position 1977 → Lysine (K) |
| 7 | mTOR | C6577T | R2193C | Cytosine(C) at position6577 → Thymine(T) |
| | | | | Arginine(R) at position 2193→ cysteine(C) |
| 8 | mTOR | C6644T | S2215F | Cytosine(C) at position 6644→ Thymine(T) |
| | | | | Serine (S) at position2215 → Phenylalanine (F) |
| 9 | mTOR | T7280C | L2427P | Thymine(T) at position 7280→Cytosine(C) |
| | | | | Leucine (L) at position 2427 → Proline (P) |
| 10 | mTOR | T7280A | L2427Q | Thymine(T) at position 7280→ Adenine(A) |
| | | | | Leucine (L) at position 2427→ Glutamine (Q) |
| 11 | TSC1 | C64T | R22W | Cytosine(C) at position 64→ Thymine(T) |
| | | | | Arginine(R) at position 22→ Tryptophan (W) |
| 12 | TSC1 | C610T | R204C | Cytosine(C) at position 610→ Thymine(T) |
| | | | | Arginine(R) at position 204→ cysteine(C) |
| 13 | TSC1 | G2432T | R811L | Guanine(G) at position 2432→ Thymine(T) |
| | | | | Arginine(R) at position 811→ Leucine (L) |
| 14 | TSC2 | G4639A | V1547I | Guanine(G) at position 4639 → Adenine(A) |
| | | | | Valine (V) at position 1547→ Isoleucine (I) |
| 15 | AKT3 | G740A | R247H | Guanine(G) at position 740 → Adenine(A) |
| | | | | Arginine(R) at position 247 → Histidine(H) |
| 16 | PIK3CA | G3052A | D1018N | Guanine(G) at position 3052→Adenine(A) |
| | | | | Aspartic acid (D) at position 1018→ Asparagine (N) |

The identified mutations were specifically detected in the brain tissue samples, but were negative for all available saliva and blood samples from mutation-positive patients. In addition, at least one of the nine different mTOR mutations was found in all FCD patients, with an allelic frequency of 1.26 to 12.6%.

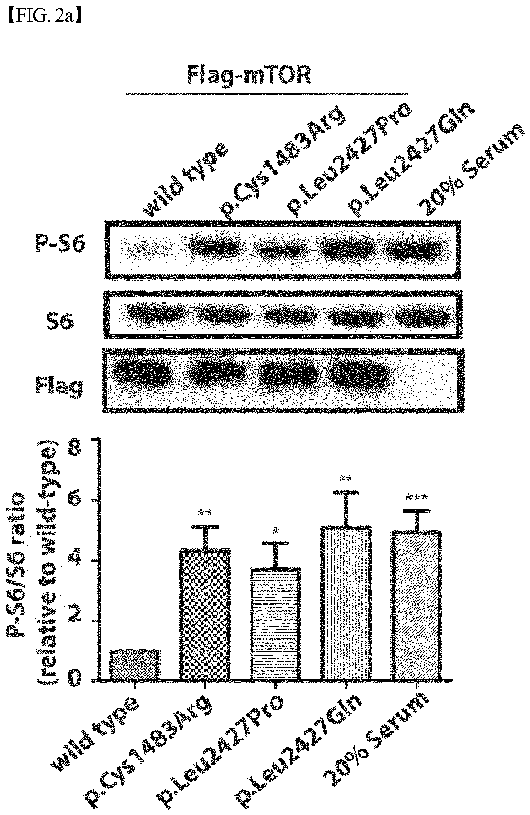

As described herein, mTOR mutant constructs expressing the mutations were obtained, and cells, after transfection of the mTOR mutant constructs thereinto, were analyzed for mTOR protein activation in terms of the phosphorylation of an S6 protein and the activity of mTOR kinase. As a result, an increase in the phosphorylation of S6 phosphorylation (FIG. 2a) and the activation of mTOR kinase (FIG. 2n) was identified, indicating that the hyperactivation of the mTOR pathway induces the phosphorylation of S6.

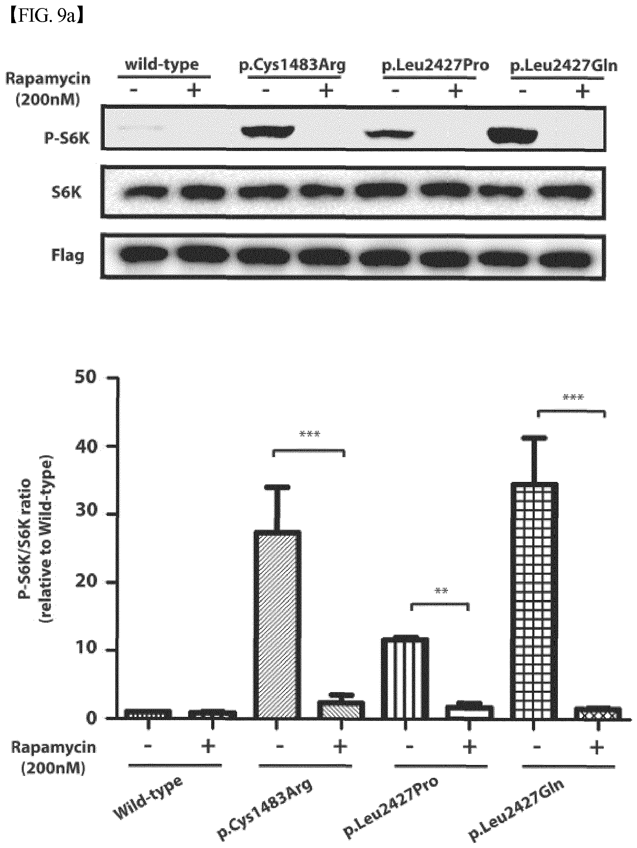

In addition, treatment with rapamycin, everolimus, or compounds of Chemical Formulas 1 to 4 was discovered to reduce the phosphorylation of S6 in the cells with the hyperactivated mTOR pathway due to the transfection of the mTOR mutant constructs thereinto (FIGS. 9a to 9c).

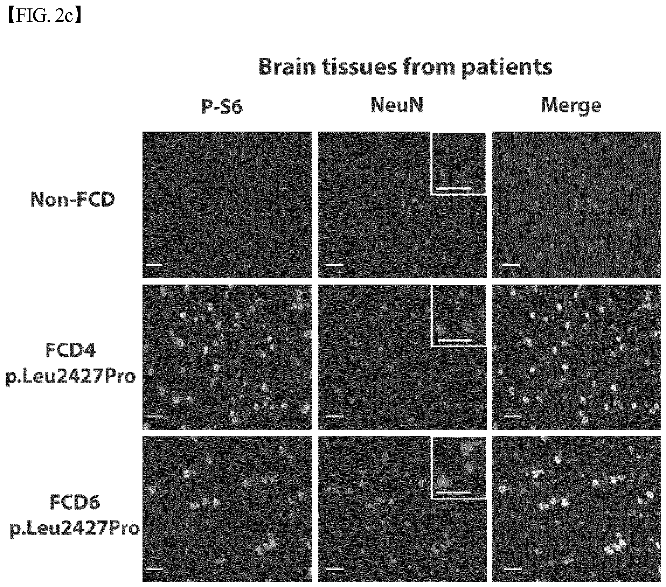

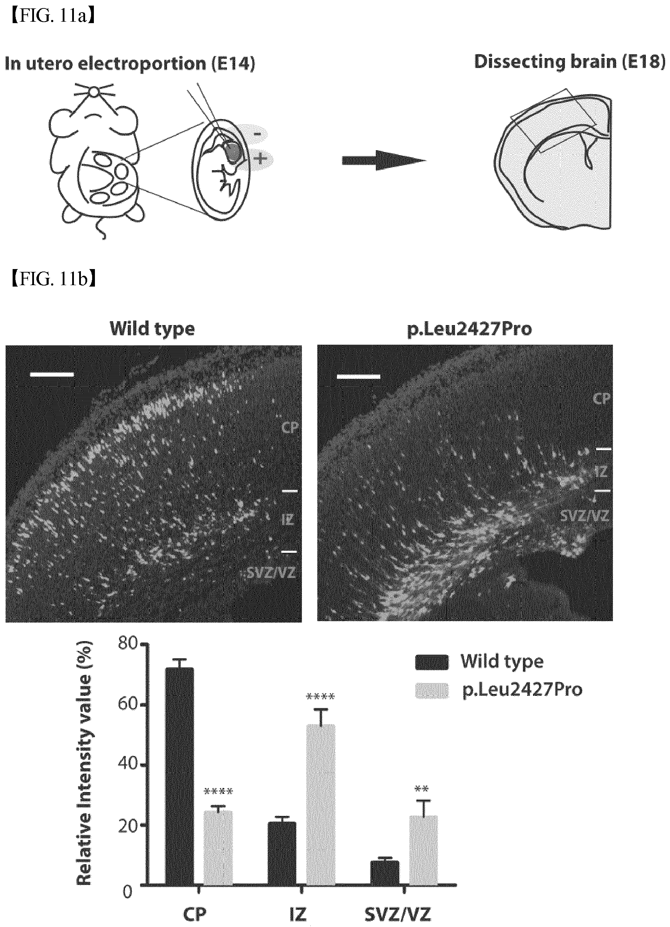

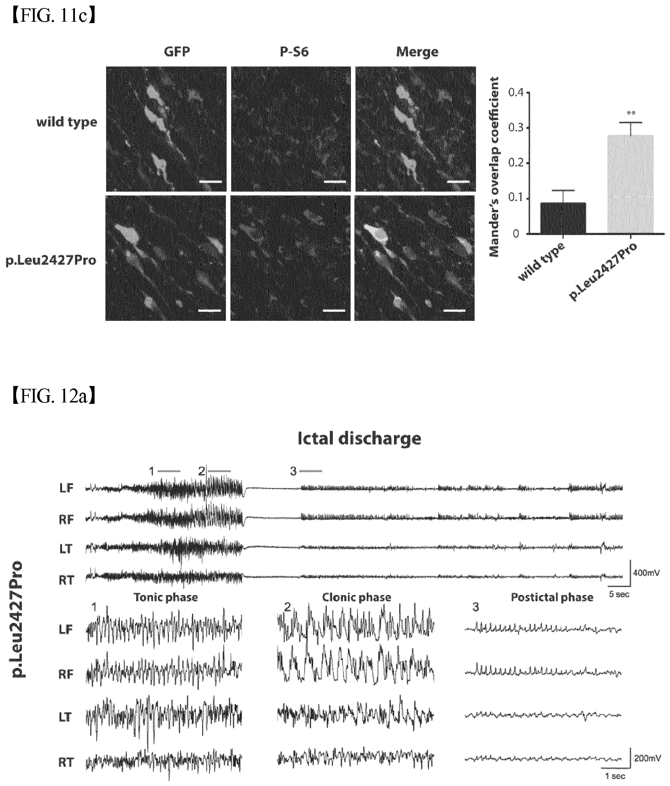

Meanwhile, the induction of FCD by the mTOR mutations, as descirbed herein, was confirmed by observing a significantly elevated level of phosphorylated S6 protein and an increased soma size of neurons in pathological brain samples of patients with FCD-caused intractable epilepsy (mTOR gene mutation) (FIGS. 2c to 2e), and by significant disruption of neuronal migration and a significantly increased level of phosphorylated S6 in the cortex of mTOR mutant construct-injected mice at embryonic day 14 (FIGS. 11b and 11c).

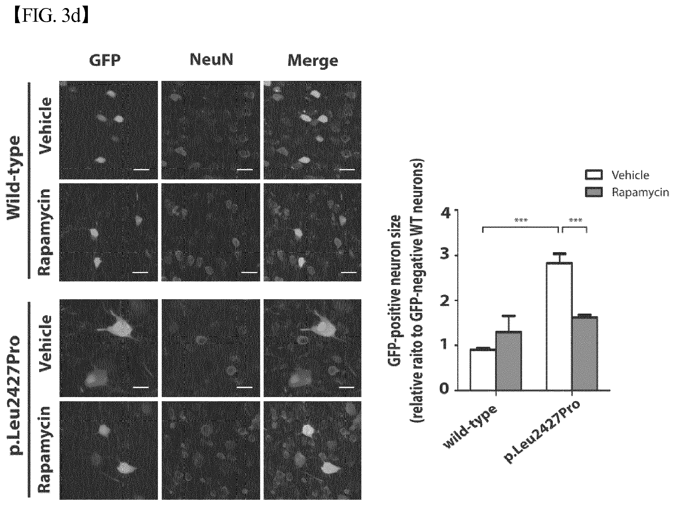

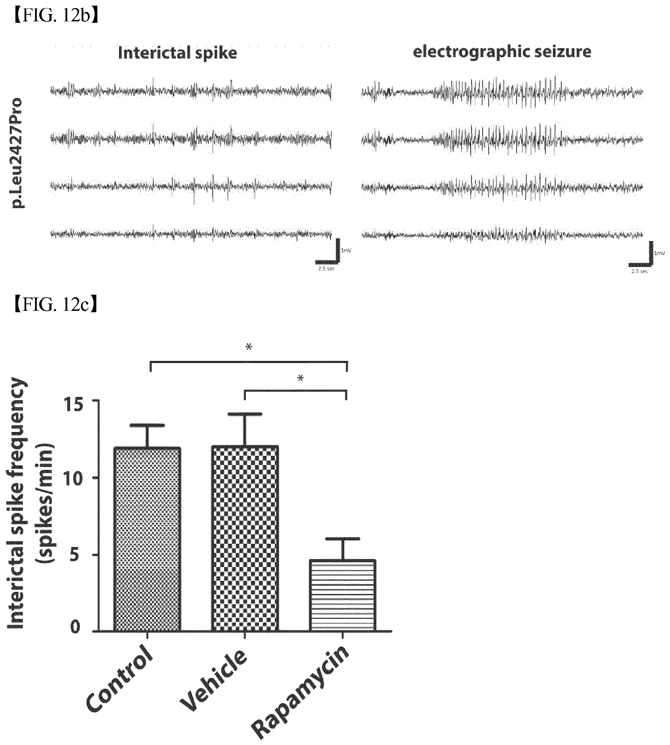

As described in the following, the lateral ventricles of embryonic mice were electroporated with mTOR mutant constructs carrying the genetic mutations at embryonic day 14 (E14), and video-electroencephalographic (video-EEG) monitoring of the mice was performed starting 3 weeks after birth. As a result, spontaneous seizure with epileptic discharge was detected in mice transfected with plasmids carrying the mTOR mutant genes of the present disclosure (FIGS. 12a and 12b). Further, it was observed that the soma sizes of GFP-positive neurons were greatly increased in affected cortical regions of electroporated mice carrying mTOR mutant constructs, showing an abnormal neuronal morphology similar to cytomegalic neurons. (FIG. 3d).

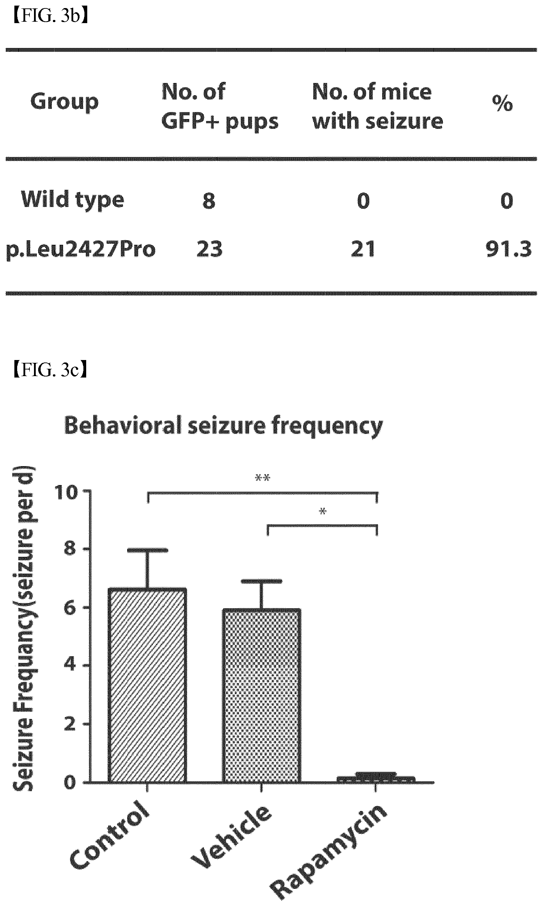

In addition, the animal models with spontaneous seizures or abnormal neurons, when administered with rapamycin, were observed to significantly decrease in the onset frequency of behavioral and electrographic seizures (FIG. 3c) and in the soma size of abnormal neurons (FIG. 3d).

As described herein, it is revealed not only that the genetic or protein mutations are specifically detected in FCD patient samples, but also that the mutations can induce FCD. Further, an mTOR inhibitor, for example, rapamycin, everolimus, and compounds of Chemical Formulas 1 to 4, is found to suppress intractable epilepsy associated with the mTOR mutations, such as mTOR hyperactivation, spontaneous seizures, behavioral seizures, electrographic seizures and generation of abnormal neurons in FCD.

In the following Example section, mTOR mutant constructs carrying the somatic mutations were obtained and transfected into cells, with the consequence of an increased level of S6K phosphorylation, which explains the activation of mTOR. Rapamycin decreased the phosphorylation. The data suggest that given the mutations, mTOR, TSC1, TSC2, AKT3 and PIK3CA genes or proteins activate the mTOR pathway, thus inducing epilepsy.

Further disclosed is a biomarker panel for the diagnosis of intractable epilepsy, comprising mTOR, TSC1, TSC2, AKT3 and PIK3CA genes or proteins carrying the mutations. Further disclosed is a diagnostic kit for detecting the biomarker panel genes or proteins in a sample from a subject, and a diagnostic method using the same. Further disclosed is a technique for constructing an epilepsy model, comprising inducing intractable epilepsy with the genetic or protein mutations.

The invention and disclosure addresses the prophylaxis, amelioration or therapy of intractable epilepsy, and a composition, a kit or a method for preventing, ameliorating or treating malformations of cortical developments, Hippocampal Sclerosis, or Sturge-Weber Syndrome, such as FCD, Hemimegalencephaly, and Tuberous Sclerosis Complex, which are common causes of intractable epilepsy. A particular embodiment of the invention relates to the prophylaxis, therapy and/or amelioration of brain somatic mutation-associated intractable epilepsy, as defined in the appended claims.

In relation to the invention, the intractable epilepsy includes epilepsy caused by the brain somatic mutation of genes involved in the PI3K-AKT-mTOR pathway, and epilepsy caused by malformations of cortical developments, Hippocampal Sclerosis, or Sturge-Weber Syndrome, such as FCD, Hemimegalencephaly and Tuberous Sclerosis Complex.

As used herein, the term "epilepsy" refers to a group of chronic neurological diseases characterized by repetitive seizures induced by excessive electric discharge in some neurons within a short time. The term "intractable epilepsy" means epilepsy that is insensitive to available antiepileptic drugs. Forms of intractable epilepsy may be those that are caused by Malformations of Cortical Developments (MCD), Hippocampal Sclerosis (HS), or Sturge-Weber Syndrome (SWS), such as Focal Cortical Dysplasia (FCD), Hemimegalencephaly (HME), and Tuberous Sclerosis Complex (TSC).

In the normal development of the cerebral cortex, neurons migrate from one region of the brain to another to form a laminar structure. The term "Focal Cortical Dysplasia" or "FCD", as used herein, is a congenital abnormality of brain development where the neurons in one area of the brain fail to migrate in the proper formation *in utero* and thus fail to form a normal laminar structure. Etiologically, FCD is accounted for by the failure of normal development in some region of the cerebrum or by the generation of some dysmorphic neurons even in a region that seems to develop normally, as observed in radiographic images. FCD occurs sporadically and is characterized by dysmorphic neurons and disrupted cortical lamination in affected cortical regions.

The brain somatic mutations associated with FCD may be mutations on mTOR genes or protein.

mTOR (mammalian target of rapamycin) protein, encoded by the FRAP1 gene in humans, is a serine/threonine protein kinase, which is functionally involved in cell growth, cell proliferation, cell death, cell survival, protein synthesis, and transcription, and belongs to the phosphatidylinositol 3-kinase-related kinase protein family. In the present disclosure, nucleotide and protein sequences of wild-type mTOR genes are represented by SEQ ID NOs. 1 and 2, respectively.

As used herein, the term "brain somatic mutation" means an alteration at one or more positions of the nucleotide sequence of a wild-type gene. In this context, it may be a nucleotide mutation of mTOR, TSC1, TSC2, AKT3 and PIK3CA genes or an amino acid mutation of proteins corresponding to the genes. By way of example, an alteration may occur on the nucleotide sequence of the wild-type mTOR gene represented by SEQ ID NO. 1. As shown in Table 1, the brain somatic mutation may be a substitution at one or more selected from the group consisting of positions 616, 1871, 4348, 4447, 5126, 5930, 6577, 6644, 7280 and 7280 of the nucleotide sequence of SEQ ID NO. 1.

Alternatively, the brain somatic mutation according to the invention may be an alteration at one or more positions of the amino acid sequence of the wild-type mTOR protein of SEQ ID NO. 2. On the amino acid sequence of SEQ ID NO. 2, for example, the mutation may include at least one selected from the group consisting of substitutions from arginine (R) at position 206 to cysteine(C), from R at position 624 to H, from Y at position 1450 to D, from C at position 1483 to R, from R at position 1709 to H, from T at position 1977 to K, from R at position 2193 to C, from S at position 2215 to F, from L at position 2427 to P, and from L at position 2427 to Q. The substituted amino acids may be encoded by the genetic codes resulting from mutations at corresponding positions on the nucleotide sequence of SEQ ID NO. 1. The base mutations and corresponding amino acid mutations are listed in Table 1.

As used herein, the term "TSC1 mutant gene" refers to a TSC1 gene in which a mutation occurs on the wild-type TSC1 nucleotide sequence of SEQ ID NO. 3. Particularly, it may be a gene containing at least one mutation selected from substitutions from cytosine (C) at position 64 to thymine (T), from cytosine (C) at position 610 to thymine (T), and from guanine (G) at position 2432 to thymine (T) on the nucleotide sequence of SEQ ID NO. 3.

As used herein, the term "TSC1 mutant protein" refers to a TSC1 protein in which a mutation occurs on the wild-type TSC1 amino acid sequence of SEQ ID NO. 4. Particularly, it may be a protein containing at least one mutation selected from substitutions from arginine (R) at position 22 to tryptophan (W), from arginine (R) at position 204 to cysteine (C), and from arginine (R) at position 811 to leucine (L) on the amino acid sequence of SEQ ID NO. 4.

The term "TSC2 mutant gene", as used herein, refers to a gene in which a mutation occurs on the wild-type TSC2 nucleotide sequence of SEQ ID NO. 5. Particularly, it may be a gene containing a substitution from guanine (G) at position 4639 to adenine (A) on the nucleotide sequence of SEQ ID NO. 5.

The term "TSC2 mutant protein", as used herein, refers to a TSC2 protein in which a mutation occurs on the wild-type TSC2 amino acid sequence of SEQ ID NO. 6. Particularly, it may be a protein containing a mutation from valine (V) at position 1547 to isoleucine (I) on the amino acid sequence of SEQ ID NO. 6.

The term "AKT3 mutant gene", as used herein, refers to a gene in which a mutation occurs on the wild-type AKT3 nucleotide sequence of SEQ ID NO. 7. Particularly, it may be a gene containing a substitution from guanine (G) at position 740 to adenine (A) on the nucleotide sequence of SEQ ID NO. 7.

The term "AKT3 mutant protein", as used herein, refers to a TSC2 protein in which a mutation occurs on the wild-type AKT3 amino acid sequence of SEQ ID NO. 8. Particularly, it may be a protein containing a mutation from arginine (R) at position 247 to histidine (H) on the amino acid sequence of SEQ ID NO. 8.

The term "PIK3CA mutant gene", as used herein, refers to a gene in which a mutation occurs on the wild-type TSC2 nucleotide sequence of SEQ ID NO. 9. Particularly, it may be a gene containing a substitution from guanine (G) at position 3052 to adenine (A) on the nucleotide sequence of SEQ ID NO. 9.

The term "PIK3CA mutant protein", as used herein, refers to a TSC2 protein in which a mutation occurs on the wild-type TSC2 amino acid sequence of SEQ ID NO. 10. Particularly, it may be a protein containing a mutation from aspartic acid (D) at position 1018 to asparagine (N) on the amino acid sequence of SEQ ID NO. 10.

In addition, the mutated proteins may contain additional mutations, so long as they do not entirely alter the activity of the molecules. Amino acid substitutions of proteins or peptides that preserve all of the activity of the molecules are known in the art (H. Neurath, R. L. Hill, The Proteins, Academic Press, New York, 1979). If necessary, the mTOR mutant proteins may be modified by, for example, phosphorylation, sulfation, acrylation, glycosylation, methylation, and/or farnesylation.

Examples of the mTor inhibitor available in the invention include those listed in the disclosures of the following Patent Publication Nos. WO 2010/044885 A2, US 2015/0132397 A1, WO 2010/118208, US 2014/0024637 A1, US 2010/0298290 A1, WO 2010/138487, US 2014/0073628 A1, US 2014/0018347 A1, US 2014/0107100 A, US 2014/0005172 A1, WO 2012/174327, WO 2010/135567, WO 2010/135568, WO 2011/001112, US 2018/0214452 A1, US 2008/0293754 A1, KR 20090119755 A, US 2010/0190749 A1, WO 2010/051042, WO 2010/051043, WO 2010/006086, WO 2010/006072, US 2015/0065524 A1, US 2011/0077268 A1, US 2011/0124641 A1, US 2015/0225407 A1, WO 2010/090716, US 2011/0021515 A1, US 2011/0003806 A1, US 2011/0003807, US 2011/0105500 A1, WO 2010/118207, EP 2551270 A2, EP 2896622 A1, KR 20140134746 A, EP 2231152 A2, US 2008/0188423 A1, US 2005/0203173 A1, US 2004/0242631 A1, US 2011/0124638 A1, US 2012/0142673 A1, US 2008/0194546 A1, US 2006/0199804 A1, US 2010/0130473 A1, WO 2006/090169, US 2008/0132502 A1, US 2008/0081809 A1, US 2008/0171743 A1, US 2011/0034454 A1, US 2009/0018134 A1, US 2010/0249131 A1, US 2010/0227858 A1, US 2009/0099174 A1, US 2009/0306126 A1, US 2009/0118336 A1, US 2009/0076009 A1, US 2009/0325954 A1, US 2010/0022534 A1, US 2009/0233926 A1, US 2009/0270390 A1, US 2010/0261723 A1, WO 2008/032033, WO 2008/032036, WO 2008/032060, WO 2008/032089, WO 2008/032091, US 2005/0032825 A1, US 2012/0289481 A1, US 2014/0266291 A1, US 2012/0114739 A1, US 2012/0178744 A1, US 2012/0332791 A1, US 2013/0150353 A1, US 2013/0150362 A1, US 2013/0281474 A1, US 2014/0234837 A1, WO 2013/016160, US 2009/0098086 A1, US 2009/0181963 A1, US 2009/0291079 A1, US 2013/0315865 A1, US 2013/0109670 A1, US 2015/0011752 A1, US 2009/0304692 A1, US 2008/0234262 A1, US 2008/0233127 A1, US 2009/0311217 A1, US2009-0149458 A, US 2009/0192147 A1, US 2009/0192176 A1, US 2009/0227575 A1, US 2009/0298820 A1, US 2010/0015141 A1, US 2010/0061982 A1, US 2010/0068204 A1, US 2010/0305113 A1, US 2010/0317657 A1, US 2010/0311714 A1, US 2013/0073652 A1, EP 2518074 A1, US 2013/0129820 A1, US 2011/0076291 A1, US 2013/0012488 A1, EP 2480549 A1, WO 2011/058027, US 2008/0242665 A1, US 2010/0331305 A1, US 2011/0086841 A1.

In detail, mTOR inhibitors useful for the invention may be as follows: AMG954, AZD8055, AZD2014, BEZ235, BGT226, rapamycin, everolimus, sirolimus, CC-115, CC-223, LY3023414, P7170, DS-7423, OSI-027, GSK2126458, PF-04691502, PF-05212384, temsirolimus, INK128, MLN0128, MLN1117, ridaforolimus, Metformin, XL765, SAR245409, SF1126, VS5584, GDC0980, and GSK2126458. Further examples ofthe mTOR inhibitors includes those listed in WO2012/104776, KR 10-1472607B, WO2010/039740, US8846670, US8263633, and WO2010/002954.

According to some embodiments of the invention, the mTOR inhibitor may be at least one selected from the group consisting of rapamycin or a salt thereof, everolimus or a salt thereof, a compound of Chemical Formula 1 or a salt thereof a compound of Chemical Formula 2 or a salt thereof, a compound of Chemical Formula 3 or a salt thereof, and a compound of Chemical Formula 4 or a salt thereof

As used herein, the term "rapamycin" refers to a macrolide lactone compound, known as sirolimus, which has immunosuppressant functions. Rapamycin is commercialized as a drug for preventing rejection of transplanted organs. Also, it is used as a therapeutic agent for pneumonia, immunoinflammatory skin disorders such as systemic lupus erythematosus and psoriasis, immunoinflammatory bowel disorders, orbital inflammation, restenosis, and rheumatoid arthritis, and as an anti-cancer agent. However, nowhere has the application of rapamycin to the prevention or treatment of brain somatic mutation-associated FCD been reported in previous documents.

As used herein, the term "everolimus" refers to a drug for treating kidney cancer. It is used when the antiangiogenic drug SUNItinib or sorafenib is no longer effective. Also, everolimus is approved for the treatment of subependymal giant cell astrocytoma associated with Tuberous Sclerosis (TS) in patients who are not suitable candidates for surgical intervention. However, to date there have been no reports on the use of everolimus in the prevention or treatment of brain somatic mutation-associated FCD.

The compounds of Chemical Formulas 1 to 4 are known as inhibitors active against mTOR; however, applicability to the prevention or treatment of brain somatic mutation-associated FCD has not been known at all.

Rapamycin, everolimus, and the compounds of Chemical Formulas 1 to 4 are useful in the invention, and their derivatives or mimics, pharmaceutically acceptable salts thereof, and hydrates are also fall within the scope of drugs available for the invention.

The pharmaceutically acceptable salts or hydrates may be salts or hydrates derived from inorganic acids or organic acids. Examples of pharmaceutically acceptable salts include salts of hydrochloric acid, hydrobromic acid, sulfuric acid, phosphoric acid, nitric acid, acetic acid, glycolic acid, lactic acid, pyruvic acid, malonic acid, succinic acid, glutaric acid, fumaric acid, malic acid, mandelic acid, tartaric acid, citric acid, ascorbic acid, palmitic acid, maleic acid, hydroxymaleic acid, benzoic acid, hydroxybenzoic acid, phenylacetic acid, cinnamic acid, salicylic acid, methane sulfonic acid, benzene sulfonic acid, and toluene sulfonic acid, but are not limited thereto. The hydrates may refer to those formed by binding rapamycin, everolimus or the compounds of Chemical Formulas 1 to 4 with water molecules.

As used herein, the term "treatment" or "therapy" refers to any action resulting in improvement or mitigation in the symptoms of a disease of interest, a reduction in affected area, the suppression or delay of the onset or progression of disease, the amelioration, mitigation, or stabilization of a disease state, partial or complete recovery, prolonged survival, and other beneficial alterations. In this regard, the term is intended to include the mitigation, amelioration, reduction or cure of symptoms of brain somatic mutation-associated FCD thanks to the mTOR inhibitor for use as defined in the appended claims.

The symptoms of brain somatic mutation-associated FCD occur as neurons in an area of the brain fail to migrate in the proper formation during brain development, and are exemplified by spontaneous seizures, behavioral seizures, electrographic seizures, and the generation of abnormal neurons in the cerebral cortex.

Accordingly, the medical use according to the invention may mean a significant decrease in the onset frequency of spontaneous seizures, behavioral seizures, and electrographic seizures and in the number or soma size of abnormal neurons in the cerebral cortex thanks to the administration of an mTOR inhibitor, for example, rapamycin, everolimus, and/or the compounds of Chemical Formulas 1 to 4, into patients with brain somatic mutation-associated FCD, as defined in the appended claims.

Depending on modalities and regimens of the pharmaceutical composition of the present disclosure, an effective amount of the mTOR inhibitor may be suitably determined by those skilled in the art.

For example, the pharmaceutical composition may comprise the mTOR inhibitor in an amount of 0.1 to 10% by weight based on the total weight of the composition, and particularly in an amount of 0.5 to 5% by weight.

The pharmaceutical composition of the present disclosure may comprise the mTOR inhibitor alone or in combination with a pharmaceutically acceptable additive. The pharmaceutically acceptable additive is an additive that is typically useful for formulations, examples of which include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil. Examples of pharmaceutically acceptable excipients available in the present disclosure include a lubricant, a humectant, a sweetener, an aromatic, an emulsifier, a flavoring agent, a suspending agent, and a preservative, but are not limited thereto. That is, pharmaceutically acceptable additives available in the pharmaceutical composition of the present disclosure may be selected according to the purpose of use by those skilled in the art without difficulty, and their amounts may be determined within the range that does not hinder the purpose and effect of the present disclosure.

The therapeutically effective amount of the pharmaceutical composition of the present disclosure may vary depending on various factors including a patient's state, and weight, the severity of disease, the dosage form of drug, the route of administration, the time of administration, etc. For a preferable effect, the active ingredient of the present disclosure may be administered in an amount of from 1 to 1000 mg/kg a day, preferably in an amount of from 50 to 500 mg/kg, and more preferably in an amount of 150 to 300 mg/kg. The composition of the present disclosure may be administered in a single dose or may be divided into multiple doses. Accordingly, the dosage does not limit the present discbsure in any aspect.

The pharmaceutical composition of the present disclosure may be administered to mammals such as rats, mice, livestock and humans via various routes. All of the administration methods are known, and, for example, the dosage may be administered orally, rectally, intravenously, intramuscularly, subcutaneously, or intracerebroventricularly.

Further disclosed is a food composition for preventing or ameliorating brain somatic mutation-associated FCD, comprising at least one selected from the group consisting of the mTOR inhibitors, for example, rapamycin or a salt thereof, everolimus or a salt thereof the compound of Chemical Formula 1 or a salt thereof, the compound of Chemical Formula 2 or a salt thereof, the compound of Chemical Formula 3 or a salt thereof, and the compound of Chemical Formula 4 or a salt thereof. The compounds of Chemical Formulas 1 to 4 are as defined above.

The food composition may be added to foods, without change or together with other food or food ingredients, and used properly according to typical modalities. The mTOR inhibitor useful in the present disclosure may be determined suitably according to the purpose of use (prevention, health or therapeutic treatment). For a general food composition, the active ingredient may be contained in an amount of 0.01 to 10 parts by weight, based on 100 parts by weight of the composition, and particularly in an amount of 0.05 to 1 parts by weight. However, the content of the mTOR inhibitor may be below the range in a food composition that is intended for long-term ingestion with the aim of improving or controlling health.

The food composition may be applied to a health food for the prevention or amelioration of brain somatic mutation-associated FCD. There are no particular limitations on the kind of health food to which the composition of the present invention can be added. Examples of such a health food include meats, sausages, breads, chocolates, candies, snacks, confectionery, pizzas, ramen noodles, other noodles, gums, dairy products such as ice cream, various soups, beverages, teas, drinks, alcoholic beverages, vitamin complexes, etc., and all kinds of commonly accepted health foods. In addition, the food composition of the present disclosure may further comprise a cytologically acceptable additive. Although not significantly important, the content of the sitologically acceptable additive may generally be determined within a range of 0.01 to 0.1 parts by weight, based on 100 parts by weight of the composition of the present discbsure.

Further disclosed is a kit or composition for the diagnosis of intractable epilepsy or a causative disease thereof, comprising an agent capable of detecting a mutation in a gene or protein involved in the PI3K-AKT-mTOR pathway, or a method for the diagnosis of intractable epilepsy or a causative disease thereof, using the same.

Further disclosed is a biomarker panel for the diagnosis of intractable epilepsy, comprising a mutant of a gene or protein involved in the PI3K-AKT-mTOR pathway. Further disclosed is a composition for inducing intractable epilepsy, comprising a mutant of a gene or protein involved in the PI3K-AKT-mTOR pathway.

The term "diagnosis", as used herein, refers to identifying the presence or characteristics of a pathological condition. For the purpose of the present disclosure, "diagnosis" may mean identifying the onset of intractable epilepsy, and furthermore, the progression or aggravation of the disease.

As used herein, the terms "diagnostic marker" "marker for diagnosis" and "diagnosis marker" are intended to indicate a substance that is found specifically in a sample of a patient with intractable epilepsy and the detection of which accounts for the onset of intractable epilepsy. For the purpose of the present disclosure, the diagnostic marker of the present disclosure may mean mutant genes or proteins of mTOR, TSC1, TSC2, AKT3 and PIK3CA, which are present specifically in the affected brain regions of intractable epilepsy patients.

As used herein, the term "biomarker panel" is intended to include one or more of the biomatkers disclosed in the present disclosure. The biomarker panel can be detected using a detection agent (or reagent) that can directly or indirectly associate with or bind to a biomarker protein or gene present in a sample.

The brain somatic mutant associated with intractable epilepsy in accordance with the invention is a mutant of a gene or protein involved in the PI3K-AKT-mTOR pathway, that is, mutants of mTOR, TSC1, TSC2, AKT3 and PIK3CA genes or proteins as defined in the appended claims. These mutant genes or proteins are as described above.

In one aspect of the disclosure, the agent capable of detecting a substitution may be a primer, a probe or an antisense nucleic acid that is specific for a substitution region.

Further disclosed is a method for providing information on the diagnosis of intractable epilepsy, comprising:
(a) treating a sample of a subject with the diagnostic kit;
(b) detecting in the sample a biomarker panel containing at least one selected from the group consisting of the substitutions listed in Table 1; and
(c) determining the onset of intractable epilepsy if the biomarker panel containing one or more of the substitutions is detected.

Further disclosed is a diagnostic kit for intractable epilepsy, comprising an agent capable of detecting the amino acid substitutions listed in Table 1.

In one aspect of the disclosure, the agent capable of detecting the substitutions may be an antibody or aptamer specific for a substitution region.

Further disclosed is a method for providing information on the diagnosis of intractable epilepsy, comprising:
(a) treating a sample of a subject with the diagnostic kit;
(b) detecting in the sample a biomarker panel containing at least one selected from the group consisting of the mutations listed in Table 1; and
(c) determining the onset of intractable epilepsy if the biomarker panel containing one or more of the substitutions is detected.

In one aspect of the disclosure, the sample may be a brain tissue sample from the subject.

Further disclosed is a biomarker panel for the diagnosis of intractable epilepsy, comprising the mutant protein or the mutant gene.

The term "agent capable of detecting a substitution", as used in the context of the detection of substitutions on nucleotide sequences, means a substance useful for detecting a substitution (mutation) on the nucleotide sequences of mTOR, TSC1, TSC2, AKT3 or PIK3CA in the sample. For example, the agent may be a primer, probe, or an antisense oligonucleotide capable of binding specifically or complementarily to a nucleotide sequence carrying a substitution. Particularly, the primer, the probe or the antisense oligonucleotide may specifically bind to the stretch carrying a substitution, but not to a wild-type sequence.

The term "complementary" as used herein means a level of complementarity sufficient to selectively hybridize with the nucleotide sequence under certain particular hybridization or annealing conditions, and particularly under physiological conditions, and is intended to include both substantial complementarity and perfect complementarity, particularly perfect complementarity.

In some aspects of the disclosure, the agent for detecting a substitution region of a gene may be an antisense oligonucleotide. The term "antisense oligonucleotide", as used herein, encompasses a nucleic acid-based molecule complementary to a target mutation region to form a duplex with the mutation region. It is applicable to the detection of the gene biomarker panel of the present disclosure.

In other aspects of the disclosure, the agent for detecting a substitution region of a gene may be a primer or a probe. Because nucleotide sequences of mTOR, TSC1, TSC2, AKT3 and PIK3CA mutant genes useful in the present disclosure are revealed, a primer or a probe for specifically amplifying a predetermined region of the gene can be designed on the basis of the nucleotide sequences.

As used herein, the term "primer" refers to a short nucleic acid strand, typically 7 to 50 bases long, having a free 3' hydroxyl group, which forms a base pair with a complementary template so as to serve as a starting point for the production of a new template strand. Primers are typically synthesized, but may be naturally occurring nucleic acids. The sequence of a primer need not be completely consistent with that of a target template, and may be accepted if it is sufficiently complementary to hybridize with the template. DNA synthesis or replication requires a suitable buffer, proper temperatures, polymerizing enzymes (DNA polymerase or reverse transcriptase), and four kinds of nucleotide triphosphates, in addition to primers. In the present disclosure, sense and antisense primers specific for mTOR polynucleotide can be used for PCR amplification so that the PCR products can be used to diagnose epilepsy. The length of the sense and antisense primers may be suitably altered depending on the information known in the art. Particularly, the primers useful in the present disclosure may be those applicable to the amplification of a mutant region of the gene provided by the present disclosure.

In other aspects of the disclosure, the agent for detecting a substitution region of a gene may be a probe. The term "probe", as used herein, is intended to refer to a fragment of a nucleotide sequence, such as RNA or DNA, ranging in length from ones to hundreds of bases, which can bind specifically to an mRNA of interest and which is tagged with a label for detecting the mRNA of interest. The probe useful in the present disclosure may be constructed in the form of oligonucleotide probes, single-stranded DNA probes, double-stranded DNA probes, or RNA probes. In an embodiment of the present disclosure, the diagnosis of epilepsy may be achieved by determining whether a probe complementary to the mTOR mutant polynucleotide of the present disclosure hybridizes with the nucleotide sequence of interest. The selection of suitable probes and hybridization conditions may be modified according to information known in the art.

The primers or probes useful in the present disclosure may be chemically synthesized using a phosphoramidite solid support method or other well-known techniques. Their nucleotide sequences may be modified using various means known in the art, so long as their fundamental properties remain unchanged. Illustrative, non-limiting examples of the modification include methylation, capping, substitution of natural nucleotides with one or more homologues, and alternation between nucleotides.

The term "agent for detecting a substitution", as used in the context of the detection of a substitution on an amino acid sequence, refers to a substance useful for detecting a mutant region of a biomarker panel protein in a sample of a patient. Particularly, the agent may be an antibody or aptamer specific for a protein composed of an amino acid sequence carrying a mutation provided by the present disclosure. In some disclosures, the antibody may be monoclonal or polyclonal

The term "antibody", as used herein, refers to a specific protein molecule that indicates an antigenic region. With respect to the purposes of the present invention, the antibody binds specifically to a mutant region of the biomarker panel protein of the present disclosure. This antibody can be produced from a protein that is encoded by the mutant gene, typically cloned into an expression vector using a conventional method. Also, partial peptides being producible from the protein which is encoded by the mutant gene fall within the scope of the antibody. To function as an antibody, the partial peptide is required to contain at least 7 amino acid residues, preferably 9 or more amino acid residues, and more preferably 12 or more amino acid residues. No particular limitations are imposed on the form of the antibodies of the present disclosure. Among them are polyclonal antibodies, monoclonal antibodies and fragments thereof which contain a paratope, and all immunoglobulin antibodies. Further, special antibodies such as humanized antibodies are also part of the disclosure.

In addition, the antibodies of the present disclosure which are used to detect the marker diagnostic of intractable epilepsy include functional fragments of antibody molecules as well as complete forms having two full-length light chains and two full-length heavy chains. The functional fragments of antibody molecules refer to fragments retaining at least an antigen-binding function, and include F_{ab}, F_{(ab')}, F_{(ab')2}, Fᵥ and the like.

The agent for detecting a biomarker panel gene or protein in accordance with the present disclosure may be embodied into a kit. The kit according to the present disclosure is capable of detecting a biomarker panel gene or a protein. The kit of the present invention may comprise a primer, a probe or an antisense oligonucleotide for detecting a biomarker panel gene, or an antibody or an aptamer for detecting a biomarker panel protein, and one or more compositions, agents or devices suitable for analysis.

In one aspect of the disclosure, the kit for detecting a biomarker panel gene in accordance with the present disclosure may be a kit for diagnosing intractable epilepsy comprising elements necessary for DNA chip function. The DNA chip kit may comprise a substrate to which an agent for detecting a biomarker panel gene is immobilized, and a reagent, an agent and an enzyme for constructing a fluorescence-labeled probe. In addition, the substrate may contain an agent for quantitatively detecting a control gene or a fragment thereof In addition, the kit designed to detect a biomarker panel gene may be a kit comprising elements necessary for PCR. Such a PCR kit may comprise a pair of primers specific for each of the mTOR mutant genes, test tubes or other suitable containers, reaction buffers (various pH and magnesium concentrations), deoxynucleotides (dNTPs), enzymes such as Tag-polymerase, a DNase inhibitor, an RNase inhibitor, DEPC-water, sterile water, and so on. In addition, the kit may further comprise a pair of primers specific for a gene useful as a quantitative control. Particularly, the kit may be a multiple PCR kit for simultaneously amplifying and analyzing biomarker panel genes through multiple PCR.

In one aspect of the disclosure, the kit for detecting a biomarker panel protein may comprise antibodies and elements necessary for the immunological detection of the antibodies, including a support, a suitable buffer, a coloring enzyme- or fluorescent-labeled secondary antibody, and a coloring substrate. Examples of the support include a nitrocellulose membrane, a 96-well plate made of polyvinyl resin or polystyrene resin, and slide glass. Among the coloring enzymes are peroxidase and alkaline phosphatase. FITC or RITC may be used as a fluorescent. ABTS (2,2'-azino-bis-(3-ethylbenzothiazoline-6-sulfonic acid)) or OPD (o-phenylenediamine), or TMB (tetramethyl benzidine) is suitable as the coloring substrate.

Genomic DNA or total protein available in the method for detecting a biomarker panel in a sample of a subject according to the present disclosure may be separated using a process known in the art.

The term "a sample of a subject", as used herein, is intended to encompass specimens including tissues and cells in which the biomarker panel gene or protein can be detected. Particularly, the sample may be a brain tissue sample, but is not limited thereto.

According to some aspects of the present disclosure, the method for detecting a biomarker panel gene in a sample of a subject comprises amplifying a nucleic acid from the sample and determining the base sequence of the amplified nucleic acid.

The amplification of the nucleic acid may be achieved by polymerase chain reaction (PCR), multiplex PCR, touchdown PCR, hot start PCR, nested PCR, booster PCR, real-time PCR, differential display PCR (DD-PCR), rapid amplification of cDNA ends (RACE) PCR, inverse PCR, vectorette PCR, thermal asymmetric interlaced PCR (TAIL-PCR), ligase chain reaction, repair chain reaction, transcription-mediated amplification, self-sustained sequence replication, or selective amplification of target polynucleotide sequences.

To determine the base sequence of the amplified nucleic acid, Sanger sequencing, Maxam-Gilbert sequencing, Shotgun sequencing, pyrosequencing, hybridization by microarray, allele-specific PCR, dynamic allele-specific hybridization (DASH), PCR extension assay, TaqMan technique, automated DNA sequencing, or next-generation DNA sequencing may be used. The next-generation DNA sequencing may be performed using a DNA analyzing system widely known in the art, for example, 454 GS FLX manufactured by Roche, Genome Analyzer manufactured by Illumina, SOLid Platform manufactured by Applied Biosystems, etc.

The detection of a biomarker panel protein in a sample of a patient may be performed by Western blotting, ELISA, radioimmunoassay, radioimmunodiffusion, ouchterlony immunodiffusion, rocket immunoelectrophoresis, immunohistostaining, immunoprecipitation assay, complement fixation assay, FACS, or protein chip assay using an antibody or aptamer specifically detecting the corresponding amino acid mutation. With the analysis methods, an antigen-antibody complex between the mTOR mutant protein and the antibody thereof can be identified, and intractable epilepsy can be diagnosed by examining the antigen-antibody complex between the mutant protein and the antibody thereof

As used herein, the term "antigen-antibody complex" is intended to refer to a product formed by the binding of a mutant protein to an antibody specific thereto. The antigen-antibody complex thus formed may be quantitatively determined by measuring the signal intensity of a detection label.

The detection label may be selected from a group consisting of enzymes, fluorescents, ligands, luminescents, microparticles, redox molecules, and radioactive isotopes, but not strictly limited thereto. If an enzyme is used as the detection label, available enzymes may include β-glucuronidase, β-D-glycosidase, β-D-galactosidase, urease, peroxidase or alkaline phosphatase, acetylcholinesterase, glucose oxidase, hexokinase and GDPase, RNase, glucose oxidase and luciferase, phosphofructokinase, phosphoenolpyruvate carboxylase, aspartate aminotransferase, phosphoenolpyruvate decarboxylase, β-lactamase or the like, but are not limited thereto. Examples of the fluorescents may include fluorescein, isothiocyanate, rhodamine, phycoerythrin, phycocyanin, allophycocyanin, o-phthalaldehyde, fluorescamine or the like, but are not limited thereto. The ligand may be exemplified by biotin derivatives, but is not limited thereto. Among the luminescents may be acridinium ester, luciferin, and luciferase. Representative of the microparticles are colloidal gold and colored latex, without limitation thereto. The redox molecules may include ferrocene, ruthenium complex, biologen, quinone, Ti ion, Cs ion, diimide, 1,4-benzoquinone, hydroquinone, K₄W(CN)₈, [Os(bpy)₃]²⁺, [RU(bpy)-₃]²⁺, [MO(CN)₈]⁴⁻ or the like, but are not limited thereto. The radioactive isotope may be exemplified by, but are not limited to, ³H, ¹⁴C, ³²P, ³⁵S, ³⁶Cl, ⁵¹Cr, ⁵⁷Co, ⁵⁸Co, ⁵⁹Fe, ⁹⁰Y, ¹²⁵I, ¹³¹I, ¹⁸⁶Re or the like.

In one aspect of the disclosure, the measurement of an antigen-antibody complex between a biomarker panel protein and an antibody thereof may be achieved through an ELISA assay. In this regard, various kinds of ELISA assay may be employed, including direct ELISA using a labeled antibody that recognizes antigen attached to a solid support, indirect ELISA using a labeled antibody that recognizes a capture antibody from the complex of the antibody that recognizes an antigen attached to the solid support, direct sandwich ELISA using a labeled antibody that recognizes the antigen of an antigen-antibody complex attached to the solid support, and indirect sandwich ELISA, in which an antibody recognizing the antigen of an antigen-antibody complex attached to a solid support is detected by a labeled secondary antibody.

Most useful is a sandwich ELISA assay in which an antibody attached to a solid support is reacted with a sample and then captured by a labeled antibody recognizing the antigen of an antigen-antibody complex to perform enzymatic staining or by a labeled secondary antibody recognizing the antigen of an antigen-antibody complex to perform enzymatic staining. The onset of intractable epilepsy can be determined by identifying the formation of a complex between the biomarker panel and the antibody.

In another aspect of the disclosure, Western blotting may be carried out using one or more antibodies active against the biomarker panel. For example, total proteins are isolated from a sample and separated according to size via electrophoresis. The electrophoresed proteins are then transferred onto a nitrocellulose membrane and reacted with the antibody. Quantitative analysis of the formed antigen-antibody complex with a labeled antibody makes it possible to determine the onset of intractable epilepsy, based on the expression level of the mutant protein encoded by the mutant gene. Such detection may be carried out by investigating an antigen-antibody complex between a mutant protein and an antibody thereof

Also, available in accordance with still another aspect of the disclosure is a protein chip in which an array of antibodies against biomarker panel proteins is immobilized at predetermined loci and high density on a substrate. The protein chip assay may comprise isolating total proteins from a sample, hybridizing the isolated proteins with the protein chip to form an antigen-antibody complex, reading the information displayed on the protein chip to identify the presence of a protein of interest, and determining the onset of intractable epilepsy.

When an mTOR mutant gene or protein is detected using any of the detection methods, diagnosis can be made of the onset of intractable epilepsy caused by malformations of cortical developments.

Further disclosed is a technique of constructing an epilepsy model, comprising inducing intractable epilepsy with the genetic mutation and protein mutation.

A particular aspect of the disclosure pertains to a composition for inducing intractable epilepsy, comprising at least one selected from among mTOR, TSC1, TSC2, AKT3, and PIK3CA mutant genes or proteins.

Also, contemplated in accordance with another aspect of the disclosure is an intractable epilepsy-induced animal into which a mutant gene or protein of at least one selected from among mTOR, TSC1, TSC2, AKT3 and PIK3CA is introduced.

Further disclosed is a method for inducing intractable epilepsy, comprising introducing a mutant gene or protein of at least one selected from among mTOR, TSC1, TSC2, AKT3 and PIK3CA into a cell, *ex vivo*.

As used herein, the term "induction" means effecting a change from a normal state into a pathological state. For the purpose of the present disclosure, the term "induction" as used in conjunction with epilepsy refers to induction of the onset of intractable epilepsy from a healthy state.

In some embodiments, epilepsy-induced cells can be prepared by introducing a mutant gene or protein of at least one of mTOR, TSC1, TSC2, AKT3, and PIK3CA into cells. The cells include brain cells or embryos. The cells into which the mutant gene or protein is introduced may be developed into an intractable epilepsy-induced animal When the mutant gene or protein is introduced, mTOR hyperactivation may occur, causing a failure in neuronal migration and a significant increase in S6K phosphorylation, with the consequent induction of epilepsy.

mTOR, TSC1, TSC2, AKT3, and PIK3CA mutant proteins with mutations on their wild-type amino acid sequences may be obtained from natural sources by extraction and purification using a method widely known in the art. Otherwise, the mutant proteins can be prepared by chemical synthesis (Merrifield, J. Amer. Chem. Soc. 85:2149-2156, 1963) or by recombinant DNA technology.

For chemical synthesis, a polypeptide synthetic method widely known in the art may be used. In recombinant DNA technology, a nucleic acid encoding a protein having a mutation on its amino acid sequence is inserted into a suitable expression vector, which is then transformed into host cells. They are cultured to express the mutant protein, followed by recovering the mutant protein, having a mutation on the amino acid sequence thereof, from the host cells. After being expressed in selected host cells, the mutant protein can be isolated and purified using a typical biochemical separation technique, for example, treatment with a protein precipitant (salting-out), centrifugation, sonication, ultrafiltration, dialysis, various chromatographic techniques such as molecular sieve chromatography (gel filtration), adsorption chromatography, ion-exchange chromatography, and affinity chromatography. Typically, the techniques are used in combination to achieve a protein of high purity.

Nucleotide sequences coding for mTOR, TSC1, TSC2, AKT3, and/or PIK3CA proteins with a mutation on their wild-type amino acid sequences can be isolated from natural sources or prepared by chemical synthesis. Nucleic acids having such nucleotide sequences may be single- or double-stranded, and may be DNA molecules (genome, cDNA) or RNA molecules. For the chemical synthesis of nucleic acids, synthetic methods widely known in the art, such as those described in the literature (Engels and Uhlmann, Agnew Chem Int. Ed. Engl. 37:73-127, 1988) may be used, as exemplified by triester, phosphite, phosphoramidite and H-phosphonate methods, PCR and other autoprimer methods, oligonucleotide synthesis on solid supports or the like.

In a particular embodiment of the present disclosure, the mutant protein or gene may be introduced into cells, embryos or animals with the aid of a recombinant vector.

The term "vector", as used herein, refers to a means for introducing a nucleotide sequence encoding a protein of interest into a host cell. Plasmid vectors, cosmid vectors, and viral vectors fall within the scope of such a vector. A suitable expression vector may include a signal sequence or leader sequence for membrane targeting or secretion, in addition to regulatory elements such as a promoter, an operator, an initiation codon, a termination codon, a polyadenylation signal, and an enhancer, and may be constructed into various structures depending on the purpose. The initiation codon and the termination codon may be generally considered a portion of a nucleotide sequence encoding a protein of interest, necessarily functional in an individual to whom a genetic construct has been administered, and must be in a frame together with the coding sequence. The promoter may be generally constitutive or inducible. Further, the expression vector may include a selectable marker for selecting host cells containing the vector. For a replicable expression vector, a replication origin is necessary. A vector may replicate by itself, or may be incorporated into a host genomic DNA.

In one aspect of the disclosure, a vector carrying a gene is designed so that the gene is irreversibly incorporated into the genome of a host cell and is stably expressed for a long time in the cell.

The mutant protein or gene of one aspect of the disclosure may be introduced into cells, particularly brain cells. In addition, it may be introduced into embryos, preferably embryos at the stage of brain formation and development.

No particular limitations are imposed on the method of introducing proteins or genes. For example, a vector may be inserted into cells, using a method such as transformation, transfection or transduction. The vector introduced into cells continuously expresses a gene inserted thereinto in the cells so as to produce a mutant protein having a mutation on its wild-type amino acid sequence.

As used herein, the term "epilepsy-induced animal" is intended to refer to a non-human animal that is transformed to exhibit increased intracellular mTOR protein activity compared to normal cells. The transformation may be induced by introducing a vector expressing at least one mutant protein of mTOR, TSC1, TSC2, AKT3 and/or PIK3CA into cells. The transgenic animal with intractable epilepsy can be effectively used as an intractable epilepsy animal model.

As used herein, the term "animal model" or "disease model" is intended to refer to an animal that suffers from a disease similar to a specific human disease and thus can be used as a model for the study of etiology and pathogenesis. For use as an animal model, an animal should support prediction of the same effect as in humans and should be easily and reproducibly constructed. In addition, the disease of the animal model should have the same etiology as in humans, or should progress in a pattern similar to that in humans. Accordingly, mammalian vertebrates, which are similar in body structure to humans, for example with respect to the internal organs, immune system, and body temperature, and which suffer from diseases such as hypertension, cancer, immunodeficiency, etc. are suitable as animal models. The animals are particularly mammals, such as horses, sheep, pigs, goats, camels, antelopes, dogs, rabbits, mice, rats, guinea pigs, and hamsters, and more particularly rodents such as mice, rats, guinea pigs, and hamsters. A mouse is most frequently used in studying human diseases because of its various advantages including small size, potent fertility, easy feeding management, high resistance to disease, hereditary uniformity, and variety of strains. Another advantage for acceptance for use as an animal model is that mice can be transformed to exhibit diseases or symptoms identical or similar to those in humans.

The animal model of one aspect of the disclosure is an epilepsy model constructed by gene manipulation to express at least one of mutant proteins of mTOR, TSC1, TSC2, AKT3 and/or PIK3CA. Exhibiting the ability to induce intractable epilepsy, the mutant protein or gene of the present disclosure can be introduced into cells or embryos to construct an intractable epilepsy model.

In one aspect of the disclosure, for example, at least one of mutant proteins or genes of mTOR, TSC1, TSC2, AKT3 and PIK3CA is introduced into an animal embryo which can then be developed into an animal in which intractable epilepsy is induced. The mutant protein or gene can be introduced into embryos with the aid of a vector carrying the gene. No particular limitations are imposed on the technique of introducing the vector into an embryo. Particularly, the vector may be introduced into an embryo at the stage of cerebral cortex formation.

The animal model with epilepsy according to one aspect of the disclosure can be effectively applied to the study of gene functions, molecular mechanisms of epilepsy, and novel anti-epileptic drugs.

Another aspect of the disclosure addresses a composition, a kit and a method for preventing, ameliorating or treating intractable epilepsy or a disease causative of intractable epilepsy. The disease causative of intractable epilepsy includes malformations of cortical developments (MCD) such as FCD, Hemimegalencephaly and Tuberous Sclerosis Complex, Hippocampal Sclerosis (HS), and Sturge-Weber Syndrome (SWS).
The invention is defined in the appended claims.

### Advantageous Effects

As described above, the administration of an mTOR inhibitor, for example, rapamycin, everolimus, and/or the compounds of Chemical Formulas 1 to 4, into patients with brain somatic mutation-associated FCD can bring about a significant decrease in the frequency of onset of brain somatic mutation-associated intractable epilepsy or its causes, for example, spontaneous seizures, behavioral seizures, and electrographic seizures, and in the number or soma size of abnormal neurons in the cerebral cortex. Further disclosed is a biomarker panel effective for the diagnosis of intractable epilepsy and a technique for the diagnosis of intractable epilepsy using the same. Moreover, intractable epilepsy can be induced in accordance with one aspect of the disclosure, and epilepsy animal models thus constructed make it possible to study genetic functions, the molecular mechanisms of epilepsy, and novel anti-epileptic drugs.

### Description of Drawings

FIG. 1a shows post-operative brain MRI images of patients carrying mTOR mutations (FCD4, FCD6), and H&E staining of pathological samples from the patients. The arrowheads (white) indicate resected brain regions and the arrows (black) indicate cytomegalic neurons (Scale bar = 50um).
FIG. 1b shows sites of mTOR-associated somatic mutations as found in FCD patients through deep sequencing.
FIG. 1c shows evolutionarily conserved amino acid residues responsible for the mTOR-associated somatic mutations on the mTOR sequences.
FIG. 2a shows the results of Western blot analysis of S6 phosphorylation in mTOR mutation-expressing HEK293T cells. "P-S6" stands for phosphorylated S6 proteins, "S6" for S6 proteins, and "Flag" for flag proteins. "20% serum" represents a positive control with mTOR activity after exposure to 20% serum for 1 hr.
FIG. 2b shows the results of an *in-vitro* kinase assay for mTOR proteins in HEK293 cells expressing the mTOR mutations of the present disclosure.
FIG. 2c shows the results of an immunohistochemical assay for S6 phosphorylation level and soma size in pathological samples from patients with FCD-caused intractable epilepsy.
FIG. 2d shows the mean numbers of phosphorylated S6 proteins in representative cortical regions of patients with FCD-caused intractable epilepsy (number of counted cells=197-1182 per case).
FIG. 2e shows the mean soma sizes of neurons in representative cortical regions of patients with FCD-caused intractable epilepsy.

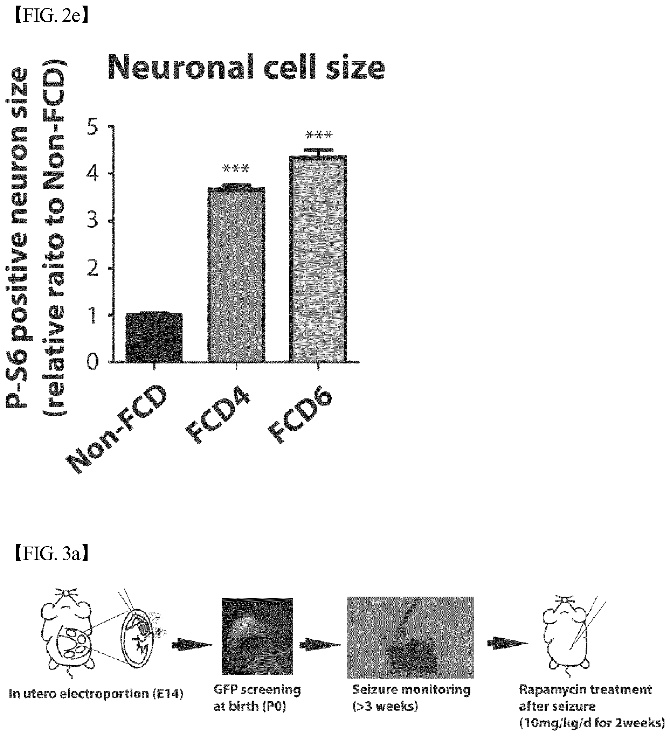

FIG. 3a shows schematic views of a procedure for electroporating embryos *in utero* at embryonic day 14 (E14) with a plasmid carrying mTOR mutations, followed by screening only mice exhibiting fluorescence with a flashlight (Electron Microscopy Science, USA) after birth, monitoring the mice for seizures through video-electroencephalography (video-EEG), and examining the effect of rapamycin after the onset of seizures. The *"in-utero* electroporation (E14)" schematically shows the electroporation of a plasmid carrying mTOR mutations at embryonic day 14, the "GFP screening at birth (P0)" schematically shows monitoring only of fluorescence-exhibiting mice with a flashlight (Electron Microscopy Science, USA) after the electroporated embryos are delivered, and the "Video-EEG monitoring (>3weeks)" schematically shows recording video-EEG with implanted electrodes after video monitoring of seizure onset from the time of weaning (>3weeks).
FIG. 3b shows the presence of spontaneous seizures in mice carrying mTOR mutations of the present disclosure, based on video-EEG recording. The "No. of GFP+pups" represents the number of mice that expressed GFP as a result of the introduction of mTOR mutations thereinto, and the "No. of mice with seizure" represents the number of mice that underwent seizures as a result of the introduction of mTOR mutations thereinto.
FIG. 3c shows frequencies of spontaneous seizures in mice carrying mTOR mutations of the present disclosure before and after rapamycin treatment. *p<0.05 and **p<0.01 (n=7-17 for each group, one-way ANOVA with Bonferroni's post test).
FIG. 3d shows changes in the soma size of GFP-positive neurons in mice carrying mTOR mutations of the present disclosure before and after rapamycin treatment.
FIG. 4 is a schematic view of the experimental design of the present disclosure, showing deep sequencing analysis in tissues from FCD patients, and *in-vitro* and *in-vivo* functional analysis.
FIG. 5a shows algorithms used to exploit brain-specific mutations using both Virmid (Genome Biology, 14(8), R90 (2013)) and MuTect software (Nature Biotechnology, 31, 213 (2013)) with regard to the deep sequencing results.
FIG. 5b shows sequencing counts of reference (Ref) and mutant (Mut) alleles, and percentages of mutated alleles from deep whole exome sequencing and amplicon sequencing of FCD patient samples.
FIG. 6 shows somatic mutations of FCD, found by deep whole exome sequencing, as colored bars in the collapsed mode of an Integrative Genomic Viewer (IGV).
FIG. 7 shows brain MRI images from FCD patients. Arrows highlight the affected cortical regions.
FIG. 8 shows domain organizations and 3-D structures of the mTOR kinase prepared using PyMol 1 (the PyMOL Molecular Graphics System, Schrodinger, LLC). "FAT" stands for FRAP, ATM, and TRRAP domains of mTOR, "FRB" for FKBP12-rapamycin binding domain, and "KD" for N and C lobes of the kinase domain. The activation and catalytic loops are indicated in red and blue, respectively. ATPrS and Mg²⁺ are shown as sticks and spheres, respectively. Identified mutation sites in FCD patients are labeled in red.
FIG. 9a shows the effects of rapamycin on HEK293K cells expressing mTOR mutations.
FIG. 9b shows the effects of rapamycin on HEK293K cells expressing mTOR mutations. "P-S6K" represents phosphorylated S6 proteins and "S6K" represents S6 proteins.
FIG. 9c shows the effects of the compounds of Chemical Formulas 1 to 4 and everolimus on HEK293K cells expressing mTOR mutations. "P-S6K" represents phosphorylated S6 proteins and "S6K" represents S6 proteins.
FIG. 10 shows the enrichment of the mTOR mutant allele of the present disclosure, as analyzed by Sanger sequencing after cytomegalic neurons with increased S6 phosphorylation, obtained from pathological tissues of patients with FCD-caused intractable epilepsy, are microdissected. NeuN-positive cytomegalic neurons with increased S6 phosphorylation are labeled with yellow dots. "LCM" represents cytomegalic cells microdissected through laser capture microdissection. For a control, bulk genomic DNA, extracted from brain samples without enrichment, was used. Scale bar, 100 µm.
FIG. 11a is a schematic view of the procedure for *in-utero* electroporation with a plasmid carrying mTOR mutations of the present disclosure at E14, followed by the analysis of brain coronal sections at E18.
FIG. 11b shows images of coronal sections of mouse brains electroporated with mTOR mutants of the present disclosure, representing the disruption of neuronal migrations and mTOR activity. "CP" stands for cortical plate, "IZ" for intermediate zone, "SVZ" for subventricular zone, "VZ" for ventricular zone, "Wild type" for the insertion of a wild-type mTOR plasmid, and "Relative intensity value" for the relative intensity of a GFP (green fluorescent protein) in each case.
FIG. 11c shows mTOR activity in the developing mouse neocortex after the introduction of the mTOR mutations of the present disclosure (Scale bars, 20µm, Error bars, s.e.m.).
FIG. 12a shows video-EEG records of spontaneous seizures in mice expressing the mTOR mutations of the present disclosure. "LF" stands for left frontal, "RF" for right frontal, "LT" for left temporal, and "RT" for right temporal.
FIG. 12b shows interictal spikes and nonconvulsive electrographic seizures in mice expressing the mTOR mutations of the present disclosure.
FIG. 12c shows a change in the frequency of interictal spikes in mice expressing the mTOR mutations of the present disclosure before and after rapamycin treatment.
FIG. 12d shows a change in the frequency of nonconvulsive electrographic seizures in mice expressing the mTOR mutations of the present disclosure before and after rapamycin treatment.
FIG. 12e shows the time of seizure onset in mice carrying a wild-type mTOR gene or a mTOR mutant of the present disclosure. (n=8-20 for each group). Error bars, s.e.m.
FIGS. 13 and 14 show the results of the treatment of HEK293T cells, expressing the mTOR mutations of the disclosure, with various mTOR inhibitors. "P-S6K" stands for phosphorylated S6 kinase, and "S6K" for S6 kinase.
FIG. 15 shows the results of Western blot analysis of HEK293T cells carrying wild-type or mutant TSC-1. (-) and (+) indicate a control and rapamycin treatment (200 nM), respectively. "P-S6K" stands for phosphorylated S6 kinase and "S6K" for S6 kinase.
FIG. 16 shows the results of Western blot analysis of HEK293T cells carrying wild-type or mutant TSC-2. (-) and (+) indicate a control and rapamycin treatment (200 nM), respectively. "P-S6K" stands for phosphorylated S6 kinase and "S6K" for S6 kinase.
FIG. 17 shows the results of Western blot analysis of HEK293T cells carrying wild-type or mutant AKT3. (-) and (+) indicate a control and rapamycin treatment (200 nM), respectively. "P-S6K" stands for phosphorylated S6K kinase and "S6K" for S6K kinase.
FIG. 18 shows the association of p.Arg22Trp and p.Arg204Cys mutations of TSC-1 with mTOR hyperactivation, as analyzed in Example 9. Immunoprecipitation assay results are given to identify the mechanism of TSC1 mutant-induced mTOR hyperactivation. "Empty" indicates non-treated cells.
FIG. 19 shows the results of the GTP-agarose pull down assay according to Example 9. The activity level of the TSC complex was analyzed by measuring the level of GTP-bound Rheb, a substrate of the TSC complex.
FIG. 20 shows results of the treatment of mutant mTOR-expressing HEK293T cells with rapamycin. **p<0.01 and ***p<0.001 (comparison with wild-type, n=3-5 for each group, one-way ANOVA with Bonferroni's post test)
FIG. 21 shows the results of the treatment of mutant mTOR-expressing HEK293T cells with rapamycin. "P-S6K" stands for phosphorylated S6 kinase, and "S6K" for S6 kinase.
FIG. 22 shows the results of the treatment of mutant mTOR-expressing HEK293T cells with the compounds of Chemical Formulas 1 to 4, and everolimus. "P-S6" stands for phosphorylated S6, and "S6" for S6 protein.
FIGS. 23a and 23b show the results of Western blot analysis of wild-type or mutant mTOR-expressing HEK293T cells before and after treatment with 6 different drugs according to Example 10. (-) and (+) indicate a control and rapamycin treatment (200 nM), respectively. "P-S6K" stands for phosphorylated S6 kinase, and "S6K" for S6 kinase.
FIGS. 24a and 24b show the results of Western blot analysis of wild-type or mutant TSC1-expressing HEK293T cells before and after treatment with 6 different drugs. (-) and (+) indicate a control and rapamycin treatment (200 nM), respectively. "P-S6K" stands for phosphorylated S6 kinase, and "S6K" for S6 kinase.
FIGS. 25a and 25b show the results of Western blot analysis of wild-type or mutant TSC1-expressing HEK293T cells before and after treatment with 6 different drugs. (-) and (+) indicate a control and rapamycin treatment (200 nM), respectively. "P-S6K" stands for phosphorylated S6 kinase, and "S6K" for S6 kinase.
FIGS. 26a and 26c show pathological samples from all FCD patients identified as carrying TSC1 and TSC2 mutations. "Non-FCD" stands for samples with normal brains, "P-S6" for phosphorylated S6 proteins, "NeuN" for a neuronal marker, and "Merge" for merged images of P-S6 and NeuN.
FIGS. 26b and 26d show percentages of cells expressing phosphorylated S6 in 4-5 cortical regions.
FIGS. 26e and 26f show the soma sizes of neuronal marker(NeuN)-positive neurons. *p<0.05, **P<0.001, ***P<0.0001 [relative to Non-FCD samples, one-way ANOVA with Bonferroni posttest]. Error bars, s.e.m Scale bars, 50um.
FIG. 27a shows the disruption of neuronal migration in TSC mouse models, resulting in malformations of cortical developments. "Control" indicates the absence of sgRNA, and red letters indicate percentages of cells expressing the plasmids. Scale bars, 250um.FIG. 27b shows the distribution of electroporated cells in the cortex. *p<0.05, ***P<0.0001 [Two-way ANOVA with Bonferroni posttest]. Error bars, s.e.m
FIG. 28 shows electrographic seizures measured in TSC mouse models with spontaneous seizures after administration with rapamycin. *p<0.05 and **p<0.01 (n=7-17 for each group, one-way ANOVA with Bonferroni's post test)

### Mode for Invention

A better understanding of the present invention may be obtained through the following examples which are set forth to illustrate, but are not to be construed as limiting the present invention.

### EXAMPLE 1: Identification of Gene by Whole Exome Sequencing, and Confirmation

### Example 1-1: Identification of 3 candidate mTOR mutations from 4 Patients through Whole Exome Sequencing

Deep whole exome sequencing (read depth 412-668x) was performed on brain tissue samples from four FCDII patients (designated FCD3, FCD4, FCD6, and FCD23, respectively). Selection was made of three candidate genetic mutations that were found simultaneously using the two algorithms Virmid and Mutect.

To obtain data of the whole exome sequencinge, libraries of sequences were prepared using the Agilent library preparation protocols (Agilent Human All Exon 50 Mb kit) according to the manufacturer's instructions. The libraries were subjected to sequencing on Hiseq2000 (Illumina). For more accurate analysis, sequencing was carried out with a read depth of about 500x, five- times higher than the general sequencing depth. The sequencing data was prepared into a file that can be analyzed using the Best Practices Pipeline suggested by Broad Institute (https://www.broadinstitute.org/gatk/).

### Example 1-2: Confirmation of 3 gene mutant candidates by site-specific amplicon sequencing and Identification of one genetic mutation (L2427P)

Site-specific amplicon was performed for the candidate mutations -(read depth, 100-347, 499x). The samples were obtained from the same brain tissue block through biological replication, thereby minimizing any unexpected sequencing artifacts or erroneous calls that can mimic low-frequency somatic mutations. For the site-specific amplicon sequencing, the samples were determined to have a mutation when the percentage of mutated reads exceeded 1%.

### Site-specific amplicon sequencing

Two pairs of primers carrying two target regions of mTOR target gene codon sites (amino acids Cys1483 and Leu2427) were designed (Table 2).

**TABLE2**

| Target region | primer | | SEQ ID NO |
|---|---|---|---|
| Chr1:11174301 ∼Chr1:11174513 | Forward | 5'-TAGGTTACAGGCCTGGATGG-3' | 11 |
| | Reverse | 5'-CTTGGCCTCCCAAAATGTTA-3' | 12 |
| Chr1:11217133 ∼Chr1:11217344 | Forward | 5'-TCCAGGCTACCTGGTATGAGA-3' | 13 |
| | reverse | 5'-GCCTTCCTTTCAAATCCAAA-3' | 14 |

Each primer had a patient-specific index, and only a single index was assigned to the sample from one patient so as to indicate patient origins of the base sequences upon the analysis of genetic mutations. PCR was performed on the target regions using the primers to amplify the nucleotide sequences of the two target regions. Subsequently, a DNA library was constructed using a Truseq DNA kit (Illumina) and libraries of the target genes were sequenced again on a Miseq sequencer (Illumina) (median read depth 135,424x). Using Bowtie2 (http://bowtie-bio.sourceforge.net/bowtie2/index.shtml), the sequences were aligned to a reference genome to generate analyzable files (bam files).

### Example 1-3: Sequencing analysis results

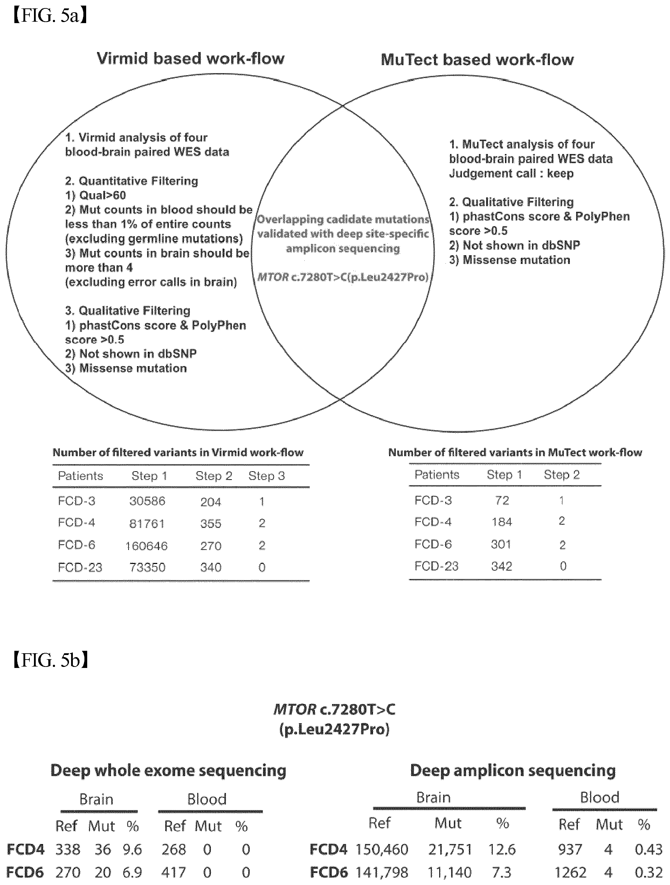

The use of two different sequencing platforms in biological replicates, as shown in FIG. 5a, revealed recurrent mTOR c.7280T>C encoding p.Leu2427Pro in two patients. The allelic frequencies in the affected brains, as shown in FIG. 5b, ranged from 9.6 to 12.6% in FCD4 and from 6.9 to 7.3% in FCD6.

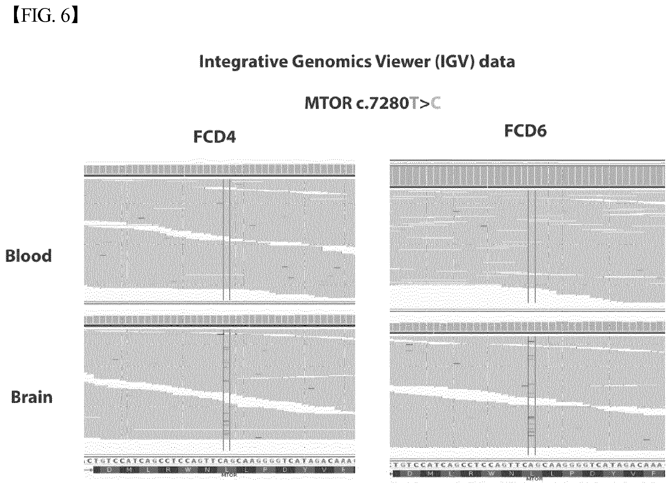

In addition, the mutation was negative in blood samples, as shown in FIGS. 5b and 6.

### EXAMPLE 2: Search of mTOR-Specific Gene Mutation in Large Cohort

Based on the data of mTOR-specific genetic mutations obtained from 4 patients in Example 1, deep sequencing of the mTOR gene was performed for a large FCDII cohort including 73 patients.

### Example 2-1: Collection of patient samples and Extraction of genomic DNA

From 73 focal cortical dysplasia (FCD)-caused intractable epilepsy patients undergoing epilepsy surgery, surgical brain tissues (1-2 g), saliva (1-2 ml), blood (about 5 ml), and formalin-fixed, paraffin-embedded brain samples were obtained with the consent thereof (Neurosurgery Dept. and Neurology Dept. of Severance Children's Hospital, Seoul, Korea). Genomic DNA was extracted from the freshly frozen brain, blood, saliva, and formalin-fixed, paraffin-embedded brain tissues using the following kits according to the instructions of the manufacturers:
Brain tissue: Qiamp mini DNA kit (Qiagen, USA), blood: Flexigene DNA kit (Qiagen, USA), saliva: prepIT2P purification kit (DNA Genotek, USA), formalin-fixed, paraffin-embedded brain tissue: Qiamp mini FFPE DNA kit (Qiagen, USA).

### Example 2-2: Sequencing

Hybrid capture sequencing of brain tissue samples from 73 FCDII patients were performed (read depth, 100-17,700×). PCR-based amplicon sequencing was carried out through site-specific amplicon sequencing (read depth, 100-347,499×, 73 patients) and mTOR amplicon sequencing (read depth, 100-20,210×, 59 patients).

For hybrid capture sequencing, an mTOR-specific probe was designed using SureDesign online tools (Agilent Technologies). Sequencing libraries were prepared using Agilent library preparation protocols according to the manufacturer's instructions. Sequencing was conducted using Hiseq2500 (Illumina) (median read depth 515x). The data obtained from the sequencing was prepared into analyzable files (bam files) using the Broad Institute best practice pipeline (https://www.broadinstitute.org/gatk/).

For mTOR amplicon sequencing, extracted genomic DNA was sequenced using customized MTOR amplicon (Truseq custom amplicon kit, Illumina) designed with illumine design studio (http://designstudio.illumina.com). DNA library preparation was performed according to the manufacturer's instructions. The libraries were sequenced on a Miseq sequencer (Illumina) (median read depth 1,647x). Analyzable bam files were generated using the BWA-MEM algorithm (http://bio-bwa.sourceforge.net).

### Example 2-3: Sequencing result

In order to find brain-specific, de novosomatic mutations, blood-brain paired whole exome sequencing data sets were analyzed using both Virmid (http://sourceforge.net/projects/virmid/) and Mutect (http://www.broadinstitute.org/cancer/cga/mutect). Only the somatic mutations that were commonly found in the two analytic approaches were used in subsequent experiments.

Of the mutations commonly found in the hybrid capture sequencing and the PCR-based amplicon sequencing, only those meeting selection standards (depth 100 or greater, and 3 or more mutated calls (mapping quality 30 or higher) were selected as disease-related candidates.

For all 2508 CRAM files (compressed files) downloaded from the 1000 Genomes Project FTP, 9 somatic mutation positions were found (chr1:11298590 for c.1871G>A, chr1:11217330 for c.4348T>G, chr1:11217231 for c.4447T>C, chr1:11199365 for c.5126G>A, chr1:11188164 for c.5930C>A, chr1:11184640 for c.6577C>T, chr1:11184573 for c.6644C>T, chr1:11174395 for c.7280T>C and c.7280T>A). All of the 9 genomic positions were negative for the somatic mutations meeting the selection standards. Accordingly, the gene mutations identified in the present disclosure were found to be disease-specific.

### Example 2-4: Sequencing result

Overlapping mutations in both hybrid capture sequencing (73 patients) and mTOR amplicon sequencing (59 patients) were detected in order to obtain a total of 9 true candidate variants (inclusive of mutations found in Example 1).

To rigorously exclude any potential sequence artifacts and erroneous calls, variants were considered as true only when identified variants (> 1% mutated reads) were reproducible in both hybrid capture and amplicon sequencing as well as in multi-samples.

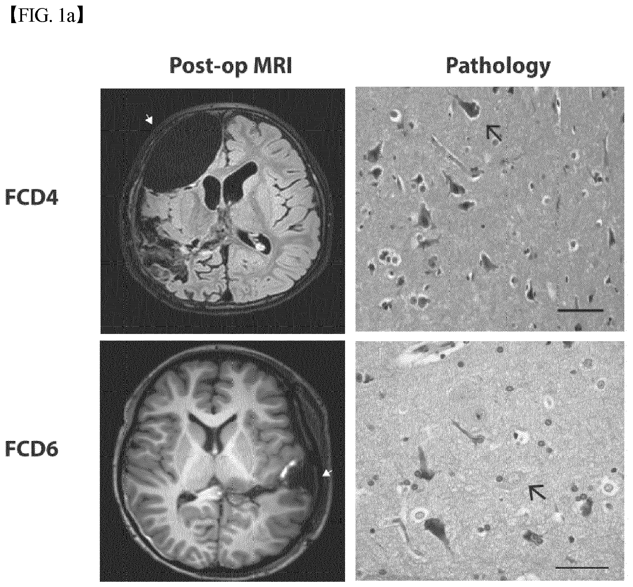

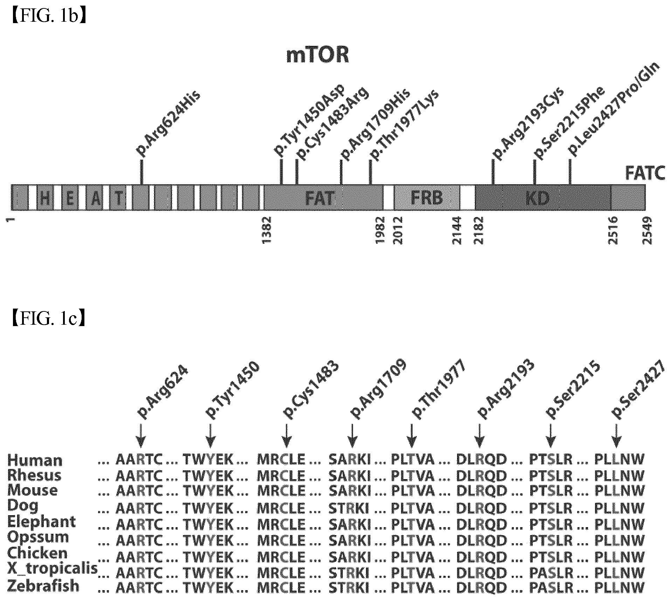

This analysis, as shown in FIG. 1b, revealed another 10 FCDII patients carrying 8 different somatic mutations in MTOR: mTOR c.1871G>A (p.Arg624His), c. 4348T>G (p.Tyr1450Asp), c.4447T>C (p.Cys1483Arg), c.5126G>A (p.Arg1709His), c.5930C>A (p.Tbr1977Lys), c.6577C>T (p.Arg2193Cys), c.6644C>T (p.Ser2215Phe), and c.7280T>A (p.Leu2427Gln). In total, 15.6% of the participants (12/77) were positive for 9 different brain somatic mutations in mTOR (Table 3).

**TABLE 3**

| Patient | Age upon Surgery | Sex | Pathology | mTOR gene mutation | mTOR protein mutation |
|---|---|---|---|---|---|
| FCD 4 | 5 years 2 months | F | Consistent with FCDIIa (Cortical dyslamination/ Dysmorphic neurons) | c.7280T>C | p.Leu2427Pro |
| FCD 6 | 5 years | F | Ditto | c.7280T>C | p.Leu2427Pro |
| FCD 91 | 7 years 1 month | F | Ditto | c.6577C>T | p.Arg2193Cys |
| FCD 104 | 1 year 2 months | M | Ditto | c.1871G>A | p.Arg624His |
| FCD 105 | 3 years 7 months | M | Ditto | c.5126G>A | p.Arg1709His |
| FCD 107 | 7 years 3 months | F | Consistent with FCDIIb (Cortical dyslamination / Dysmorphic neurons / balloon cells) | c.6644C>T | p.Ser2215Phe |
| FCD 113 | 10 years | F | Ditto | c.7280T>A | p.Leu2427Gln |
| FCD 116 | 7 years 9 months | M | Ditto | c.5930C>A | p.1M977Lys |
| FCD 121 | 11 months | M | Ditto | c.4348T>G | p.Tyr1450Asp |
| FCD 128 | 4 years 4 months | F | Ditto | c.4447T>C | p.Cys1483Arg |
| FCD 143 | 2 years 10 months | F | Ditto | c.6644C>T | p.Ser2215Phe |
| FCD 145 | 4 years 1 month | F | Ditto | c.5930C>A | p.Thr1977Lys |

All identified mutations were negative for all available saliva and blood samples from mutation-positive patients. 100% of the exomes from the 1000 Genomes database were mutation-negative. Among the mutations, p.Thr1977Lys, p.Ser2215Phe, and p.Leu2427Pro were recurrently found in two patients. All mutation-positive patients were found to have a single mTOR mutation. The allelic frequencies of identified mutations range from 1.26% to 12.6%. As can be seen in FIG. 1c, the affected amino acids are evolutionarily conserved.

### EXAMPLE 3: Identification of mTOR Mutation-Induced mTOR Hyperactivation

To determine whether mTOR p.Tyr1450Asp, p.Cys1483Arg, p.Leu2427Gln, and p.Leu2427Pro lead to mTOR hyperactivation, HEK293T cells were transfected with wild-type or mutant mTOR vectors, and S6 phosphorylation, a well-known biomarker of mTOR activation along with S6K phosphorylation, was subjected to Western blot analysis.

### Example 3-1: Mutagenesis and mTOR mutant construct

A pcDNA3.1 flag-tagged wild-type mTOR construct was provided from Dr. Kun-Liang Guan at University of California, San Diego. This construct was used to generate mTOR mutant vectors (Y1450D, C1483R, L2427Q and L2427P) with a QuikChange II site-directed mutagenesis kit (200523, Stratagene, USA).

To construct pCIG-mTOR mutant-IRES-EGFP vectors, an annealing primer set [forward primer 5'-AATTCCAATTGCCCGGGCTTAAGATCGATACGCGTA-3' (SEQ ID NO. 15) and reverse primer 5'-ccggtacgcgtatcgatcttaagcccgggcaattgg-3' (SEQ ID NO. 16)] were inserted into pCIG2 (CAG promoter-MCS-IRES-EGFP) to generate pCIG-C1 having new restriction enzyme sites MfeI and MIuI. Using the primers [hmTOR-MfeI-flag-F; gATcACAATTGTGGCCACCATGGACTACAAGGACGACGATGACAAGatgc (SEQ ID NO. 17) and hmTOR-MluI-R;tgatcaACGCGTttaccagaaagggcaccagccaatatagc (SEQ ID NO. 18)], PCR fragments corresponding to wild-type and mutated mTOR genes were subcloned into the MfeI and MluI sites of pCIG-C1 to construct pCIG-mTOR wild type-IRES-EGFP and pCIG-mTOR mutant-IRES-EGFP vectors, respectively. Primers for mutagenesis are listed in Table 4, below.

**TABLE 4**

| Name | Primer | | SEQ ID NO |
|---|---|---|---|
| Y1450D | Forward | 5'-TCGTGCAGTTTCTCATCCCAGGTAGCCTGGATC-3' | 19 |
| | Reverse | 5'-GATCCAGGCTACCTGGGATGAGAAACTGCACGA-3' | 20 |
| C1483R | Forward | 5'-GGCCTCGAGGCGGCGCATOCGGC-3' | 21 |
| | Reverse | 5'-GCCGCATGCGCCGCCTCGAGGCC-3' | 22 |
| L2427Q | Forward | 5'-GTCTATGACCCCTTGCAGAACTGGAGGCTGATG-3' | 23 |
| | Reverse | 5'-CATCAGCCTCCAGTTCTGCAAGGGGTCATAGAC-3' | 24 |
| L2427P | Forward | GTCTATGACCCCTTGCCGAACTGGAGGCTGATG | 25 |
| | Reverse | CATCAGCCTCCAGTTCGGCAAGGGGTCATAGAC | 26 |

### Example 3-2. Transfection with wild-type and mutant mTOR vectors and Western blot

HEK293T cells (Thermo Scientific) was cultured in DMEM (Dulbecco's Modified Eagle's Medium) containing 10% FBS at 37°C and 5% CO₂. The cells were transfected with empty flag-tagged vector, flag-tagged mTOR wild type, and flag-tagged mTOR mutants using jetPRIME transfection reagent (Polyplus, France). The cells were serum-starved with DMEM containing 0.1% FBS for 24 hours after transfection and then incubated at 37°C and 5% CO₂ in PBS containing 1 mM MgCl₂ and CaCl₂ for 1 hour. The cells were lysed with PBS containing 1% Triton X-100 and Halt protease and phosphatase inhibitor cocktail (78440, Thermo Scientific, USA).

Proteins were resolved by SDS-PAGE and transferred to PVDF membranes (Millipore, USA). The membranes were blocked with 3% BSA in TBS containing 0.1% Tween 20 (TBST). They were washed 4 times with TBST. The membranes were each incubated overnight at 4°C with primary antibodies including a 1/1000 dilution of anti-phospho-S6 ribosomal protein (5364, Cell Signaling Technology, USA), anti-S6 ribosomal protein (2217, Cell Signaling Technology, USA), and anti-flag M2 (8164, Cell Signaling Technology, USA) in TBST. After incubation, the membranes were washed 4 times with TBST. They were incubated with a 1/5000 dilution of HRP-linked anti-rabbit or anti-mouse secondary antibodies (7074, Cell Signaling Technology, USA) for 2 hours at room temperature. After washing with TBST, immunodetection was performed using ECL reaction reagents.

### Example 3-3. Monitoring of S6 phosphorylation level in mutant mTOR-expression cell

An *in vitro* mTOR kinase assay was performed. To this end, the kinase activity of mTOR was assayed using a K-LISA mTOR activity kit (CBA055, Calbiochem, USA) according to the manufacturer's protocol The transfected cells (HEK293T cells) were lysed with TBS containing 1% Tween 20, Halt protease, and phosphatase inhibitor cocktail. One mg of total lysates was pre-cleared by adding 15 µl of protein G-beads (10004D, Life technologies, USA), and incubated at 4°C for 15 min. Anti-flag antibodies were added to the pre-cleared lysates and incubated overnight at 4°C. Then, 50 µl of 20% slurry protein G-beads was added and incubated at 4°C for 90 min. The supernatant was carefully discarded. The pelleted beads were washed 4 times with 500 µl of lysis buffer and once with the 1x kinase buffer of the K-LISA mTOR activity kit (K-LISA mTOR activity kit). The pelleted beads were resuspended in 50 µl of 2x kinase buffer and 50 µl of mTOR substrate (p70S6K-GST fusion protein), followed by incubation at 30°C for 30 min. The reaction mixture was incubated in a glutathione-coated 96-well plate and incubated at 30°C for 30 min. The phosphorylated substrate was detected using an anti-p70S6K-pT389 antibody, an HRP antibody-conjugate and a TMB substrate. Relative activities were measured by reading the absorbance at 450nm.

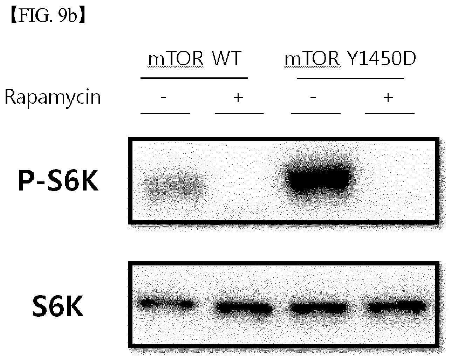

As is understood from the data of FIGS. 2a and 9b, S6 phosphorylation was robustly increased in mutant mTOR-expressing cells.

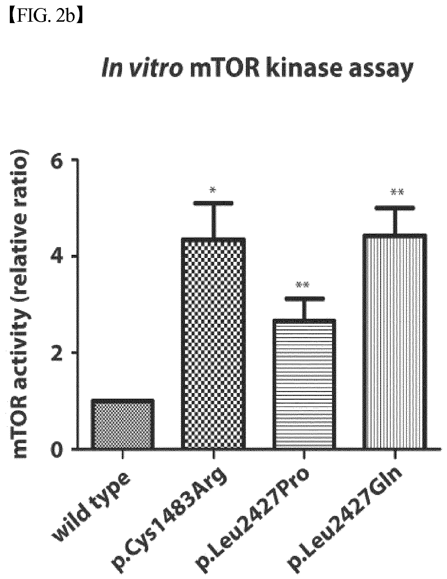

Wild-type and mutant mTOR proteins were pulled down from MTOR wild-type and mutant-expressing HEK293T cells, respectively, and assayed *in vitro* for mTOR kinase activity. As shown in FIG. 2b, pCys1483Arg, p.Leu2427Gln, and p.Leu2427Pro mTOR proteins had constitutively increased kinase activity.

### Example 3-4. Change in S6K phosphorylation after drug treatment

After treatment with drugs (rapamycin, everolimus, compounds of Chemical Formulas 1 to 4), the mutant mTOR-expressing cells were analyzed for S6K phosphorylation.

Transfection of mTOR mutants into HEK293T cells was carried out in the same manner as in Example 3-2. Then, the cells were serum-starved with DMEM containing 0.1% FBS for 24 hours and incubated at 37°C and 5% CO₂ in PBS containing 1 mM MgCl₂ and CaCl₂ for 1 hour, followed by treatment with rapamycin, everolimus, or compounds of Chemical Formulas 1 to 4 (Torin1, INK128, AZD8055, GSK2126458); Torin was purchased from TOCRIS. INK128, AZD8055, and GSK2126458 were provided from Selleckchem, and everolimus was purchased from LC laboratory. Subsequently, Western blotting was performed in the same manner as in Example 9.

As can be seen in FIGS. 9a and 9b, the phosphorylation of S6K in mutant mTOR-expressing cells was inhibited by rapamycin treatment.

In addition, the mutant mTOR-expressing cells were monitored for S6K phosphorylation after treatment with everolimus or the compounds of Chemical Formulas 1 to 4.

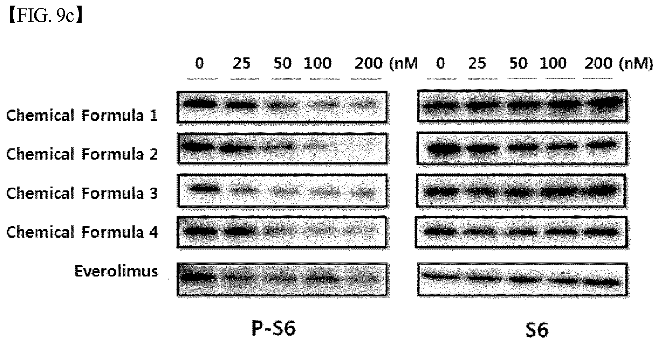

Likewise, everolimus or the compounds of Chemical Formulas 1 to 4 inhibited the phosphorylation of S6K in the mutant mTOR-expressing cells, as shown in FIG. 9c.

### Example 3-5. Change of S6K phosphorylation with various mTOR inhibitors

Cells expressing various mTOR mutations were treated in the same manner as in Example 3-2 with rapamycin, everolimus, or the compounds of Chemical Formulas 1 to 4 and monitored for S6K phosphorylation. The mTOR mutations were R624H, Y1450D, C1483R, R1709H, Y1977K, S2215F, L2427P, and L2427Q.

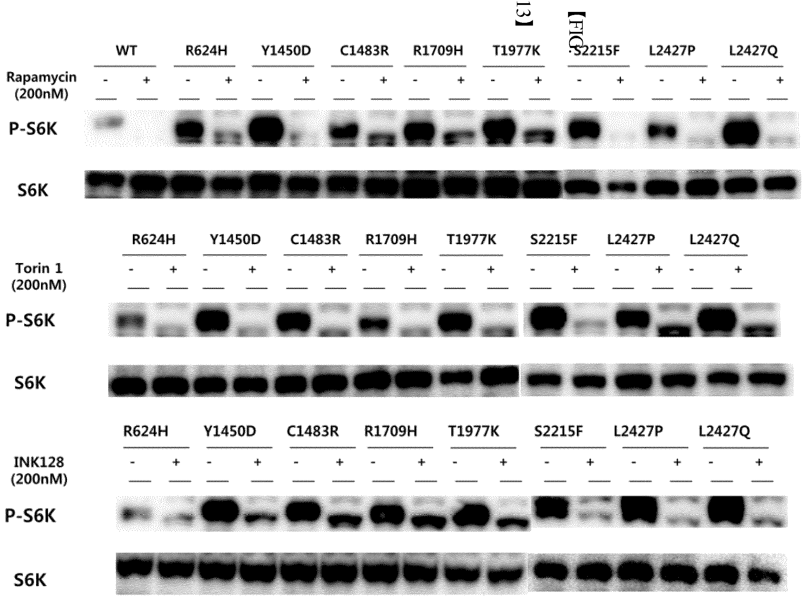

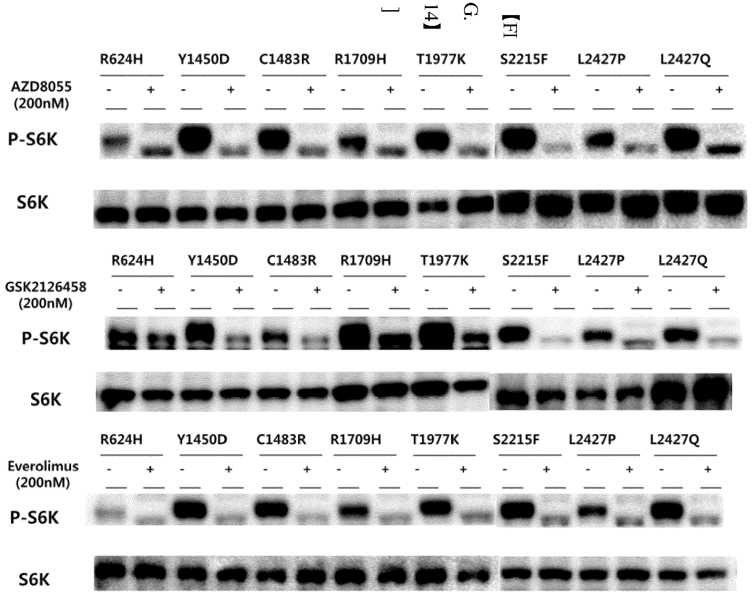

Briefly, the mutant mTOR-expressing cells were monitored for S6K phosphorylation level after treatment with everolimus or the compounds of Chemical Formulas 1 to 4. The results are depicted in FIGS. 13 and 14. As shown, the phosphorylation of S6K in all the mutant mTOR-expressing cells was inhibited by everolimus or the compounds of Chemical Formulas 1 to 4.

### EXAMPLE 4: mTOR Hyperactivation Induced by mTOR Mutation

### Example 4-1: Immunostaining of brain tissue section of FCD patient

To determine whether the affected brains of FCDII patients carrying mutations are associated with mTOR hyperactivation, immunostaining was performed for S6 phosphorylation and NeuN (a neuronal marker) in brain tissue sections obtained from FCDII patients carrying the p.Leu2427Pro mutation.

--Brain specimens that did not exhibit any malformations in cortical development (non-MCD) were collected in the operating room from the tumor-free margin of individual patients with glioblastoma as part of a planned resection, and were pathologically conformed to be normal brains without tumors. Surgical tissue blocks were fixed overnight in freshly prepared phosphate-buffered (PB) 4% paraformaldehyde, cryoprotected overnight in 20% buffered sucrose, and prepared into gelatin-embedded tissues blocks (7.5% gelatin in 10% sucrose/PB) before storage at -80°C. Cryostat-cut sections (10 um thick) were collected, placed on glass slides, and blocked in PBS-GT (0.2% gelatin and 0.2% Triton X-100 in PBS) at room temperature for 1 hr before staining with the following antibodies: rabbit antibody to phosphorylated S6 ribosomal protein (Ser240/Ser244) (1:100 dilution; 5364, Cell signaling Technology), and mouse antibody to NeuN (1:100 dilution; MAB377, Millipore). Subsequently, samples were washed in PBS and stained with the following secondary antibodies: Alexa Fluor 555-conjugated goat antibody to mouse (1:200 dilution; A21422, Invitrogen) and Alexa Fluor 488-conjugated goat antibody to rabbit (1:200 dilution; A11008, Invitrogen). DAPI included in a mounting solution (P36931, Life technology) was used for nuclear staining. Images were acquired using a Leica DMI3000 B inverted microscope. Cells positive for NeuN were counted using a 10x objective lens; 4-5 fields were acquired per subject within neuron-rich regions, and 100 or more cells were scored per region. The number of DAPI-positive cells represents total cell counts. Neuronal cell sizes were measured in NeuN-positive cells using the automated counting protocol of ImageJ software (http://rsbweb.nih.gov/ij/).

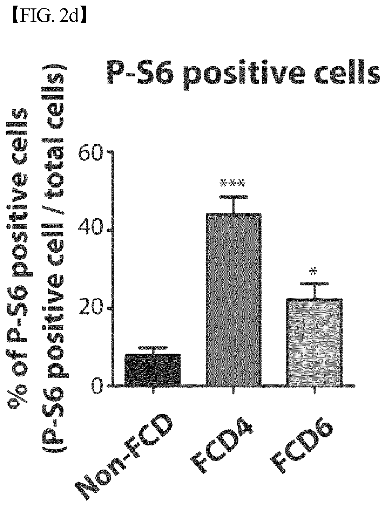

As seen in Fig. 2c, the results showed a marked increase in the number of neuronal cells positive for phosphorylated S6 in FCDII patients (FCD4 and FCD6) carrying p.Leu2427Pro mutation, but not in non-FCD brains, as shown in FIG. 2d. In addition, the cell sizes of phosphorylated S6-positive neurons were measured, and a robust increase in the soma size in pathological samples was observed, as shown in FIG. 2e.

### Example 4-2: Microdissection of S6 phosphorylation-increased, cytomegalic neurons in brain tissue of FCD patient and subsequent Sanger sequencing

Surgical tissue blocks were fixed overnight in freshly prepared phosphate-buffered (PB) 4% paraformaldehyde, cryoprotected overnight in 20% buffered sucrose, and prepared into gelatin-embedded tissue blocks (7.5% gelatin in 10% sucrose/PB) before storage at -80°C. Cryostat-cut sections (10 um thick) were collected, placed on glass slides, and blocked in PBS-GT (0.2% gelatin and 0.2% Triton X-100 in PBS) at room temperature for 1 hr before staining with the following antibodies: rabbit antibody to phosphorylated S6 ribosomal protein (Ser240/Ser244) (1:100 dilution; 5364, Cell signaling Technology) and mouse antibody to NeuN (1:100 dilution; MAB377, Millipore). Subsequently, samples were washed in PBS and stained with the following secondary antibodies: Alexa Fluor 555-conjugated goat antibody to mouse (1:200 dilution; A21422, Invitrogen) and Alexa Fluor 488-conjugated goat antibody to rabbit (1:200 dilution; A11008, Invitrogen).

DAPI, included in a mounting solution (P36931, Life technology), was used for nuclear staining. After immunofluorescence staining, phosphorylated S6 immunoreactive neurons (n = about 20 per case) were microdissected with a PALM Laser capture system (Carl Zeiss, Germany) and collected in an adhesive cap (Carl Zeiss, Germany).

Thereafter, genomic DNA was extracted from the collected neurons using a QiAamp microkit (Qiagen, USA), and mutation regions (mTOR c.7280T>C) were amplified by PCR using the primers: sense 5'-CCCAGGCACTTGATGATACTC-3' (SEQ ID NO. 27) and antisense, 5'-CTTGCTTTGGGTGGAGAGTT-3' (SEQ ID NO. 28).

The PCR products thus obtained were purified with a MEGAquick spin total fragment purification kit (Intron, Korea), followed by Sanger sequencing with the aid of the BioDye Terminator and an automatic sequencer system (Applied Biosystems).

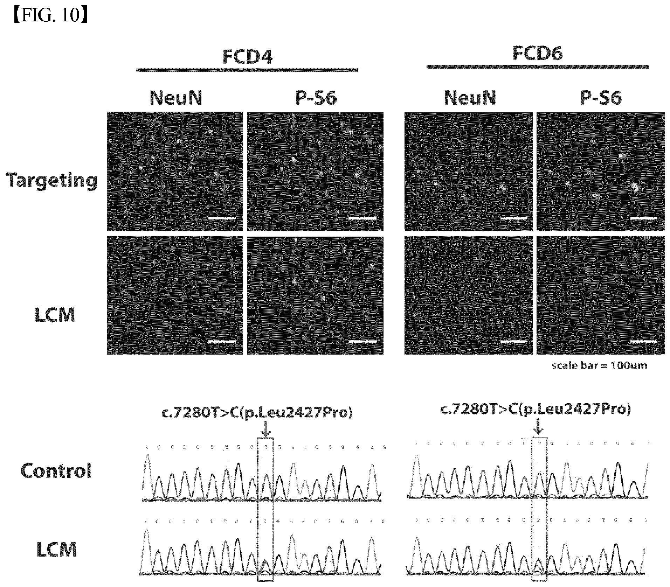

As shown in FIG. 10, moreover, the microdissection of cytomegalic neurons positive for phosphorylated S6 in the same pathological tissues showed the enrichment of the p.Leu2427Pro mutant allele in Sanger sequencing. These results suggest that the identified mTOR mutations are strongly associated with both the aberrant mTOR activation and the dysregulation of neuronal growth.

### EXAMPLE 5: Effect of mTOR Hyperactivation on Cerebral Development in Animal Model

The recurrent mutation p.Leu2427Pro was selected for *in vivo* functional analysis. *in utero* electroporation of mTOR mutant constructs was performed to analyze the effect of the mTOR mutations on cortical radial neuronal migration and S6 phosphorylation in mice.

### Example 5-1: Construction of animal model

Timed pregnant mice (E14) (Damul Science) were anesthetized with isoflurane (0.4 L/min of oxygen and isoflurane vaporizer gauge 3 during surgery).

The uterine horns were exposed, and a lateral ventricle of each embryo was injected using pulled glass capillaries with 2 µg/ml of Fast Green (F7252, Sigma, USA) combined with 2-3 µg of mTOR mutant plasmids, constructed in Example 3-1, carrying mTOR C1483Y, mTOR E2419K, and mTOR L2427P mutants. Plasmids were electroporated on the head of the embryo by discharging 50 V with the ECM830 eletroporator (BTX-Harvard apparatus) in five electric pulses of 100ms at 900-ms intervals.

### Example 5-2: Image analysis of mouse model

Embryonic mice were electroporated at embryonic day 14 (E14). Then, their brains were harvested after 4 days of development (E18) and fixed overnight in freshly prepared phosphate-buffered (PB) 4% paraformaldehyde, cryoprotected overnight in 30% buffered sucrose, and prepared into gelatin-embedded tissue blocks (7.5% gelatin in 10% sucrose/PB) before storage at -80°C.

Cryostat-cut sections (30 um thick) were collected and placed on glass slides. DAPI, included in a mounting solution (P36931, Life Technology) was used for nuclear staining. Images were acquired using a Leica DMI3000 B inverted microscope or a Zeiss LSM510 confocal microscope. Fluorescence intensities reflecting the distribution of electroporated cells within the cortex were converted into gray values and measured from the ventricular zone (VZ) to the cortical plate (CP, using ImageJ software (http://rsbweb.nih.gov/ij/). Mander's co-localization analysis was carried out using Fiji software (http://fiji.sc/wiki/index.php/Colocalization_Analysis).

### Example 5-3: Experiment result

As shown in FIG. 11a, mTOR wild-type or p.Leu2427Pro constructs containing an IRES-GFP reporter were electroporated into the developing mouse cortex at embryonic day (E) 14, and measured for cortical radial migration and the S6 phosphorylation of GFP-positive neurons at E18.

It was observed, as shown in FIG. 11b, that brain sections expressing the mTOR mutant construct showed a significant decrease in GFP-positive cells in the cortical plate (CP) and an increase in GFP-positive cells in the intermediate zone (IZ), the subventricular zone (SVZ), and the ventricular zone (VZ), thereby indicating the disruption of cortical radial neuronal migration.

In addition, as shown in FIG. 11c, mTOR mutant expressing GFP-positive cells were observed to coexist with cells having an elevated level of S6 phosphorylation in brain sections. These findings suggest that the identified mTOR mutations cause the aberrant activation of mTOR kinase protein and the disruption of proper cortical development *in vivo.*

### EXAMPLE 6: Identification of mTOR Hyperactivation-Induced Disease Phenotype in Animal Model

### Example 6-1. Identification of spontaneous seizure and abnormal neurons in animal model

A determination was made to see whether the focal cortical expression of mTOR induces spontaneous seizures in mice after *in utero* electroporation. Subsequent to *in utero* electroporation at E14, properly delivered mice pups at birth that showed GFP signals on the electroporated cortical region were selected, as shown in FIG. 3a.

Thereafter, continuous video-electroencephatographic monitoring of the mice was performed starting 3 weeks after birth. After weaning, the mice were monitored by video-recoding for 12 hrs per day until tonic-clonic seizures were observed. Then, mice with seizures were monitored using video-electroencephalography for 6 hrs per day over two days to characterize the spontaneous seizures with epileptic discharge.

Briefly, after weaning (> 3 weeks), seizures were observed only through video monitoring. Thereafter, electrodes for recoding electroencephalograms were surgically implanted. A total of five electrodes were located in the epidural layer: based on the bregma, two electrodes on the frontal lobes (AP+2.8mm, ML±1.5mm), two electrodes on the temporal lobes (AP-2.4mm, ML±2.4mm), and one electrode on the cerebellum. After more than 4 days of recovery from surgery, EGG signals were recorded between 6 p.m. and 2 a.m for 2-5 days (6 hrs per day). Signals were amplified with an amplifier (GRASS model 9 EEG/Polysomnograph, GRASS technologies, USA), and analyzed with the pCLAMP program (Molecular Devices, USA). Alternatively, a RHD2000 amplifier and board (Intan Technologies, USA) and MATLAB EEGLAB (http://sccn.ucsd.edu/eeglab) were used for analysis.

For EGG analysis, 10-12 h continuous recording data was analyzed for interictal spike and nonconvulsive electrographic seizure counts. 1-min samples were selected from the data at standardized preset time points separated by exactly 1 h.

Each 1-min sample was assessed for the number of interictal epileptiform spikes and nonconvulsive electrographic seizures therein by an observer who was unaware of the treatment of the mice. Interictal spikes were defined as fast (<200ms) epileptiform wave forms that occurred regularly and were at least twice the amplitude of the background activity. Nonconvulsive electrographic seizure episodes were counted when the EEG recording showed at least two connected spike-wave complexes (1-4Hz) with amplitudes as at least twice as background, and were observed simultaneously in the majority of the four recording channels per mouse.

Surprisingly, as shown in FIGS. 3b and 12a, more than 90% of the mice expressing mTOR mutant constructs displayed spontaneous seizures with epileptic discharge, including high-voltage fast activity, high-voltage spikes and waves, and low-voltage fast activity. These mutant mice also showed interictal spikes and electrographic seizures (FIG. 12b). The mice in which spontaneous seizures were induced were observed to exhibit systemic tonic-clonic seizures consisting of a tonic phase, a clonic phase, and a postictal phase, similar to those found in FCDII patients. Further, brain waves are characterized by synchronized multi-waves of low-voltage, fast activity in the tonic phase, high-voltage standing waves in the clonic phase, and synchronized attenuated amplitudes in the postictal phase. However, neither spontaneous seizures with epileptic discharges nor electrographic seizure were observed in control mice electroporated with the mTOR wild-type construct, as shown in FIG. 3b.

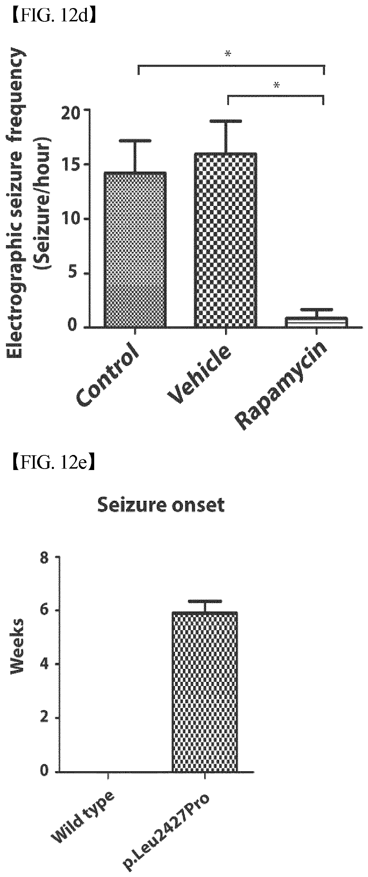

The average seizure onset of p.Leu2427Pro mice started on average roughly 6 weeks after birth (FIG. 12e), which is approximately equivalent to that of human FCDII patients (∼4 years). The seizure frequency was about 6 events per day (FIG. 3c).

After the confirmation of seizures, investigation was made to see whether the mice electroporated with mTOR mutant constructs showed abnormal neuronal morphology, such as cytomegalic neurons.

It was observed that the soma sizes of GFP-positive neurons were greatly increased in affected cortical regions of electroporated mice carrying mTOR mutations (FIG. 3d).

### Example 6-2. Effect of drug on spontaneous seizure and abnormal neuron

Animal models with spontaneous seizures or abnormal neurons were monitored after administration with rapamycin.

Briefly, rapamycin or everolimus (LC Labs, USA) was dissolved in 100% ethanol to give a 20mg/ml stock solution and stored at -20°C. Immediately before injection, the stock solution was diluted in 5% polyethyleneglycol 400 and 5% Tween80 to yield final concentrations of 1 mg/ml rapamycin and 4% ethanol. Mice were injected with 1 to 10 mg/kg rapamycin for 2 weeks (10 mg/kg/d intraperitoneal injection, two weeks).

Rapamycin, as shown in FIGS. 3c, 12c and 12d, almost completely freed the mTOR mutant construct-carrying mice from spontaneous seizures, with a simultaneous dramatic decrease in the onset frequency of interictal spikes and nonconvulsive electrographic seizure.

Also, abnormal soma sizes of neurons were reduced in the animal model administered with rapamycin, as shown in FIG. 3d.

### EXAMPLE 7: Identification of Mutation in Intractable Epilepsy Patient by Sequencing

Genomic DNA was extracted in substantially the same manner as that of Example 2 from samples from a total of 77 patients, listed in Examples 1 and 2, and subjected to hybrid capture sequencing and PCR-based amplicon sequencing. Of mutations found in both the sequencing analyses, those that met the selection standards (a total read depth of 100 or more, 3 or more mutated calls, and a mapping quality score of 30 or more) were detected in TSC1, TSC2, AKT3, and PIK3CA.

TSC1 c.64C>T (p.Arg22Trp), c.610C>T (p.Arg204Cys), c.2432G>T (p.Arg811Leu); TSC2 c.4639C>T (p.Val1547Ile); AKT3 c.740G>A (p.Arg247His), PIK3CA c.3052G>A (p.Asp1018Asn).

In eight of 51 patients negative for mTOR mutations, TSC1, TSC2, AKT3, and PIK3CA gene mutations were detected only in affected brain regions. Accordingly, 21 of a total of 77 intractable epilepsy patients were found to have mutations only in affected brain regions.

mTOR c.616C>T (pArg206Cys) mTOR c.1871G>A (p.Arg624His), c. 4348T>G (p.Tyr1450Asp), c.4447T>C (p.Cys1483Arg), c.5126G>A (p.Arg1709His), c.5930C>A (p.Thr1977Lys), c.6577C>T (p.Arg2193Cys), c.6644C>T (p.Ser2215Phe), and c.7280T>A (p.Leu2427Gln); TSC1 c.64C>T (p.Arg22Trp), c.610C>T (p.Arg204Cys), c.2432G>T (p.Arg811Leu); TSC2 c.4639C>T (p.Val1547Ile); AKT3 c.740G>A (p.Arg247His), PIK3CA c.3052G>A (p.Asp1018Asn).

**TABLE 5**

| Disease/gen der | Age at surgery | phathology | MRI result | protein | Modified nucleotide | Modified Amino acid | Hybrid Capture % Mutated allele | PCR amplicon sequencing% Mutated allele |
|---|---|---|---|---|---|---|---|---|
| FCD 4/female | 5yr 2m | Cortical dyslamination, Dysmorphic neurons, consistent with FCDIIa | No abnormal signal intensity | MTOR | c.7280T>C | p.Leu2427Pro | 7.94% | 12.6% |
| FCD 6/female | 5yr | Cortical dyslamination, Dysmorphic neurons, consistent with FCDIIa | No abnormal signal intensity | MTOR | c.7280T>C | p.Leu2427Pro | 6.90% | 728% |
| FCD 64/female | 6yr 9m | Cortical dyslamination, Dysmorphic neurons, consistent with FCDIIa | Cortical dysplasia involving left fronto-parietal lobe | TSCl | c.610C>T | pArg204Cys | 1.75% | 1.0% |
| HME 66/male | 2yr 8m | Cortical laminar disturbance with large giant neurons | Rt. hemimegalenc ephDaly | PIK3CA | c.3052G>A | p.Asp1018Asn | 1.03% | 2.30% |
| SWS 77/male | 11m | Cortical dyslamination, Dysmorphic neurons, consistent with FCDIIa | Difuse brain atrophy, Right hemisphere | MTOR | c.616C>T | p.Arg206Cys | 3.93% | 3.45% |
| FCD 81/female | 12yr | Cortical dyslamination, Dysmorphic neurons, consistent with FCDIIa | No abnormal signal intensity | TSC1 | c.64C>T | p.Arg22Trp | 2.81% | 2.0% |
| HS86/male | 13yr 2m | Hippocampal sclerosis | Suggestive of HS, left. | AKT3 | c.740G>A | pArg247His | 1.72% | 10% |
| FCD 91/female | 7yr 1m | Cortical dyslamination, Dysmorphic neurons, consistent with FCDIIa | Volume decrease of the left cerebral hemisphere and multifocal lesions in the WM | MTOR | c.6577C>T | pArg2193Cys | 2.99% | 1.26% |
| FCD 94/female | 10yr 3m | Cortical dyslamination, Dysmorphic neurons, consistent with FCDIIa | Subependymal heterotopia, Rt peri-trigone area | TSC2 | c.4639C>T | p.Val1547Ile | 1.19% | 1.55% |
| FCD 98/male | 14yr 3m | Cortical dyslaminati on, Dysmorphic neurons, consistent with FCDIIa | No abnormal signal intensity | TSC1 | c.64C>T | p.Arg22Trp | 2.52% | 1.98% |
| FCD 104/male | lyr 2m | Cortical dyslamination, Dysmorphic neurons, consistent with FCDIIa | Cortical dysplasia involving right precentral and postcentral gyri, | MTOR | c.1871G>A | pArg624His | 1.80% | 4.41% |
| FCD 105/male | 3yr 7m | Cortical dyslamination, Dysmorphic neurons, consistent with FCDIIa | No abnormal signal intensity | MTOR | c.5126G>A | pArg1709His | 1.63% | 1.52% |
| FCD 107/female | 7yr 3m | Cortical dyslamination, dysmorphic neurons, balloon cells, consistent with FCDIIb | Cortical Dysplasia involving left occipitoparietal lobe and precentral gyrus | MTOR | c.6644(>T | p.Ser2215Phe | 2.41% | 2.11% |
| FCD 113/female | 10yr | Cortical dyslamination, dysmorphic neurons, balloon cells, consistent with FCDIIb | Cortical dysplasia involving left occipital and parietal lobe | MTOR | c.7280T>A | p.Leu2427Gln | 3.05% | 5.11% |
| FCD 116/male | 7yr 9m | Cortical dyslamination, dysmorphic neurons, balloon cells, consistent with FCDIIb | Cortical dysplasia involving left superior frontal gyrus | MTOR | c.5930C>A | p.Thr1977Lys | 3.25% | 2.93% |
| FCD 121/male | 11m | Cortical dyslamination, dysmorphic neurons, balloon cells, consistent with FCDIIb | Cortical dysplasia involving entire right lobe and left superior/middl e frontal gyrus | MTOR | c.4348T>G | p.Tyr1450Asp | 2.64% | 3.76% |
| FCD 123/female | 12yr 4m | Cortical dyslamination, dysmorphic neurons, balloon cells, consistent with FCDIIb | Cortical Dysplasia involving right frontal lobe | TSC1 | c.64C>T | p.Arg22Trp | 2.21% | 1.37% |
| FCD 128/female | 4yr 4m | Cortical dyslamination, dysmorphic neurons, balloon cells, consistent with FCDIIb | Cortical dysplasia, right frontal lobe | MTOR | c.4447T>C | p.Cys1483Arg | 6.38% | 9.77% |
| HME141fe male | 1yr 9m | Cortical laminar disturbance with large giant neurons | Lt. hemimegalenc ephaly | TSC1 | c2432G>T | pArg811Leu | 1.03% | 1.68% |
| FCD 143/female | 2yr 10m | Cortical dyslamination, dysmorphic neurons, balloon cells, consistent with FCDIIb | No abnormal signal intensity | MTOR | c.6644C>T | p.Ser2215Phe | 2.82% | 2.33% |
| FCD 145/female | 4yr 1m | Cortical dyslamination, dysmorphic neurons, balloon cells, consistent with FCDIIb | Cortical dysplasia involving left precentral gyrus | MTOR | c.5930C>A | p.Thr1977Lys | 1.46% | 1.51% |

### EXAMPLE 8: in vivo Analysis of mTOR Hyperactivation

### 8-1. Mutagenesis and Construction of TSC1, TSC2, and AKT3 mutant constructs

pcDNA3 carrying HA-tagged wild-type TSC1, TSC2 or AKT3 (pcDNA3 HA-tagged wild-type TSC1, TSC2, AKT3 construct) was purchased from Addgene (USA). The construct was used to generate mutant vectors with a QuikChange II site-directed mutagenesis kit (200523, Stratagene, USA).

pcDNA3 carrying HA-tagged wild-type TSC1, TSC2 or AKT3 (pcDNA3 HA-tagged wild-type TSC1, TSC2, AKT3 construct) was purchased from Addgene (USA). In the pcDNA3 TSC1, TSC2, AKT3 wild-type vector, the mutagenesis of TSC-1 R22W and R204C was achieved by use of TSC-1 R22W-F and R22W-R primers for R22W mutagenesis and by use of TSC-1 R204C-F and R204C-R primers for R204C mutagenesis. TSC-2 V1547I-F and V1547I-R primers were used for TSC-2 V15471 mutagenesis in the pcDNA3 TSC2 wild-type vector. For the mutagenesis of AKT3 R247H in the pcDNA3 AKT3 wild-type vector, R247H-F and R247H-R primer were designed.

A QuikChange II site-directed mutagenesis kit (200523, Stratagene, USA) was used to create point mutations. Because each primer has a site-specific point mutation sequence, a mutation is induced in copies of the sequence upon PCR. Primers useful for the mutagenesis are listed in Table 6, below.

**TABLE 6**

| gene | Location n of Modification | | Primer | | SEQ ID NO |
|---|---|---|---|---|---|
| TSC-1 | C64T | R22W | TSC-1 R22W-F | gtcacgtcgtcccacacacccagcatg | 29 |
| | | | TSC-1 R22W-R | catgctgggtgtgtgggacgacgtgac | 30 |
| | C610T | R204C | TSC-1 R204C-F | ctttcatactgtaatgagaacacaaaaaegagacgaagttgca | 31 |
| | | | TSC-1 R204C-R | tgcaacttcgtctcctttttgtgttctcattacagtatgaaag | 32 |
| TSC-2 | G4639A | V1547I | TSC-2 V1547I-F | tctccaacatacaggatggcgatcttgtgggtg | 33 |
| | | | TSC-2 V1547I-R | cacccacaagatcgccatcctgtatgttggaga | 34 |
| AKT3 | G740A | R247H | AKT3 R247H-F | caccatagaaacgtgtgtggtcctcagagaacacc | 35 |
| | | | AKT3 R247H-R | ggtgttctctgaggaccacacacgtttctatggtg | 36 |

### 8-2. Cell culture, Transfection, and Western blot

In order to examine whether TSC-1, TSC-2 or AKT3 mutation causes the aberrant activation of mTOR, wild-type or mutant vectors were transfected into HEK293T and the phosphorylation of SK6, widely known as an mTOR gene marker, was analyzed by Western blotting.

Briefly, HEK293T cells (Thermo Scientific) were cultured in DMEM (Dulbecco's Modified Eagle's Medium) supplemented with 10% FBS at 37°C and 5% CO₂. The cells were transfected with empty flag-tagged vector, HA-tagged TSC1 wild-type, HA-tagged TSC2 wild-type, HA-tagged AKT3 wild-type, HA-tagged TSC1 mutant, HA-tagged TSC2 mutant and HA-tagged AKT3 mutant, respectively, using jetPRIME transfection reagent (Polyplus, France).

The cells were serum-starved with 0.1% FBS in DMEM for 24 hours after transfection and then incubated at 37°C and 5% CO₂ in PBS containing 1 mM MgCl₂ and CaCl₂ for 1 hour. The cells were lysed with PBS containing 1% Triton X-100 and Halt protease and phosphatase inhibitor cocktail (78440, Thermo Scientific, USA). Proteins were resolved by SDS-PAGE and transferred to PVDF membranes (Millipore, USA). The membranes were blocked with 3% BSA in TBS containing 0.1% Tween 20 (TBST). They were washed 4 times with TBST. The membranes were incubated overnight with primary antibodies including a 1/1000 dilution of anti-phospho-S6 ribosomal protein (5364, Cell Signaling Technology, USA), anti-S6 ribosomal protein (2217, Cell Signaling Technology, USA), and anti-flag M2 (8164, Cell Signaling Technology, USA) in TBST at 4°C, respectively. After incubation, the membranes were washed 4 times with TBST. They were incubated with a 1/5000 dilution of HRP-linked anti-rabbit or anti-mouse secondary antibodies (7074, Cell Signaling Technology, USA) for 2 hours at room temperature. After washing with TBST, immunodetection was performed using ECL reaction reagents.

### 8-3. Treatment of mutant-expressing cells with rapamycin and Western blot

After treatment with rapamycin, the mutant-expressing cells of Example 8-2 were monitored for S6K phosphorylation.

Briefly, HEK293T cells were transfected with mTOR, TSC1, TSC2, or AKT3 mutants in the same manner as in Example 8-2. The transfected cells were starved for 24 hrs with empty DMEM and incubated at 37°C and 5% CO₂ for 1 hr with rapamycin in PBS containing 1 mM MgCl₂ and CaCl₂, followed by Western blotting in the same manner as in Example 2-2.

### 8-4. Experiment data

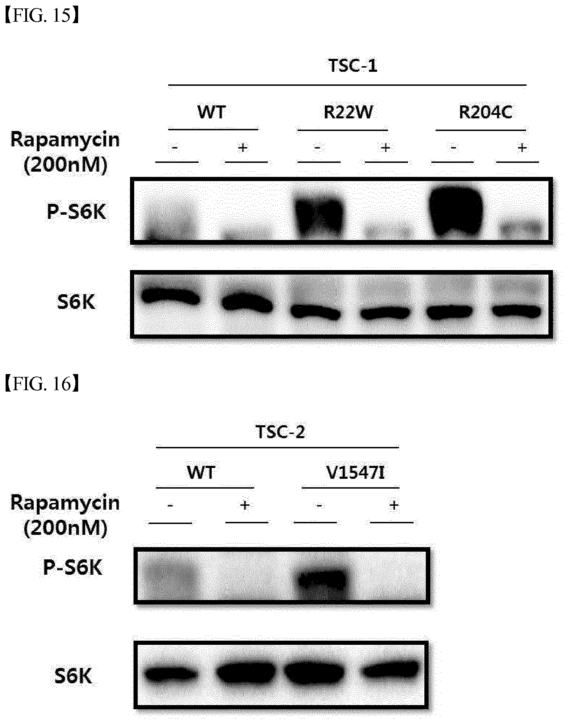

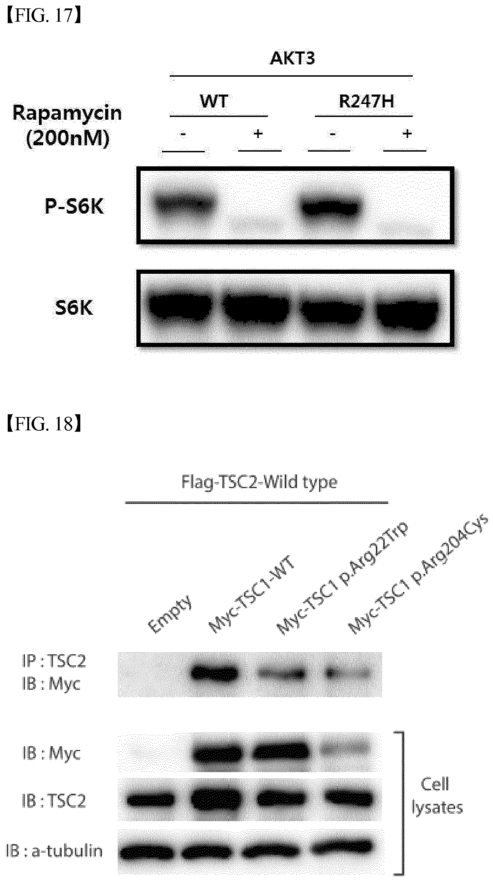

In order to examine whether the p.Arg22Trp and p.Arg204Cys mutations of TSC-1, the p.Val1547Ile mutation of TSC-2, or the p.Arg247His mutation of AKT3 induces mTOR activation, HEK293T cells were transfected with vectors carrying TSC1, TSC2, and AKT3 wild-type and mutants, and S6K phosphorylation, a well-known index for mTOR mutation, was monitored via Western blotting. The mutant-expressing cells were treated with rapamycin before monitoring the phosphorylation of S6K, as described in Examples 8-2 and 8-3. The results are depicted in FIGS. 15 to 17, and can be described for individual mutant genes) as follows:
(1) *in vitro* activity of TSC-1 mutant
   As can be seen in FIG. 15, S6K phosphorylation was increased in cells expressing mutant TSC-1, and reduced by rapamycin treatment.
(2) *in vitro* activity of TSC-2 mutant
   As can be seen in FIG. 16, S6K phosphorylation was increased in cells expressing mutant TSC-2, and reduced by rapamycin treatment.
(3) *in vitro* activity of AKT3 mutant
   As can be seen in FIG. 17, S6K phosphorylation was increased in cells expressing mutant AKT3, and reduced by rapamycin treatment.

### EXAMPLE 9: Activation of mTOR Pathway by TSC1 and TSC2 Mutants

### 9-1: Immunoprecipitation assay

To examine whether mutations in *TSC1* and *TSC2* disrupt the formation of the TSC complex, immunoprecipitation assays were conducted on wild-type and mutant TSC1 or TSC2-expressing HEK293T cells. In this regard, TSC1 and TSC2 mutant proteins prepared in the same manner as in Example 8-3 were incubated overnight with an anti-TSC2 antibody (3990, Cell signaling Technology, USA) or an anti-myc antibody (2276, cell signaling technology, USA), and then with protein A+G magnetic beads for 2 hrs. After washing with PBS containing 1% Triton-X100, the beads were incubated in an SDS buffer at 37°C for 10 min. After being eluted, proteins were resolved on SDS/PAGE gel and transferred to a PVDF membrane. Immunoblotting was performed in the same manner as in Example 2-3.

The results are depicted in FIG. 18. It was found that the TSC-1 p.Arg22Trp and p.Arg204Cys mutations located near the TSC2 binding domain strongly inhibited TSC1 binding to TSC2. These data imply that the TSC1 mutant disrupted the formation of the TSC complex, leading to the hyperactivation of mTOR kinase.

### 9-2: GTP-agarosepull down assay

Cells were harvested in a lysis buffer (20 mM Tris-HCl pH: 7.5, 5 mM MgCl2, 2 mM PMSF, 20 µg/mL leupeptin, 10 µg/mL aprotinin, 150 mM NaCl and 0.1% Triton X-100) and then lysed by sonication for 15 sec. The cell lysates were centrifuged at 4°C and 13,000 g. The supernatant was separated and incubated with 100µl of GTP-agarose beads (Sigma-Aldrich, cat no. G9768) at 4°C for 30 min. The beads were washed with a lysis buffer and again incubated overnight with the supernatant. GTP-bound proteins were extracted and visualized by immunoblots.

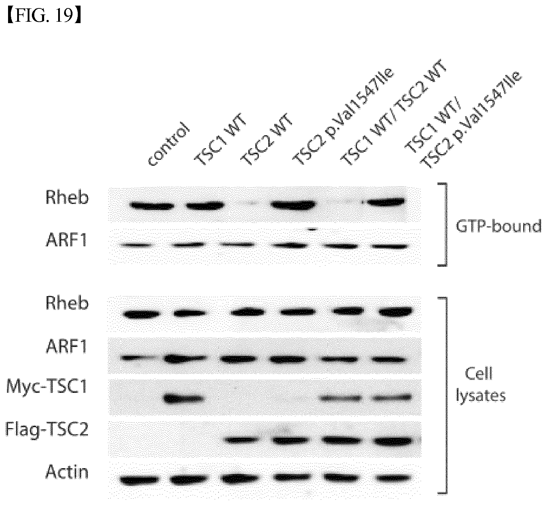

The expression of GTP-bound Rheb protein was found to decrease in wild-type TSC2-expressing cells, but not in TSC2 p.Val1547Ile mutant-expressing cells because the GAP (GTPase activating protein) activity of TSC2 was decreased (FIG. 19( 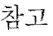 2 fig. 2)), suggesting that the TSC2 mutant reduces the function of the GAP domain, thereby activating the mTOR pathway.

### EXAMPLE 10: Monitoring of S6K Phosphorylation Level in Drug-Treated, Mutant mTOR-Expressing Cells

### 10-1. Mutant mTOR-expressing cell

Mutant mTOR-expressing cells were treated with drugs (rapamycin, everolimus, compounds of Chemical Formulas I to 4) and monitored for S6K phosphorylation level.

In this regard, HEK293T cells were transfected with the mutants in the same manners as in Examples 8-2 and 8-3, serum-starved for 24 hrs with 01.% FBS in DMEM, and incubated at 37°C and 5% CO₂ for 1 hr with 1 mM MgCl₂ and CaCl₂ in PBS before treatment with rapamycin, everolimus, or the compounds of Chemical Formulas 1 to 4 (Torinl, INK128, AZD8055, GSK2126458): Torin was purchased from TOCRIS; INK128, AZD8055 and GSK2126458 were from Selleckchem; and everolimus was from LC laboratory. Subsequently, Western blotting was performed in the same manner as in Example 2-4.

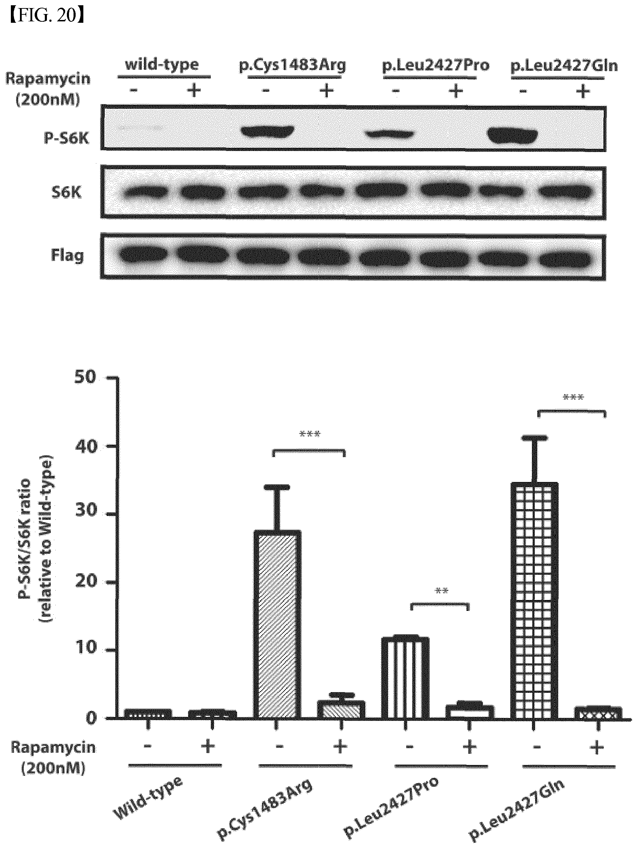

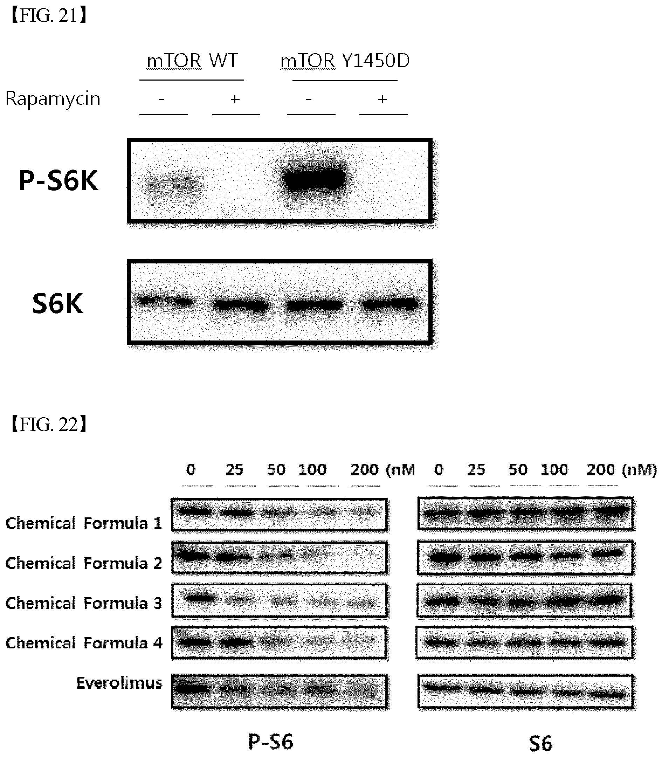

As is understood from the data of FIGS. 20 and 21, S6K phosphorylation was inhibited in mutant mTOR-expressing cells. Briefly, FIG. 20 shows levels of S6K phosphorylation in cells respectively expressing the mOTR mutants C1483R, L2427P and L2427Q after rapamycin treatment. FIG. 21 shows levels of S6K phosphorylation in mTOR mutation Y1450D-expressing cells after rapamycin treatment.

FIG. 22 shows levels of S6 in mTOR mutation L2427P-expressing cells after treatment with 0, 25, 50, 100, and 200nM of rapamycin.

S6K phosphorylation was monitored following treatment with everolimus, or the compounds of Chemical Formulas 1 to 4. As can be seen in FIG. 22, the phosphorylation of S6 in mutant mTOR-expressing cells was inhibited by everolimus or the compounds of Chemical Formulas 1 to 4. A distinct decrease in S6 phosphorylation was apparent at a concentration of 50nM or higher.

### Example 10-2. Change of S6K phosphorylation with various mTOR inhibitors

Cells expressing various mTOR mutations were treated in the same manner as in Example 9-1 with rapamycin, everolimus, or the compounds of Chemical Formulas 1 to 4, and monitored for S6K phosphorylation. The mTOR variants were R624H, Y1450D, C1483R, R1709H, Y1977K, S2215F, L2427P, and L2427Q.

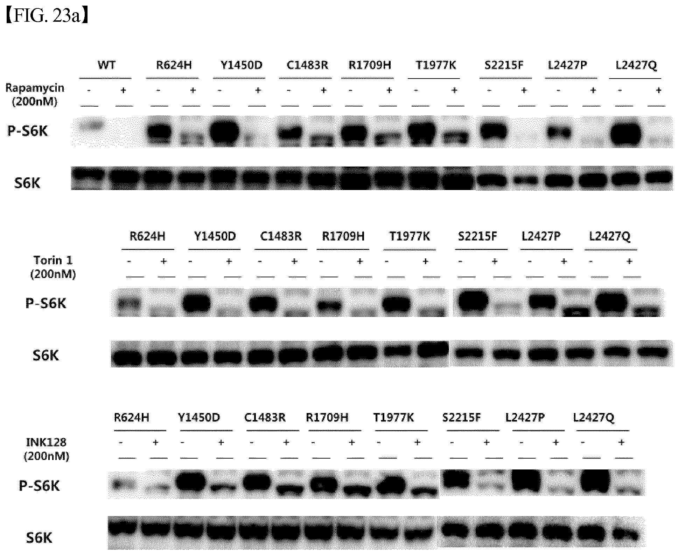

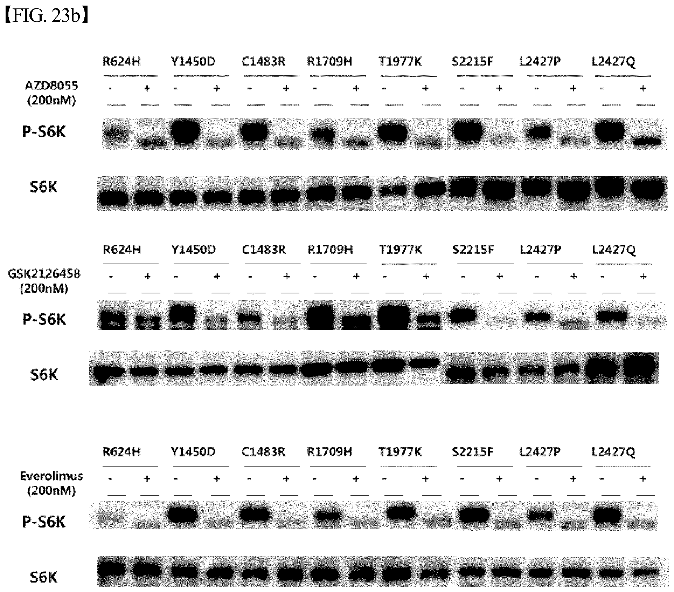

Briefly, the mutant mTOR-expressing cells were monitored for S6K phosphorylation level after treatment with everolimus or the compounds of Chemical Formulas 1 to 4. The results are depicted in FIGS. 23a and 23b. As shown, the phosphorylation of S6K in all the mutant mTOR-expressing cells was inhibited by everolimus or the compounds of Chemical Formulas 1 to 4.

### EXAMPLE 11: Monitoring of S6K Phosphorylation in TSC1 or TSC2 Mutant-Expressing Cells Treated with Drugs

HEK293T cells were transfected with TSC1 or TSC2 mutants in the same manner as in Example 8, serum-starved for 24 hrs with 01.% FBS in DMEM, and incubated at 37°C and 5% CO₂ for 1 hr with 1 mM MgCl₂ and CaCl₂ in PBS.

Thereafter, the cells were treated with rapamycin, everolimus, or the compounds of Chemical Formulas 1 to 4 (Torinl, INK128, AZD8055, GSK2126458): Torin was purchased from Tocris; INK128, AZD8055 and GSK2126458 were from Selleckchem; and everolimus was from LC laboratory. Subsequently, Western blotting was performed in the same manner as in Example 10.

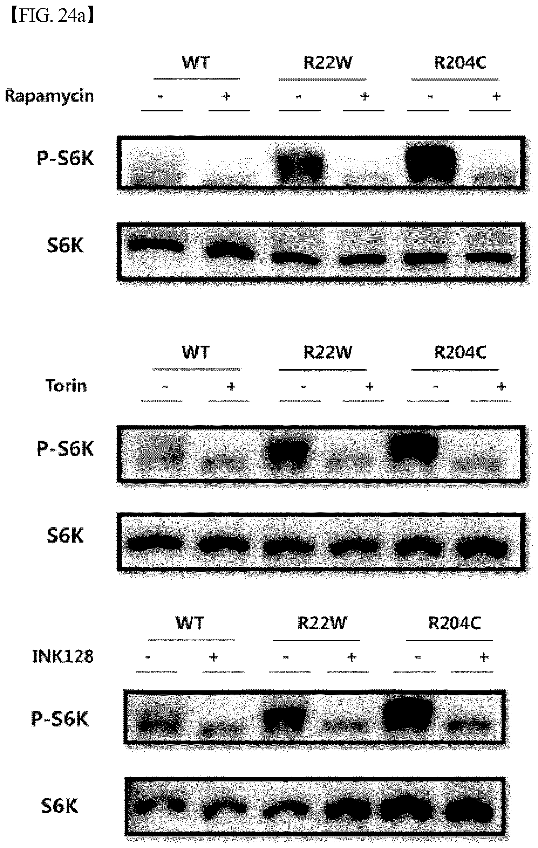

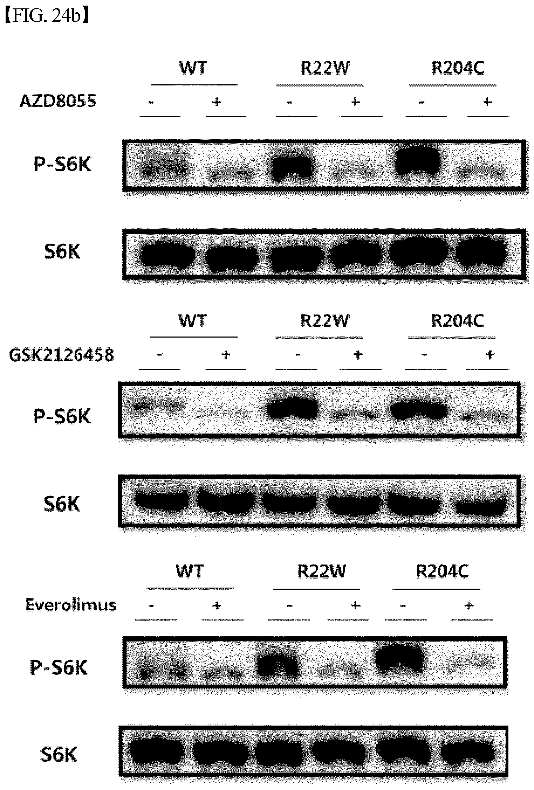

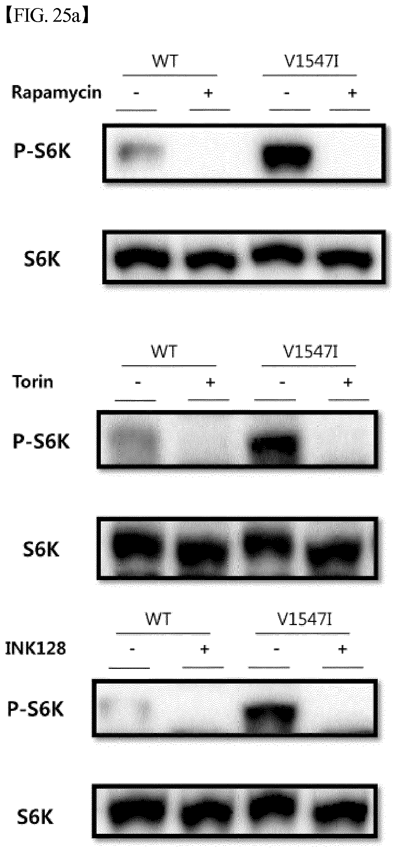

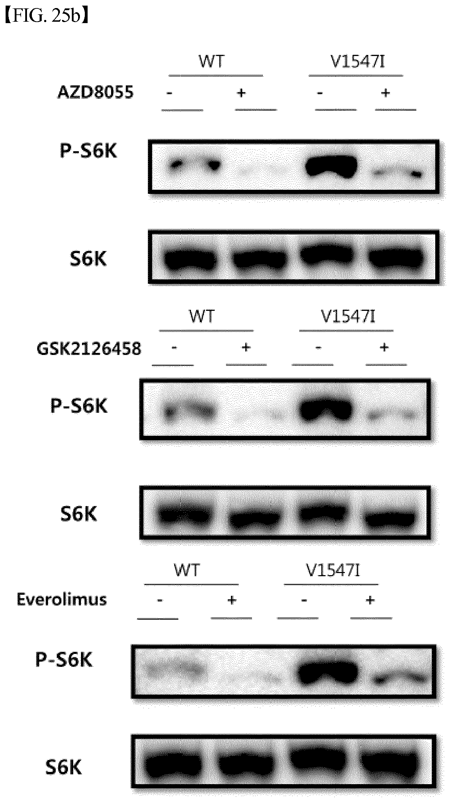

The TSC1 or TSC2 mutant-expressing cells were treated with rapamycin and monitored for S6K phosphorylation. The results are depicted in FIGS. 24a and 24b for the TSC1 mutant and in FIGS. 25a and 25b for the TSC2 mutant.

As is understood from the data of FIGS. 24a, 24b, 25a and 25b, S6K phosphorylation in TSC1 or TSC2 mutant-expressing cells was inhibited by rapamycin. Also, S6K phosphorylation was monitored in the TSC1 or TSC2-expressing cells following treatment with everolimus or the compounds of Chemical Formulas 1 to 4. As can be seen, everolimus or compounds of Chemical Formulas 1 to 4 was found to inhibit the phosphorylation of S6K in TSC1 or TSC2 mutant-expressing cells.

### EXAMPLE 12: Immunostaining of Brain Tissue Section of FCD Patient

To determine whether the affected brains of FCDII patients carrying mutations are associated with mTOR hyperactivation, immunostaining was performed for S6 phosphorylation and NeuN (a neuronal marker) in brain tissue sections obtained from FCD patients carrying the p.Leu2427Pro mutation.

Non-malformations of cortical development (non-MCD) brain specimens were collected in the operating room from the tumor-free margin of individual patients with glioblastoma as part of a planned resection, which was pathologically conformed as a normal brain without tumors. Surgical tissue blocks were fixed overnight in freshly prepared phosphate-buffered (PB) 4% paraformaldehyde, cryoprotected overnight in 20% buffered sucrose, and prepared into gelatin-embedded tissues blocks (7.5% gelatin in 10% sucrose/PB) before storage at -80°C. Cryostat-cut sections (10 um thick) were collected and placed on glass slides. FFPE slides were deparaffinized and rehydrated to remove paraffin. Then, a heat-induced retrieval process was performed on the deparaffinized FFPE slides using a citrate buffer to enhance the staining intensity of the antibodies. The slides were then blocked in PBS-GT (0.2% gelatin and 0.2% Triton X-100 in PBS) at room temperature for 1 hr before staining with the following antibodies: rabbit antibody to phosphorylated S6 ribosomal protein (Ser240/Ser244) (1:100 dilution; 5364, Cell signaling Technology) and mouse antibody to NeuN (1:100 dilution; MAB377, Millipore). Subsequently, samples were washed in PBS and stained with the following secondary antibodies: Alexa Fluor 555-conjugated goat antibody to mouse (1:200 dilution; A21422, Invitrogen) and Alexa Fluor 488-conjugated goat antibody to rabbit (1:200 dilution; A11008, Invitrogen).

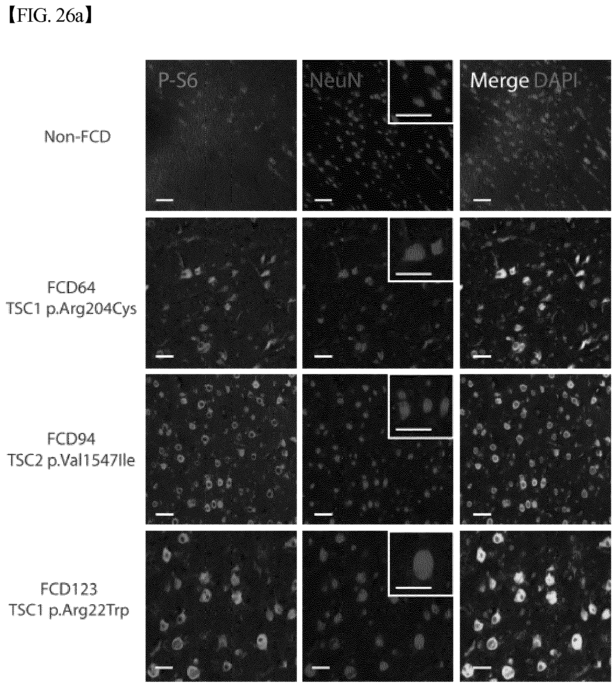

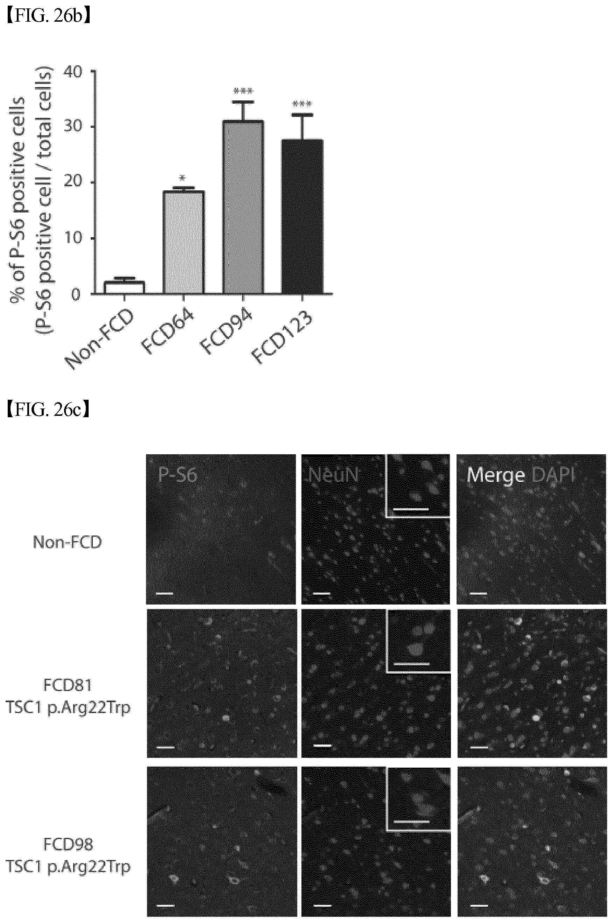

DAPI, included in a mounting solution (P36931, Life technology), was used for nuclear staining. Images were acquired using a Leica DMI3000 B inverted microscope. Cells positive for NeuN were counted using a 10x objective lens; 4-5 fields were acquired per subject within neuron-rich regions, and 100 or more cells were scored per region. The number of DAPI-positive cells represents total cell counts. Neuronal cell sizes were measured in NeuN-positive cells using the automated counting protocol of ImageJ software (http://rsbweb.nih.gov/ij/). The experimental results are given in FIGS. 26a to 26f.

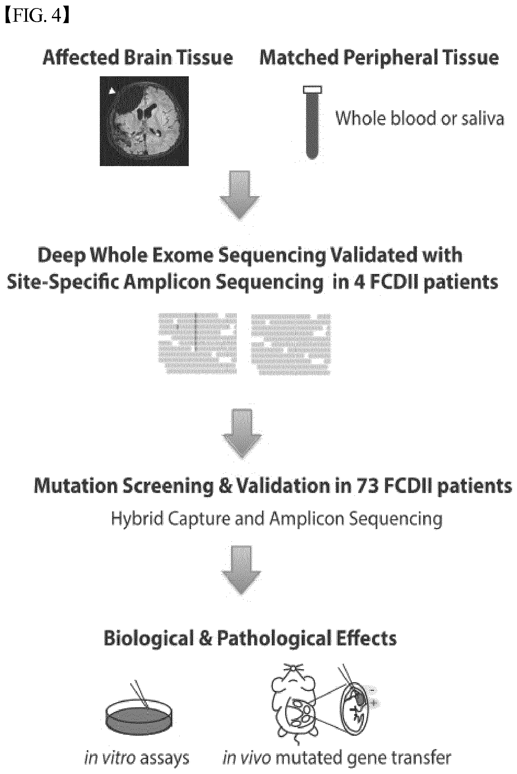

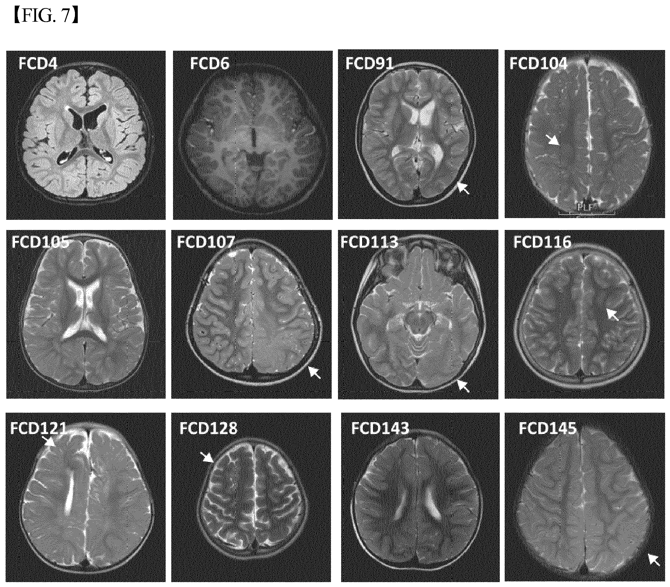

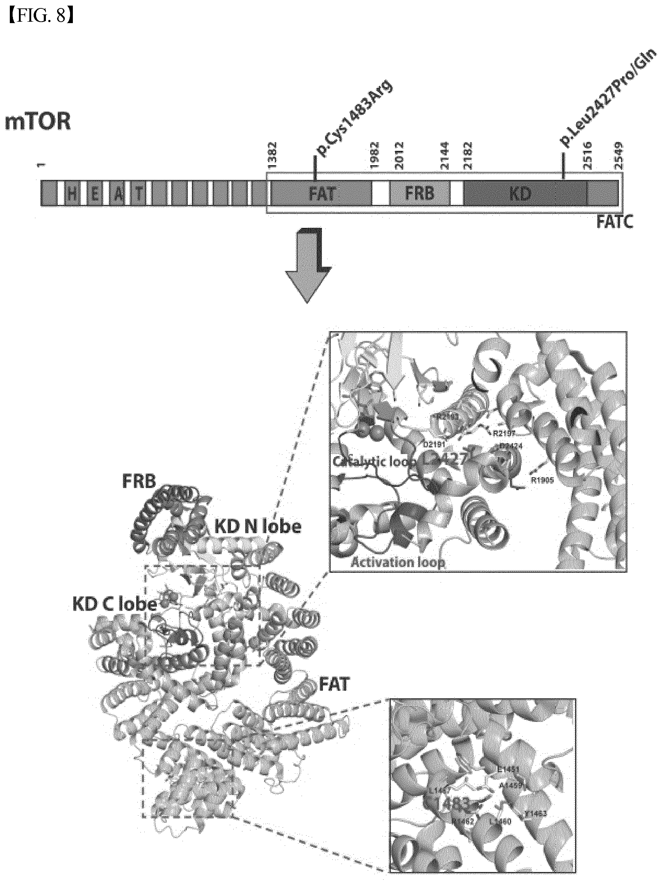

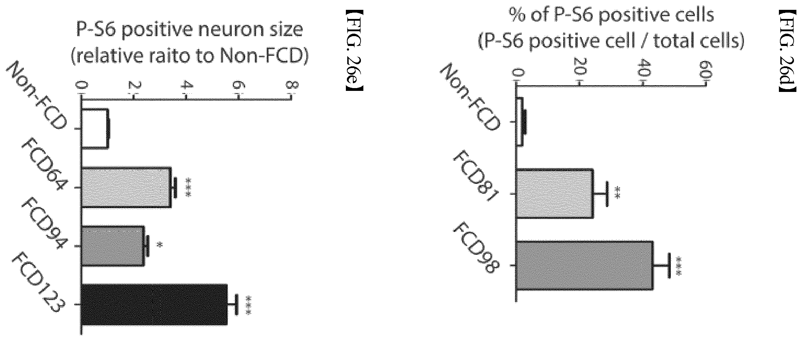

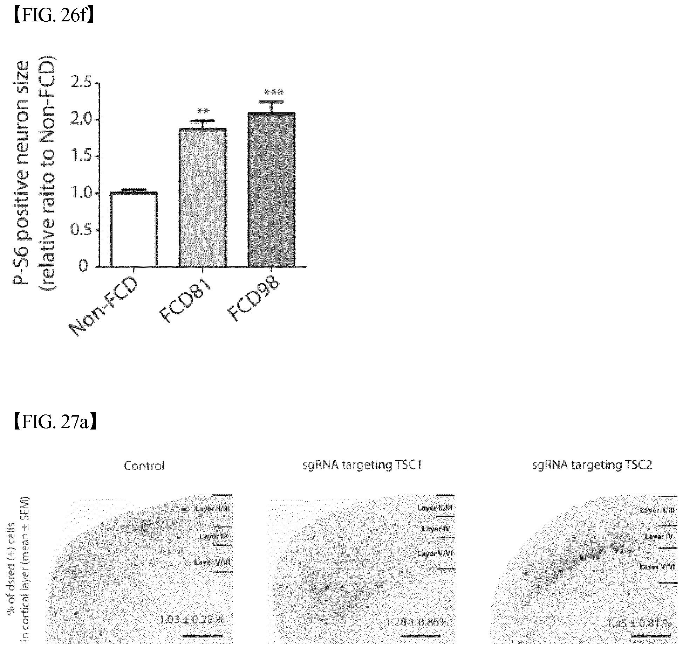

As seen in Figs. 2a to 26f, the results showed a marked increase in the number of neuronal cells positive for phosphorylated S6 in FCD patients # 64, 81, 94, 98, and 128 carrying TSC1 or TSC2 mutations, but not in non-FCD brains. In addition, the results revealed a robust increase in the number of neuronal cells that were positive for phosphorylated S6 in patients carrying *TSC1* and *TSC2* mutations compared with non-FCD brains, as shown in FIGS. 26b and 26d, and in the soma size of phosphorylated S6-posiive neurons in the pathological samples, as shown in FIGS. 26e and 26f.

### EXAMPLE 13: Construction of TSC1 or TSC2 Mouse Model

### 13-1: Construction of TSC1- or TSC2-targeting CRISPR/Cas9 vector

A commercially available pX330 plasmid (Addgene, #42230) was used as an initial template. Using a QuikChange site-directed mutagenesis kit (Stratagene, La Jolla, CA), the sgRNA (single guide ribonucleotide) cloning site was modified to change the restriction enzyme recognition site BbsI (GAAGAC) to BsaI (GGTCTC). Subsequently, sgRNAs, targeting respective TSC1 and TSC2 genes, was inserted, the sequences of which are as follows.
TSC1 : 5-TGCTGGACTCCTCCACACTG-3' (SEQ ID NO. 37)
TSC2 : 5'-AATCCCAGGTGTGCAGAAGG-3' (SEQ ID NO. 38)

To generate a plasmid carrying an mCherry fluorescent reporter (U6-sgRNA-Cas9-IRES-mCherry), IRES-mCheny was amplified PCR with the IRES3-mCherry-CL plasmid serving as a template. After PCR amplification, the IRES-mCherry was inserted between the Cas9 sequence and the NLS ofpx330.

### Example 13-2. Construction of mouse model

First, the TSC1- or TSC2-targeting U6-sgRNA-Cas9-IRES-mCherry plasmid, prepared in Example 19-1, was diluted at a ratio of 3:1 with pCAG-Dsred (Addgene #11151) to enhance red signals. Timed pregnant mice (E14) (Damul Science) were anesthetized with isoflurane (0.4 L/min of oxygen and isoflurane vaporizer gauge 3 during surgery operation). The uterine horns were exposed, and the lateral ventricle of each embryo was injected using pulled glass capillaries with 2 µg/ml of Fast Green (F7252, Sigma, USA) combined with 2-3 µg of the mixture of the two plasmids. Plasmids were electroporated on the head of the embryo by discharging 50 V with the ECM830 eletroporator (BTX-Harvard apparatus) in five electric pulses of 100ms at 900-ms intervals. After delivery, selection was made of the mouse pups that exhibited fluorescence, screened using a flashlight (Electron Microscopy Science, USA).

### 13-3: Assay for neuronal migration in TSC1 or TSC2 mouse model

Brains were harvested from adult mice (P>56) prepared in Example 13-2, fixed overnight in freshly prepared phosphate-buffered (PB) 4% paraformaldehyde, cryoprotected overnight in 30% buffered sucrose, and prepared into gelatin-embedded tissue blocks (7.5% gelatin in 10% sucrose/PB) before storage at -80°C.

Cryostat-cut sections (30 um thick) were collected and placed on glass slides. DAPI, included in a mounting solution (P36931, Life technology), was used for nuclear staining. Images were acquired using a Zeiss LSM780 confocal microscope. Fluorescence intensities, reflecting the distribution of electroporated cells within the cortex, were converted into gray values and measured from layer II/III to layer V/VI using ImageJ software (http://rsbweb.nih.gov/ij/).

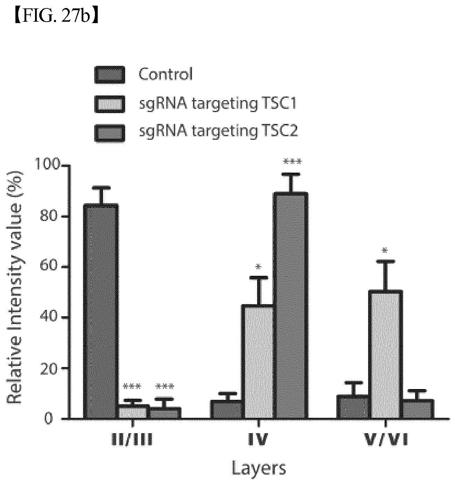

As can be seen in FIGS. 27a and 27b, dsRed-positive neurons in brain tissue sections of the TSC mouse models were decreased in the cortical layer II/III and increased in the layer II and layer V/VI, indicating that the radial migration of cortical neurons was prevented.

### 13-4: Video-Electroencephalography monitoring

After weaning (> 3 weeks), the mice were observed for seizures through video monitoring. Thereafter, electrodes for recoding electroencephalograms were surgically implanted. A total of five electrodes were located in the epidural layer: based on the bregma, two electrodes on the frontal lobes (AP+2.8mm, ML±1.5mm), two electrodes on the temporal lobes (AP-2.4mm, ML±2.4mm), and one electrode on the cerebellum. After 4 days of recovery from surgery, EGG signals were recorded for 6 hrs per day between 6 p.m. and 2 a.m. over 2-5 days. Signals were amplified using an RHD2000 amplifier and board (Intan Technologies, USA) and analyzed using MATLAB EEGLAB (http://sccn.ucsd.edu/eeglab).

The mice whose brains exhibited local TSC1- or TSC2-knockout resulting from use of the CRISPR/Cas9 plasmid displayed spontaneous seizures with epileptic discharges, including high-voltage fast activity, high-voltage spikes and waves, and low-voltage fast activity. The mice in which spontaneous seizures were induced were observed to exhibit systemic tonic-clonic seizures consisting of a tonic phase, a clonic phase, and a postictal phase, similar to those found in FCDII patients. Further, brain waves are characterized by synchronized multi-waves of low-voltage, fast activity in the tonic phase, high-voltage standing waves in the clonic phase, and synchronized attenuated amplitudes in the postictal phase. The seizure frequency was about 10 events per day.

### 13-5: Soma size of neurons in TSC1 or TSC2 mouse model

After EGG monitoring, the brains of the mice were excised by perfusion fixation using a phosphate-buffered (PB) 4% paraformaldehyde solution with the aid of a Masterflex compact peristaltic pump (Cole-Parmer international, USA). The brains were fixed in a freshly prepared phosphate-buffered (PB) 4% paraformaldehyde solution, cryoprotected overnight in 30% buffered sucrose, and prepared into gelatin-embedded tissue blocks (7.5% gelatin in 10% sucrose/PB) before storage at -80°C. Cryostat-cut sections (30 um thick) were collected, placed on glass slides, and blocked in PBS-GT (0.2% gelatin and 0.2% Triton X-100 in PBS) at room temperature for 1 hr before staining with the following antibodies: mouse antibody to NeuN (1:500 dilution; MAB377, Millipore). Subsequently, samples were washed in PBS and stained with the following secondary antibody: Alexa Fluor 488-conjugated goat antibody to mouse (1:200 dilution; A11008, Invitrogen). DAPI included in a mounting solution (P36931, Life technology) was used for nuclear staining. Images were acquired using a Zeiss LSM780 confocal microscope. Neuronal cell sizes were measured using ImageJ software (http://rsbweb.nih.gov/ij/).

Neurons were found to significantly increase in soma size for the mice with the local TSC1- or TSC2-knockout by use of the CRISPR/Cas9 plasmid, compared to normal neurons, but remained unchanged in size for the mice into which the plasmid was electroporated without sgRNA, which was consistent with the dysmorphic neurons of patients with Malformations of Cortical Developments.

### EXAMPLE 14: Effect of Drug on Spontaneous Seizure in TSC2 Mouse Model

The animal models exhibiting spontaneous seizures were monitored after administration with rapamycin. Briefly, rapamycin (LC Labs, USA) was dissolved in 100% ethanol to give a 20mg/ml stock solution, and stored at -20°C. Immediately before injection, the stock solution was diluted in 5% polyethyleneglycol 400 and 5% Tween80 to final concentrations of 1 mg/ml rapamycin and 4% ethanol. Mice were injected with 1 to 10 mg/kg rapamycin for 2 weeks (10 mg/kg/d intraperitoneal injection).

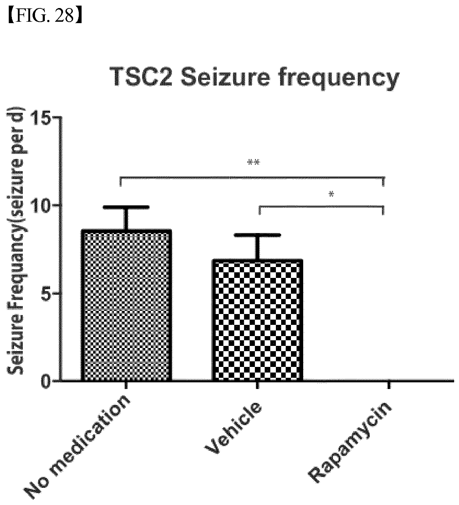

Rapamycin, as shown in FIG. 28, almost completely freed the animal models from spontaneous seizures.
## Claims

1. A mTOR inhibitor compound for use in prevention or treatment of Focal Cortical Dysplasia (FCD) type II or an epilepsy associated with FCD type II, wherein the FCD type II is caused by brain somatic mutation of mTOR, TSC1, TSC2, AKT3, or PIK3CA.

2. The mTOR inhibitor compound for use according to claim 1, wherein the brain somatic mutation is an amino acid substitution of mTOR, TSC1, TSC2, AKT3, or PIK3CA protein, or a nucleotide substitution of gene encoding mTOR, TSC1, TSC2, AKT3, or PIK3CA protein causing the amino acid substitution.

3. The mTOR inhibitor compound for use according to claims 1 or 2, wherein the brain somatic mutation induces the mTOR hyperactivation in brain.

4. The mTOR inhibitor compound for use according to any of claims 1 to 3, wherein the mTOR inhibitor is selected from the group consisting of Rapamycin, Everolimus, the compounds represented by chemical formulae 1 to 4, [5-[2,4-bis[(3S)-3-methylmorpholin-4-yl]pyrido[2,3-d]pyrimidin-7-yl]-2-methoxyphenyl]methanol (AZD8055), 3-[2,4-bis[(3S)-3-methylmorpholin-4-yl]pyrido[2,3-d]pyrimidin-7-yl]-N-methylbenzamide (AZD2014), 2-methyl-2-[4-(3-methyl-2-oxo-8-quinolin-3-ylimidazo[4,5-c]quinolin-1-yl)phenyl]propanenitrile (BEZ235), (Z)-but-2-enedioic acid;8-(6-methoxypyridin-3-yl)-3-methyl-1-[4-piperazin-1-yl-3-(trifluoromethyl)phenyl]imidazo[4,5-c]quinolin-2-one (BGT226), Everolimus, 5-ethyl-3-[2-methyl-6-(1H-1,2,4-triazol-5-yl)pyridin-3-yl]-7,8-dihydropyrazino[2,3-b]pyrazin-6-one (CC-115), 3-[6-(2-hydroxypropan-2-yl)pyridin-3-yl]-5-(4-methoxycyclohexyl)-7,8-dihydropyrazino[2,3-b]pyrazin-6-one (CC-223), 8-[5-(2-hydroxypropan-2-yl)pyridin-3-yl]-1-[(2S)-2-methoxypropyl]-3-methylimidazo[4,5-c]quinolin-2-one (LY3023414), the compound represented by chemical formula 5 (P7170), 1-{(2R)-4-[2-(2-Aminopyrimidin-5-yl)-6-(morpholin-4-yl)-9-(2,2,2-trifluoroethyl)-9H-purin-8-yl]-2-methylpiperazin-1-yl}ethan-1-one (DS-7423), (4-[(5Z)-4-amino-5-(7-methoxyindol-2-ylidene)-1H-imidazo[5,1-f][1,2,4]triazin-7-yl]cyclohexane-1-carboxylic acid) (OSI-027), (2,4-difluoro-N-[2-methoxy-5-(4-pyridazin-4-ylquinolin-6-yl)pyridin-3-yl]benzenesulfonamide) (GSK2126458), 2-amino-8-[4-(2-hydroxyethoxy)cyclohexyl]-6-(6-methoxypyridin-3-yl)-4-methylpyrido[2,3-d]pyrimidin-7-one (PF-04691502), 1-[4-[4-(dimethylamino)piperidine-1-carbonyl]phenyl]-3-[4-(4,6-dimorpholin-4-yl-1,3,5-triazin-2-yl)phenyl]urea (PF-05212384), Temsirolimus, 5-(4-amino-1-propan-2-ylpyrazolo[3,4-d]pyrimidin-3-yl)-1,3-benzoxazol-2-amine (INK128), [6-(2-amino-1,3-benzoxazol-5-yl)imidazo[1,2-a]pyridin-3-yl]-morpholin-4-ylmethanone (MLN1117), Ridaforolimus, Metformin, N-[4-[[3-(3,5-dimethoxyanilino)quinoxalin-2-yl]sulfamoyl]phenyl]-3-methoxy-4-methylbenzamide (XL765), 2-amino-8-ethyl-4-methyl-6-(1H-pyrazol-5-yl)pyrido[2,3-d]pyrimidin-7-one (SAR245409), (3S)-4-[[(1S)-1-carboxy-2-hydroxyethyl]amino]-3-[[2-[[(2S)-5-(diaminomethylideneamino)-2-[[4-oxo-4-[[4-(4-oxo-8-phenylchromen-2-yl)morpholin-4-ium-4-yl]methoxy]butanoyl]amino]pentanoyl]amino]acetyl]amino]-4-oxobutanoate (SF1126), 5-(8-methyl-2-morpholin-4-yl-9-propan-2-ylpurin-6-yl)pyrimidin-2-amine (VS5584) and (2S)-1-[4-[[2-(2-aminopyrimidin-5-yl)-7-methyl-4-morpholin-4-ylthieno[3,2-d]pyrimidin-6-yl]methyl]piperazin-1-yl]-2-hydroxypropan-1-one (GDC0980) and pharmaceutically-acceptable salts thereof;

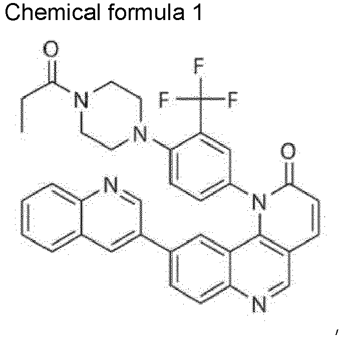

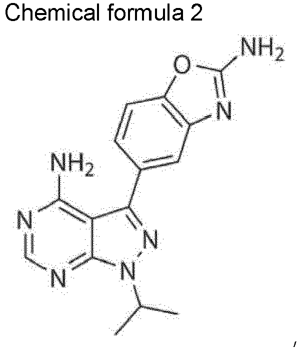

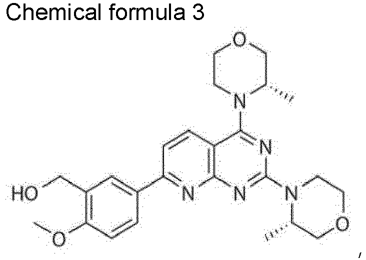

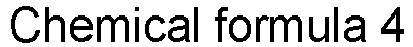

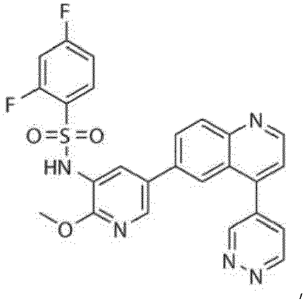

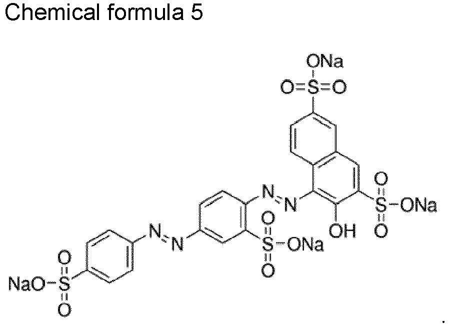

5. The mTOR inhibitor compound for use according to claim 1, wherein the mTOR inhibitor is selected from the group consisting of Rapamycin or its salts, Everolimus and its salts, the compounds represented by chemical formulae 1 to 4 and their salts:

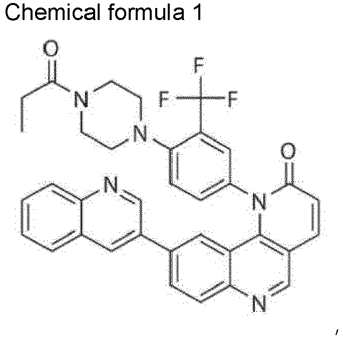

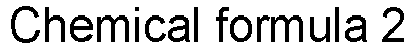

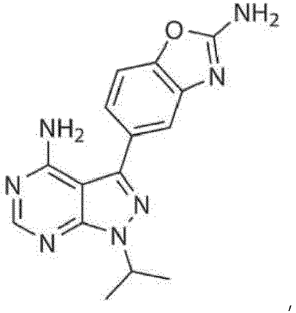

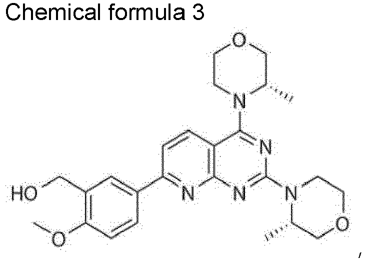

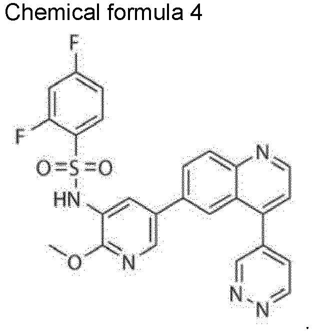

6. The mTOR inhibitor compound for use according to claim 1, wherein the brain somatic mutation comprises at least one selected from the group consisting of amino acid substitutions arginine (R) at position 206 to cysteine(C), R at position 624 to H, Y at position 1450 to D, C at position 1483 to R, R at position 1709 to H, T at position 1977 to K, R at position 2193 to C, S at position 2215 to F, L at position 2427 to P, and L at position 2427 to Q in SEQ ID NO: 2,
amino acid substitutions arginine (R) at position 22 to tryptophan(W), R at position 204 to C, and R at position 811 to L in SEQ ID NO: 4,
amino acid substitution valine (V) at position 1547 to isoleucine (I) in SEQ ID NO: 6,
amino acid substitution arginine (R) at position 247 to histidine (H) in SEQ ID NO: 8, and amino acid substitution aspartic acid (D) at position 1018 to asparagine (N) in SEQ ID NO: 10.

7. The mTOR inhibitor compound for use according to claim 1, wherein the brain somatic mutation includes at least one selected from the group consisting of nucleotide mutations Cytosine (C) at position 616 to Thymine (T), Guanine(G) at position 1871 to Adenine(A), Thymine (T) at position 4348 to Guanine(G), Thymine(T) at position 4447 to Cytosine(C), Guanine(G) at position 5126 to Adenine(A), Cytosine(C) at position 5930 to Adenine(A), Cytosine(C) at position6577 to Thymine(T), Cytosine(C) at position 6644 to Thymine(T), Thymine(T) at position 7280 to Cytosine(C), Thymine(T) at position 7280 to Adenine(A) in SEQ ID NO: 1,
nucleotide mutations Cytosine(C) at position 64 to Thymine(T), Cytosine(C) at position 610 to Thymine(T), and Guanine(G) at position 2432 to Thymine(T) in SEQ ID NO: 3,
nucleotide mutation Guanine(G) at position 4639 to Adenine(A) in SEQ ID NO: 5,
nucleotide mutation Guanine(G) at position 740 to Adenine(A) in SEQ ID NO: 7, and nucleotide mutation Guanine(G) at position 3052 to Adenine(A) in SEQ ID NO: 9.

8. The mTOR inhibitor compound for use according to claim 1, further comprising a compound selected from the group consisting of a pharmaceutically acceptable diluent, excipient, stabilizing agent, surfactant, gelling agent, pH adjusting agent, anti-oxidant and preservative.

## Patentansprüche

1. mTOR-Inhibitorverbindung zur Verwendung bei der Prävention oder Behandlung von fokaler kortikaler Dysplasie (FCD) Typ II oder einer Epilepsie, die mit FCD Typ II assoziiert ist, wobei die FCD Typ II durch eine somatische Mutation von mTOR, TSC1, TSC2, AKT3 oder PIK3CA im Gehirn verursacht wird.

2. mTOR-Inhibitorverbindung zur Verwendung gemäß Anspruch 1, wobei die somatische Mutation im Gehirn eine Aminosäuresubstitution von mTOR-, TSC1-, TSC2-, AKT3- oder PIK3CA-Protein oder eine die Aminosäuresubstitution verursachende Nukleotidsubstitution eines Gens, das für mTOR-, TSC1-, TSC2-, AKT3- oder PIK3CA-Protein codiert, ist.

3. mTOR-Inhibitorverbindung zur Verwendung gemäß Anspruch 1 oder 2, wobei die somatische Mutation im Gehirn die mTOR-Hyperaktivierung im Gehirn induziert.

4. mTOR-Inhibitorverbindung zur Verwendung gemäß einem der Ansprüche 1 bis 3, wobei der mTOR-Inhibitor ausgewählt ist aus der Gruppe bestehend aus Rapamycin, Everolimus, den durch die chemischen Formeln 1 bis 4 dargestellten Verbindungen, [5-[2,4-Bis[(3S)-3-methylmorpholin-4-yl]pyrido[2,3-d]pyrimidin-7-yl]-2-methoxyphenyl]methanol (AZD8055), 3-[2,4-Bis[(3S)-3-methylmorpholin-4-yl]pyrido[2,3-d]pyrimidin-7-yl]-N-methylbenzamid (AZD2014), 2-Methyl-2-[4-(3-methyl-2-oxo-8-chinolin-3-ylimidazo[4,5-c]chinolin-1-yl)phenyl]propannitril (BEZ235), (Z)-But-2-endicarbonsäure, 8-(6-Methoxypyridin-3-yl)-3-methyl-1-[4-piperazin-1-yl-3-(trifluormethyl)phenyl]imidazo[4,5-c]chinolin-2-on (BGT226), Everolimus, 5-Ethyl-3-[2-methyl-6-(1H-1,2,4-triazol-5-yl)pyridin-3-yl]-7,8-dihydropyrazino[2,3-b]pyrazin-6-on (CC-115), 3-[6-(2-Hydroxypropan-2-yl)pyridin-3-yl]-5-(4-methoxycyclohexyl)-7,8-dihydropyrazino[2,3-b]pyrazin-6-on (CC-223), 8-[5-(2-Hydroxypropan-2-yl)pyridin-3-yl]-1-[(2S)-2-methoxypropyl]-3-methylimidazo[4,5-c]chinolin-2-on (LY3023414), der durch die chemische Formel 5 dargestellten Verbindung (P7170), 1-{(2R)-4-[2-(2-Aminopyrimidin-5-yl)-6-(morpholin-4-yl)-9-(2,2,2-trifluorethyl)-9H-purin-8-yl]-2-methylpiperazin-1-yl}ethan-1-on (DS-7423), (4-[(5Z)-4-Amino-5-(7-methoxyindol-2-yliden)-1H-imidazo[5,1-f][1,2,4]triazin-7-yl]cyclohexan-1-Carbonsäure) (OSI-027), (2,4-Difluor-N-[2-methoxy-5-(4-pyridazin-4-ylchinolin-6-yl)pyridin-3-yl]benzensulfonamid) (GSK2126458), 2-Amino-8-[4-(2-hydroxyethoxy)cyclohexyl]-6-(6-methoxypyridin-3-yl)-4-methylpyrido[2,3-d] pyrimidin-7-on (PF-04691502), 1-[4-[4-(Dimethylamino)piperidin-1-carbonyl]phenyl]-3-[4-(4,6-dimorpholin-4-yl-1,3,5-triazin-2-yl)phenyl]harnstoff (PF-05212384), Temsirolimus, 5-(4-Amino-1-propan-2-ylpyrazolo[3,4-d]pyrimidin-3-yl)-1,3-benzoxazol-2-amin (INK128), [6-(2-Amino-1,3-benzoxazol-5-yl)imidazo[1,2-a]pyridin-3-yl]-morpholin-4-ylmethanon (MLN1117), Ridaforolimus, Metformin, N-[4-[[3-(3,5-dimethoxyanilin)chinoxalin-2-yl]sulfamoyl]phenyl]-3-methoxy-4-methylbenzamid (XL765), 2-Amino-8-ethyl-4-methyl-6-(1H-pyrazol-5-yl)pyrido[2,3-d]pyrimidin-7-on (SAR245409), (3S)-4-[[(1S)-1-carboxy-2-hydroxyethyl]amino]-3-[[2-[[(2S)-5-(diaminomethylidenamino)-2-[[4-oxo-4-[[4-(4-oxo-8-phenylchromen-2-yl)morpholin-4-ium-4-yl]methoxy]butanoyl]amino]pentanoyl]amino]acetyl]amino]-4-oxobutanoat (SF1126), 5-(8-Methyl-2-morpholin-4-yl-9-propan-2-ylpurin-6-yl)pyrimidin-2-amin (VS5584) und (2S)-1-[4-[[2-(2-Aminopyrimidin-5-yl)-7-methyl-4-morpholin-4-morpholin-4-ylthieno[3,2-d]pyrimidin-6-yl]methyl]piperazin-1-yl]-2-hydroxypropan-1-on (GDC0980) und pharmazeutisch annehmbaren Salzen davon;

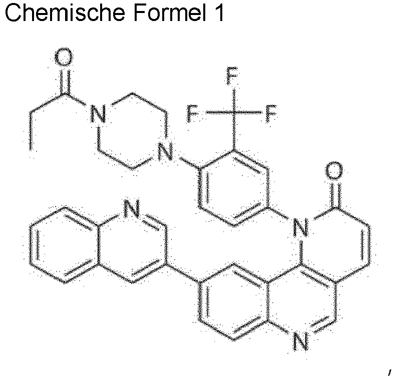

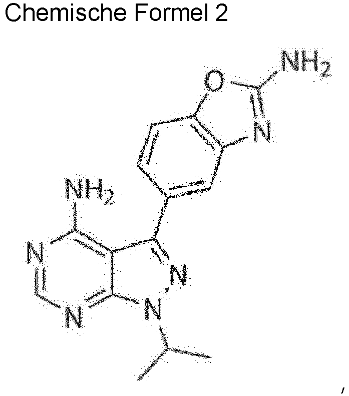

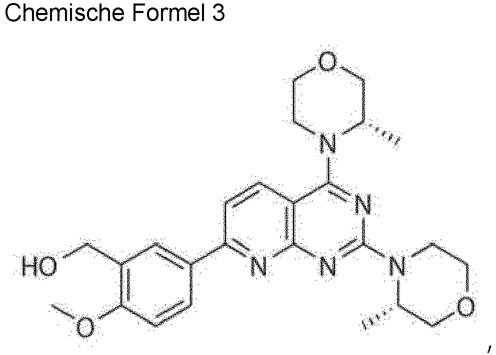

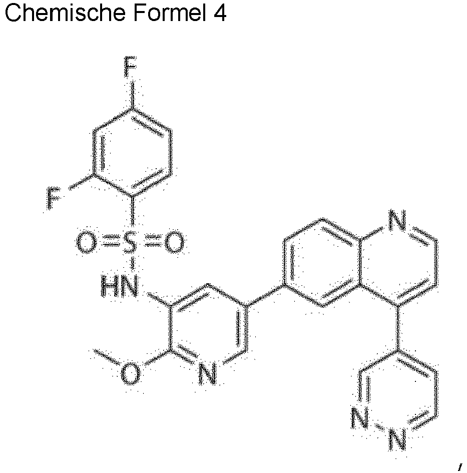

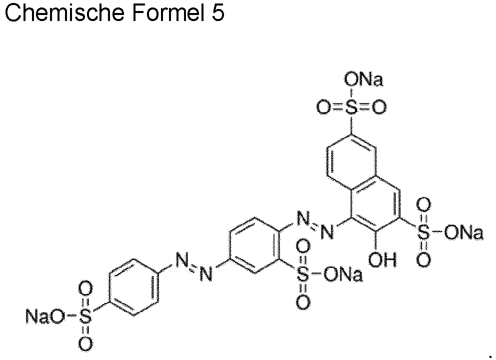

5. mTOR-Inhibitorverbindung zur Verwendung gemäß Anspruch 1, wobei der mTOR-Inhibitor aus der Gruppe ausgewählt ist, die aus Rapamycin oder seinen Salzen, Everolimus und seinen Salzen, den durch die chemischen Formeln 1 bis 4 dargestellten Verbindungen und ihren Salzen besteht:

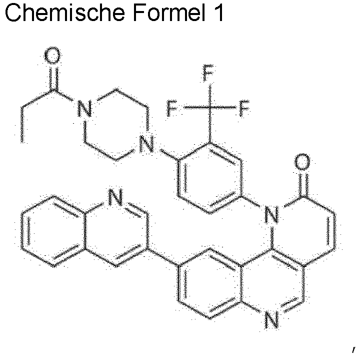

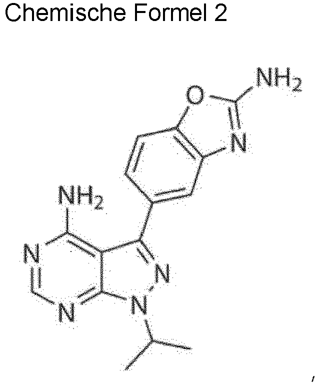

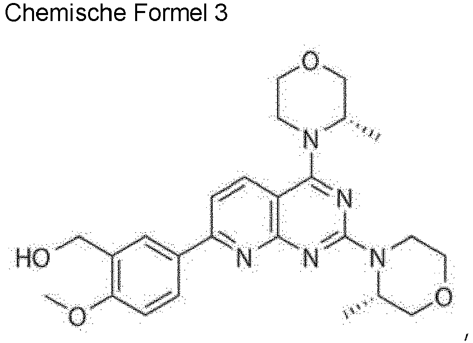

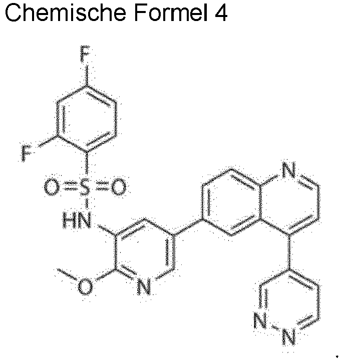

6. mTOR-Inhibitorverbindung zur Verwendung gemäß Anspruch 1, wobei die somatische Mutation im Gehirn mindestens eine umfasst, die ausgewählt ist aus der Gruppe bestehend aus Aminosäuresubstitutionen Arginin (R) an Position 206 zu Cystein(C), R an Position 624 zu H, Y an Position 1450 zu D, C an Position 1483 zu R, R an Position 1709 zu H, T an Position 1977 zu K, R an Position 2193 zu C, S an Position 2215 zu F, L an Position 2427 zu P und L an Position 2427 zu Q in SEQ ID NO: 2,
Aminosäuresubstitutionen Arginin (R) an Position 22 zu Tryptophan(W), R an Position 204 zu C und R an Position 811 zu L in SEQ ID NO: 4,
Aminosäuresubstitution Valin (V) an Position 1547 zu Isoleucin (I) in SEQ ID NO: 6,
Aminosäuresubstitution Arginin (R) an Position 247 zu Histidin (H) in SEQ ID NO: 8 und
Aminosäure-Substitution Asparaginsäure (D) an Position 1018 zu Asparagin (N) in SEQ ID NO: 10.

7. mTOR-Inhibitorverbindung zur Verwendung gemäß Anspruch 1, wobei die somatische Mutation im Gehirn mindestens eine umfasst, die ausgewählt ist aus der Gruppe bestehend aus Nukleotidmutationen Cytosin (C) an Position 616 zu Thymin (T), Guanin (G) an Position 1871 zu Adenin (A), Thymin (T) an Position 4348 zu Guanin (G), Thymin (T) an Position 4447 zu Cytosin (C), Guanin (G) an Position 5126 zu Adenin (A), Cytosin (C) an Position 5930 zu Adenin (A), Cytosin (C) an Position 6577 zu Thymin (T), Cytosin (C) an Position 6644 zu Thymin (T), Thymin (T) an Position 7280 zu Cytosin (C), Thymin (T) an Position 7280 zu Adenin (A) in SEQ ID NO: 1,
Nukleotidmutationen Cytosin (C) an Position 64 zu Thymin (T), Cytosin (C) an Position 610 zu Thymin (T) und Guanin (G) an Position 2432 zu Thymin (T) in SEQ ID NO: 3,
Nukleotid-Mutation Guanin (G) an Position 4639 zu Adenin (A) in SEQ ID NO: 5,
Nukleotid-Mutation Guanin (G) an Position 740 zu Adenin (A) in SEQ ID NO: 7 und
Nukleotid-Mutation Guanin (G) an Position 3052 zu Adenin (A) in SEQ ID NO: 9.

8. mTOR-Inhibitorverbindung zur Verwendung gemäß Anspruch 1, weiterhin eine Verbindung umfassend, welche ausgewählt ist aus der Gruppe bestehend aus einem pharmazeutisch annehmbaren Verdünnungsmittel, Exzipienten, Stabilisierungsmittel, Tensid, Geliermittel, pH-Einstellmittel, Antioxidationsmittel und Konservierungsmittel.

## Revendications

1. Composé inhibiteur de mTOR pour l'utilisation dans la prévention ou le traitement de la dysplasie corticale focale (FCD) de type II ou une épilepsie associée à la FCD de type II, la FCD de type II étant causée par la mutation somatique spécifique au cerveau de mTOR, TSC1, TSC2, AKT3, ou PIK3CA.

2. Composé inhibiteur de mTOR pour l'utilisation selon la revendication 1, la mutation somatique spécifique au cerveau étant une substitution d'acide aminé de la protéine mTOR, TSC1, TSC2, AKT3, ou PIK3CA, ou une substitution de nucléotide du gène codant pour la protéine mTOR, TSC1, TSC2, AKT3, ou PIK3CA causant la substitution d'acide aminé.

3. Composé inhibiteur de mTOR pour l'utilisation selon les revendications 1 ou 2, la mutation somatique spécifique au cerveau induisant l'hyperactivation de mTOR dans le cerveau.

4. Composé inhibiteur de mTOR pour l'utilisation selon l'une quelconque des revendications 1 à 3, l'inhibiteur de mTOR étant sélectionné dans le groupe constitué de la rapamycine, de l'éverolimus, des composés représentés par les formules chimiques 1 à 4, du [5-[2,4-bis[(3S)-3-méthylmorpholin-4-yl]pyrido[2,3-d]pyrimidin-7-yl]-2-méthoxyphényl]méthanol (AZD8055), 3-[2,4-bis[(3S)-3-méthylmorpholin-4-yl]pyrido[2,3-d]pyrimidin-7-yl]-N-méthylbenzamide (AZD2014), 2-méthyl-2-[4-(3-méthyl-2-oxo-8-quinoléin-3-ylimidazo[4,5-c]quinoléin-1-yl)phényl]propanenitrile (BEZ235), de l'acide (Z)-but-2-ènedioïque ; de la 8-(6-méthoxypyridin-3-yl)-3-méthyl-1-[4-pipérazin-1-yl-3-(trifluorométhyl)phényl]imidazo[4,5-c]quinoléin-2-one (BGT226), de l'éverolimus, de la 5-éthyl-3-[2-méthyl-6-(1H-1,2,4-triazol-5-yl)pyridin-3-yl]-7,8-dihydropyrazino[2,3-b]pyrazin-6-one (CC-115), 3-[6-(2-hydroxypropan-2-yl)pyridin-3-yl]-5-(4-méthoxycyclohexyl)-7,8-dihydropyrazino[2,3-b]pyrazin-6-one (CC-223), 8-[5-(2-hydroxypropan-2-yl)pyridin-3-yl]-1-[(2S)-2-méthoxypropyl]-3-méthylimidazo[4,5-c]quinoléin-2-one (LY3023414), du composé représenté par la formule chimique 5 (P7170), de la 1-{(2R)-4-[2-(2-aminopyrimidin-5-yl)-6-(morpholin-4-yl)-9-(2,2,2-trifluoroéthyl)-9H-purin-8-yl]-2-méthylpipérazin-1-yl}éthan-1-one (DS-7423), de l'acide (4-[(5Z)-4-amino-5-(7-méthoxyindol-2-ylidène)-1H-imidazo[5,1-f][1,2,4]triazin-7-yl]cyclohexane-1-carboxylique) (OSI-027), du (2,4-difluoro-N-[2-méthoxy-5-(4-pyridazin-4-ylquinoléin-6-yl)pyridin-3-yl]benzènesulfonamide) (GSK2126458), de la 2-amino-8-[4-(2-hydroxyéthoxy)cyclohexyl]-6-(6-méthoxypyridin-3-yl)-4-méthylpyrido[2,3-d]pyrimidin-7-one (PF-04691502), 1-[4-[4-(diméthylamino)pipéridine-1-carbonyl]phényl]-3-[4-(4,6-dimorpholin-4-yl-1,3,5-triazin-2-yl)phényl]urée (PF-05212384), du temsirolimus, de la 5-(4-amino-1-propan-2-ylpyrazolo[3,4-d]pyrimidin-3-yl)-1,3-benzoxazol-2-amine (INK128), [6-(2-amino-1,3-benzoxazol-5-yl)imidazo[1,2-a]pyridin-3-yl]-morpholin-4-ylméthanone (MLN1117), du ridaforolimus, de la metformine, du N-[4-[[3-(3,5-diméthoxyanilino)quinoxalin-2-yl]sulfamoyl]phényl]-3-méthoxy-4-méthylbenzamide (XL765), de la 2-amino-8-éthyl-4-méthyl-6-(1H-pyrazol-5-yl)pyrido[2,3-d]pyrimidin-7-one (SAR245409), du (3S)-4-[[(1S)-1-carboxy-2-hydroxyéthyl]amino]-3-[[2-[[(2S)-5-(diaminométhylidèneamino)-2-[[4-oxo-4-[[4-(4-oxo-8-phénylchromèn-2-yl)morpholin-4-ium-4-yl]méthoxy]butanoyl]amino]pentanoyl]amino]acétyl]amino]-4-oxobutanoate (SF1126), de la 5-(8-méthyl-2-morpholin-4-yl-9-propan-2-ylpurin-6-yl)pyrimidin-2-amine (VS5584) et de la (2S)-1-[4-[[2-(2-aminopyrimidin-5-yl)-7-méthyl-4-morpholin-4-ylthiéno[3,2-d]pyrimidin-6-yl]méthyl]pipérazin-1-yl]-2-hydroxypropan-1-one (GDC0980) et de leurs sels pharmaceutiquement acceptables ;

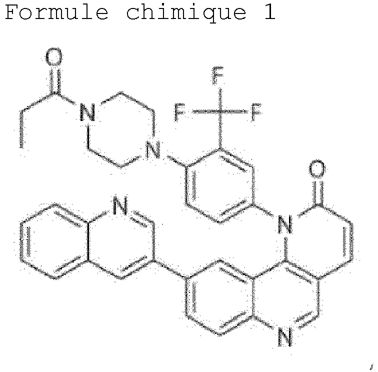

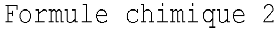

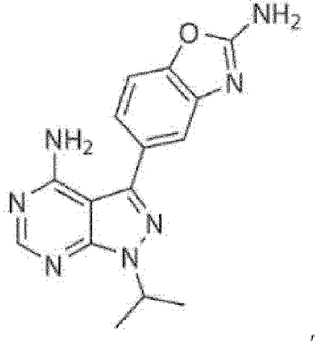

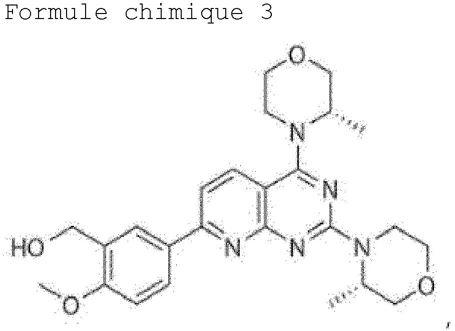

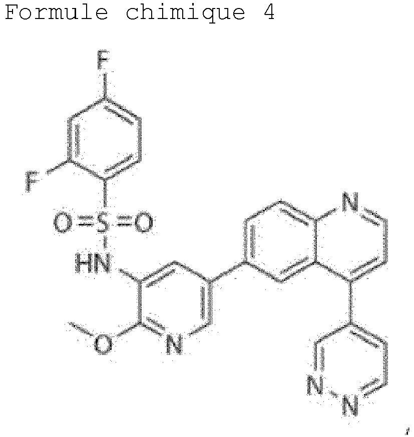

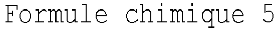

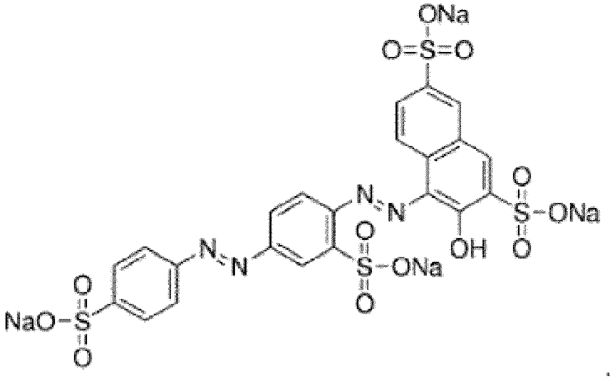

5. Composé inhibiteur de mTOR pour l'utilisation selon la revendication 1, l'inhibiteur de mTOR étant sélectionné dans le groupe constitué de la rapamycine ou ses sels, de l'éverolimus et ses sels, des composés représentés par les formules chimiques 1 à 4 et de leurs sels :

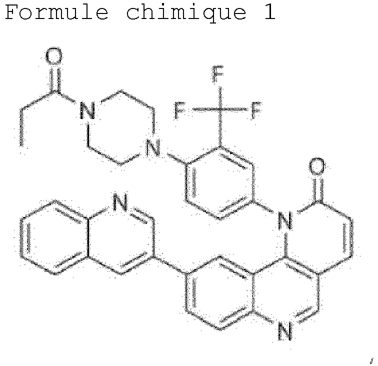

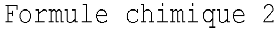

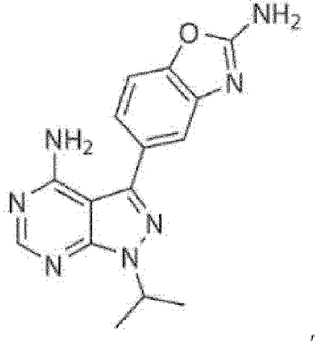

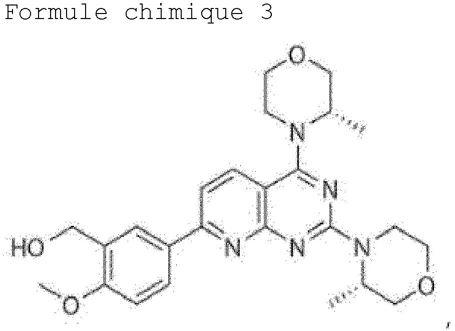

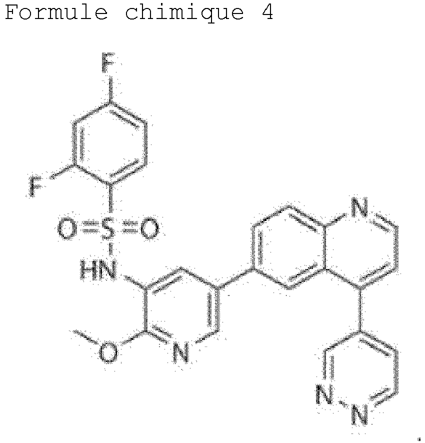

6. Composé inhibiteur de mTOR pour l'utilisation selon la revendication 1, la mutation somatique spécifique au cerveau comprenant au moins l'une sélectionnée dans le groupe constitué des substitutions d'acide aminé arginine (R) à la position 206 par la cystéine (C), R à la position 624 par H, Y à la position 1 450 par D, C à la position 1 483 par R, R à la position 1 709 par H, T à la position 1 977 par K, R à la position 2 193 par C, S à la position 2 215 par F, L à la position 2 427 par P, et L à la position 2 427 par Q dans SEQ ID n° : 2,
des substitutions d'acide aminé arginine (R) à la position 22 par le tryptophane (W), R à la position 204 par C, et R à la position 811 par L dans SEQ ID n° : 4,
de la substitution d'acide aminé valine (V) à la position 1 547 par l'isoleucine (I) dans SEQ ID n° : 6,
de la substitution d'acide aminé arginine (R) à la position 247 par l'histidine (H) dans SEQ ID n° : 8, et
de la substitution d'acide aminé acide aspartique (D) à la position 1 018 par l'asparagine (N) dans SEQ ID n° : 10.

7. Composé inhibiteur de mTOR pour l'utilisation selon la revendication 1, la mutation somatique spécifique au cerveau comprenant au moins l'une sélectionnée dans le groupe constitué des mutations de nucléotide cytosine (C) à la position 616 par la thymine (T), guanine (G) à la position 1 871 par l'adénine (A), thymine (T) à la position 4 348 par la guanine (G), thymine (T) à la position 4 447 par la cytosine (C), guanine (G) à la position 5 126 par l'adénine (A), cytosine (C) à la position 5 930 par l'adénine (A), cytosine (C) à la position 6 577 par la thymine (T), cytosine (C) à la position 6 644 par la thymine (T), thymine (T) à la position 7 280 par la cytosine (C), thymine (T) à la position 7 280 par l'adénine (A) dans SEQ ID n° : 1,
des mutations du nucléotide cytosine (C) à la position 64 par la thymine (T), cytosine (C) à la position 610 par la thymine (T), et guanine (G) à la position 2 432 par la thymine (T) dans SEQ ID n° : 3,
de la mutation du nucléotide guanine (G) à la position 4 639 par l'adénine (A) dans SEQ ID n° : 5,
de la mutation du nucléotide guanine (G) à la position 740 par l'adénine (A) dans SEQ ID n° : 7, et
de la mutation du nucléotide guanine (G) à la position 3 052 par l'adénine (A) dans SEQ ID n° : 9.

8. Composé inhibiteur de mTOR pour l'utilisation selon la revendication 1, comprenant en outre un composé sélectionné dans le groupe constitué d'un diluant, excipient, agent stabilisant, tensioactif, agent gélifiant, agent d'ajustement du pH, agent anti-oxydant et conservateur pharmaceutiquement acceptables.
